# EUROPEAN PATENT APPLICATION

(11) **EP 2 633 884 A1**
(43) Date of publication of application: **04.09.2013**
(21) Application number: 13156136.7
(22) Date of filing: 07.01.2011
(51) Int. Cl.: A61P 3/04, A61K 31/427

(54) **Obesity small molecules**

(30) Priority: 07.01.2010 EP 10150270
(62) Divisional of application: 11700051.3
(71) Applicant: Akron Molecules GmbH, 1030 Vienna (AT)
(72) Inventor: Penninger, Josef, 1130 Vienna (AT); Pospisilik, Andrew, 79108 Freiburg (DE); Mcmanus, Shane, 1030 Vienna (AT)
(74) Representative: Sonn & Partner Patentanwälte

(57) **Abstract**

The present invention relates to new therapies to treat obesity and related diseases, as well as for reducing triglyceride levels and body weight.

## Description

The present invention relates to the field of pharmaceutical compounds and methods for the treatment of overweight and obesity.

The world health organization currently estimates that as of 2009 over 1 billion individuals worldwide are overweight. Almost one third of these individuals are clinically obese, markedly raising their chances of cardiovascular disease, type-2 diabetes, cancer, and stroke. The regulation of body fat content in animals results from the integration of multiple nutrient, sensory, and hormonal inputs primarily at the level of the brain and adipose tissues. This integrative network is influenced not only by genetics, but also by circadian rhythm and physical and social environments. Obesity is thus, a complex, systems level disease.

Spurned by the discovery of leptin, tremendous progress has been made in identifying the molecular players and pathways regulating adiposity. The impressive bounds made through the study of gene-targeted mice and the tracking of monogenic obesity disorders in humans, have been complemented by studies in lower organisms. Virtually all key metabolic regulators examined to date display conserved functions across phyla, including for instance, insulin signaling, mTOR, and key lipases such as ATGL. Similar to mammals, model organisms such as *Drosophila melanogaster, Danio rerio,* and *Caenorhabditis elegans* employ multiple molecular and tissue-based regulatory networks to balance energy needs, nutritional state, and aging and thus represent potent genomics tools for the study of metabolism. For instance, an RNAi feeding model was used to identify potential regulators of fat storage in the *C*. *elegans* genome.

It is a goal of the present invention to identify further genes, enzymatic pathways and active compounds for their modulation suitable for the treatment of triglyceride dysfunction, overweight and obesity.

The present invention therefore provides a method of reducing weight and/or body fat in a subject comprising the administration of a therapeutic compound selected from the compounds of table 1. E.g. a therapeutic dose disclosed or approved for other therapeutic uses for each of these compounds can be used.

In a further aspect the present invention provides a method of reducing triglyceride levels, in particular LDL levels, in a subject comprising the administration of a therapeutic compound selected from the compounds of table 1.

In a related aspect the present invention provides the use of a compound of table 1 for the manufacture of a medicament for the therapeutic administration to reduce body weight and/or body fat or to treat obesity in a subject. Also provided are these compounds for use in the therapies discloed herein. The invention is further defined by the subject matter of the claims.

In particular the present invention relates to use of the following compounds for the above tratments, in particular for reducing weight and/or body fat in a subject:
- Vandetanib,
- Dasatinib,
- Tanespimycin (17-allylamino-demethoxygeldamycin, "17-AAG"),
- S-17092,
- Sorafenib,
- Lintopride,
- Fenoprofen,
- Sulfaphenazole,
- Fluticasone (including Fluticasone propionate, "Ubizol"),
- Rolipram,
- Febuxostat,
- Verapamil (including Norverapamil),
- a therapeutic compound selected from a modulator of gene CG9438, or an ortholog thereof, in particular the human orthologue CYP3A4, the compounds being selected from erlotinib, gefitinib and lapatinib,
- a therapeutic compound selected from a modulator of gene CG8222, or an ortholog thereof, in particular the human orthologue KDR, the compounds being selected from axitinib, pazopanib and semaxanib (also known as Semaxinib or SU 5416),
- a therapeutic compound selected from a modulator of gene CG6919, or an ortholog thereof, in particular the human orthologue HTR4, the compounds being selected from cisapride, mosapride, piboserod, prucalopride, renzapride, tegaserod, tropisetron and zacopride,
or any combination of the above compounds with any one or more compounds selected from the compounds of table 1, preferably with the compounds given above. However, in other embodiments the compounds may be used alone in the inventive treatment described herein. According to this embodiment the invention provides for the inventive therapy wherein the compound is administered as the only therapeutic compound active (or indicated) in a treatment of reducing weight and/or body fat or of obesity in a subject. In particular, the inventive compound, especially Lintopride, is used for administration without a DPP IV inhibitor as disclosed in the WP 2006/005613

The inventive use of the compounds given herein includes the use of any pharmaceutically acceptable salt or hydrate form thereof.

In preferred embodiments the inventive compounds are used in the treatment of obesity, in particular severe obesity. Obesity is defined as an excess of body fat. Body mass index (BMI - the ratio of body weight in kg to the square of the height of an individual in m), is a useful measure of fat distribution. Importantly it allows the stratification of patient categories to identify increased risk of morbidity and mortality and the identification of suitable interventions. Furthermore it provides a firm basis for the assessment of intervention strategies. The stratification of obesity using BMI is as follows. BMI ≥25 = Overweight, BMI 25-29.99 = Preobese, BMI 30-34.99 = Obese class I, BMI 35-39.99 = Obese class II, BMI ≥ 40 = Obese class III (WHO 2000). Class II obesity is severe obesity and class III obesity is referred to as extreme obesity, associated with an extremely high risk of comorbidities including Type 2 diabetes mellitus; Hypertension; Dyslipidemia; Cardiovascular disease including Coronary artery disease, Stroke and Congestive heart failure; Nonalcoholic fatty liver disease (steatosis, steatohepatitis, cirrhosis); Respiratory disease including Obstructive sleep apnea, Obesity-hypoventilation syndrome, Asthma, Restrictive lung disease; Cancer; Osteoarthritis; Cholelithiasis; Gastroesophageal reflux disease; Gynecologic abnormalities including Infertility, Abnormal menses; Venous stasis; Skin problems such as Intertrigo and Cellulitis; Increased risk of complications during surgery or pregnancy; Urinary incontinence; Idiopathic intracranial hypertension (Hensrud et al., 2006). As the degree of obesity increases so does the risk of all cause mortality. Extreme obesity has been estimated to lead to a shortening of life of between 5 and 20 years depending on other factors including sex, age and racial group (Fontaine et al., 2003). Bariatric surgery is a common treatment for severely obese patients and the numbers of surgery performed per year is increasing along with the increased prevalence of obesity and in particular severe obesity. The number of bariatric surgeries performed in the US increased from 13,386 in 1998 to an estimated 170,000 in 2005 (Ecinosa et al., 2005). However only 0.6% of over 11 million extremely obese patients in 2002 are eligible for surgery actually underwent a bariatric procedure (Ecinosa et al., 2005). Furthermore extreme obesity is also associated with an increased prevalence of psychiatric illnesses. Between a half and two-thirds of all bariatric surgery candidates possess an Axis I psychiatric disorder such as depression, somatization, social phobia, hypochondriasis, or obsessive-compulsive disorder (Rosick et al., 2005; Sarwer et al., 2004).

The subject to be treated according to the present invention can be any nun-human animal or a human. Preferably the subject is a mammal, in particular preferred embodiments a human.

According to the present invention obesity, diseases associated with obesity, e.g. diabetes, can be treated or prevented, in particular in the meaning of a prophylactic administration. "Preventing" or "prevention" herein does not require absolute success in the sense of an absolute prevention of a heart disease but indicates a reduced risk of developing a disease, or developing a disease with reduced severity. Likewise, "treatment" shall not be construed as an absolute cure, but may also relate to amelioration of the disease or disease symptoms.

"About" is used to refer to certain dosages that can vary from a given value, nevertheless with the same effects as the indicated dose. In some embodiments "about" may refer to +/- 20% or 10% of a given value.

Preferably the compound is administered in a dosage sufficient to treat or prevent said diseases. Administration can e.g. be a sinlge dose administration or a successive or repeated administration, e.g. twice a day, daily or in an interval of at least 1 day, at least 2 days, at least 3 days, at least 1 week, preferably at least 2 weeks, at least 4 weeks, at least 8 weeks or even more preferred at least 12 weeks.

According to a further preferred embodiment of the present invention, the compound is provided in a pharmaceutical composition or a medicament. The composition or medicament may comprise a pharmaceutical carrier. Pharmaceutical carrier substances serve for a better tolerance of the medicament and allow for a better solubility as well as a better bioavailability of the active substances contained in the medicament. Examples of this are emulsifiers, thickening agents, redox components, starch, alcohol solutions, polyethylene glycol or lipids. The choice of a suitable pharmaceutical carrier is highly dependent on the manner of administration. For oral administrations, liquid or solid carriers may be used, for injections, liquid final compositions are required. For cellular targeting suitable vehicles can be includes such as liposomes or microsomes.

Preferably, the medicament or the compound to be used according to the invention comprises buffer substances or tonic substances. By means of a buffer, the pH of the medicament can be adjusted to physiological conditions, and moreover, pH fluctuations can be attenuated, or buffered, respectively. An example thereof is a phosphate buffer. Tonic substances serve for adjusting the osmolarity and may comprise ionic substances, such as, e.g., inorganic salts, such as NaCl, or also non-ionic substances, such as, e.g. glycerol or carbohydrates.

The inventive compound or medicament can be administered topical, enteral or parenteral, in particular preferred oral or rectal, intravenous, intraarterial, intramuscular, subcutaneous, intradermal or intraperitoneal, transdermal, transmucosal or inhalational. Preferred routes of administration of the inventive agent according to the present invention are parenteral routes, preferably intraperitoneal or intravenous administration, intravenous administration being specifically preferred. Intravenous administration can be performed e.g. via bolus injection or by continuous intravenous delivery over a longer time period (e.g. 30 min to 6 h, especially 1 to 3 h). Further routes include oral or transdermal or subcutaneous routes. In particular preferred is oral administration. For digestible agents, such as active proteins, peptides or siRNA, parenteral routes are preferred.

The medicament or the compound to be used according to the invention can be prepared to be suitable for oral or intranasal administration. These administration forms of the medicament of the present invention allow for a rapid an uncomplicated uptake of the active substances via the mucous membranes. For a nasal intake, nose drops or nose sprays are suitable. For an oral administration, solid or liquid medicaments may, e.g., be taken directly or in a dissolved or diluted state, respectively.

The medicament or compound to be used according to the invention can be prepared for an intravenous, intra-arterial, intramuscular, intravascular, systemic, intraperitoneal or subcutaneous administration. For this purpose, e.g., injections or transfusions are suitable. Administrations directly into the bloodstream have the advantage that the active substances of the medicament will be distributed in the entire body and will quickly reach the target tissue, in particular the heart muscle.

The compound may be administered in a effective therapeutic dose. Effective doses are in the range of dosages known for these compounds for other, non-obesity related administrations. In particular, for a specific use a dosage can be determined by a simple test using drosophila or mouse test systems, e.g. as shown in example 3. Preferably the dosage is determined in a mouse test, e.g. using DIO B6 mice, at six weeks of age, mice are fed high fat diet to induce obesity, e.g. a 60 kcal% fat diet. The appropriate dosage can be correlated with reduced obesity symptoms, in particular fat mass or body weight. Example dosages are at least 0.01 mg/kg, at least 0.1 mg/kg, at least 1 mg/kg, at least 10 mg/kg and/or up to 1 mg/kg, up to 10 mg/kg, up to 100 mg/kg, up to 1 g/kg, and any dosages in between. Preferred dosage ranges are between 0.01 mg/kg and 1 g/kg, preferably between 0.1 mg/kg and 100 mg/kg.

### Vandetanib

Vandetanib also known as Zactima; ZD6474; 4-Bromo-2-fluorophenyl)-[6-methoxy-7-(1-methyl-piperidin-4-ylmethoxy)-quinazolin-4-yl]-amine is an orally available tyrosine kinase inhibitor (TKI).

Vandetanib is currently in clinical trials for the treatment of various cancers, including colorectal cancer, non-small cell lung cancer, hepatocellular carcinoma and medullary thyroid cancer. Vandetanib is currently under review by the FDA Oncologic Drugs Advisory Committee (ODAC) proposed to be indicated for the treatment of patients with unresectable locally advanced or metastatic medullary thyroid cancer.

It may be administrered orally at doses ranging from 15mg to 300mg once daily. An oral dose of 300mg once daily is the maximum tolerated dose in humans. Doses used in the clinic range between 100mg and 300mg.

### Dasatinib

Dasatinib is also known as BMS-354825. Dasatinib is indicated for the treatment of adults with chronic, accelerated, or myeloid or lymphoid blast phase chronic myeloid leukemia (CML) with resistance or intolerance to prior therapy including imatinib.

A recommended oral starting dose of dasatinib in chronic phase CML is 100 mg once daily. The recommended starting dose for accelerated, myeloid, or lymphoid blast phase CML or Philadelphia chromosome-positive ALL is 70 mg twice daily. Doses of up to 140 mg once daily have been used in patients with chronic phase CML, and up to 100 mg twice daily in those with advanced phase CML, or with ALL. It may also be administered 15 to 240 mg per day (60kg adult human dose), preferably orally.

### Sorafenib

Sorafenib and the tosylate salt form Sorafenib Tosylate (chemical name 4-(4-{3-[4-chloro-3(trifluoromethyl)phenyl]ureido}phenoxy)-N2-methylpyridine-2-carboxamide-4-methylbenzenesulfonate) inhibits tumour cell proliferation and angiogenesis by targeting RAF Kinases and VEGF Receptors. Sorafenib is generally prepared as its tosylate salt form. Sorafenib and pharmaceutically acceptable salts thereof are disclosed in WO0042012. Sorafenib is also disclosed in WO0041698. Both these patents also disclose processes for the preparation of sorafenib.

Sorafenib is approved for the treatment of primary kidney cancer (advanced renal cell carcinoma) and advanced primary liver cancer (hepatocellular carcinoma). The maximum tolerated does in humans is an oral administration of 400mg twice daily. References: WO0042012, WO0041698 (incorporated herein by reference).

Sorafenib antagonizes activity of gene CG8222 (PDGF- and VEGF-receptor related). Whole body and fat body-specific knock-down of this gene resulted in a loss of trigylcerides in the fly. The human orthologue of this gene is KDR (kinase insert domain receptor (a type III receptor tyrosine kinase. It functions as mediator of endothelial proliferation, survival, migration, tubular morphogenesis and sprouting. The signalling and trafficking of this receptor are regulated by multiple factors, including Rab GTPase, P2Y purine nucleotide receptor, integrin al-phaVbeta3 and T-cell protein tyrosine phosphatase.

Sorafenib may be administered orally and has a half life of 25-48 hours. The dosage can be between 50 and 600 mg (adult human dose), preferably about 200 mg.

### Tanespimycin

Tanespimycin, also known as 17-allylamino-demethoxygeldamycin (17-AAG) and KOS-953, is an ansamycin antibiotic which acts as an anti-tumour agent. Specifically, Tanespimycin binds and inhibits Hsp90 (Heat shock protein 90). Hsp90 is a protein chaperone that binds to signalling proteins, known as client proteins. These client proteins include key cancer-relevant targets such as mutated p53, Bcr-Abl, Her2, Akt, Raf-1, B-Raf, and others. Tanespimycin is able to disrupt the Hsp90-client protein complexes and lead to the degradation of the client proteins. Tanespimycin and the related compound Al-vespimycin (INN) also known as 17-dimethylamino- geldanamycin (17-DMAG) or KOS-1022 are less toxic analogues of geldanamycin (GA).

Tanespimycin has been investigated for the treatment of patients with Relapsed-refractory Multiple Myeloma, Metastatic Pa-pillary or Clear Cell Renal Cell Carcinoma, Recurrent Advanced Ovarian Epithelial or Primary Peritoneal Cavity Cancer, Metastatic Breast Cancer and Refractory or Advanced Solid Tumours or Hematologic Malignancies.

Tanespimycin is water insoluble, and thus it is administered to patients intravenously using organic solvents such as DMSO. A formulation of tanespimycin, KOS-953, that contains Cremophory EL (polyethoxylated castor oil) rather than DMSO has also been developed. Recommended phase II doses of 295 mg/m2, 308 mg/m2, and 450 mg/m2 have been administered to patients.

Knock-down of the Drosophila gene CG1242 resulted in a reduction of triglyceride levels in the fly. The human orthologue of this gene is the heat shock protein HSP90AA1. HSP90 proteins are highly conserved molecular chaperones that have key roles in signal transduction, protein folding, protein degradation, and morphologic evolution. HSP90 proteins normally associate with other cochaperones and play important roles in folding newly synthesized proteins or stabilizing and refolding denatured proteins after stress. HSP90AA1 is one of the two major cytosolic HSP90 proteins.

In preferred embodiments 17-N-Allylamino-17-demethoxygeldanamycin may be administered i.p., i.v., or oral, preferably i.p., e.g. at doses of 60, 40, and 26.67 mg/kg i.p. and oral doses of 40 mg/kg. Preferably the dose may be between 1 mg/kg of body weight to 500 mg/kg, preferably at least 20 mg/kg or even above 80 mg/kg.

### S-17092

"S-17092" or "S 17092-1", (2S, 3aS, 7aS)-1{[(R, R)-2-phenylcyclopropyl]carbonyl}-2-[(thiazolidin-3-yl)carbonyl] oc-tahydro-1H-indole, is a selective inhibitor of the enzyme Prolyl endopeptidase. This enzyme is involved in the metabolic breakdown of a number of neuropeptide neurotransmitters in the brain and so inhibiting the action of the enzyme increases the activity of these neuropeptides. This produces nootropic effects which make S-17092 a promising and novel treatment for neurodegenerative conditions such as Alzheimer's disease and Parkinson's disease. S 17092 might possess some mood-stabilizing potential in addition to its cognition-enhancing properties.

S-17092 has been administered orally to patients at doses of 100, 400, 800 and 1200 mg once daily (Morain et al., 2000).

Knockdown of the drosophila gene CG5355 resulted in a loss of triglyceride levels in the fly. Tissue specific deletion in the liver and fat body also lead to a reduction of triglyceride levels in the fly. A human orthologue of CG5355 is PREP. PREP (Prolyl endopeptidase) is a cytosolic prolyl endopeptidase that cleaves peptide bonds on the C-terminal side of prolyl residues within peptides that are up to approximately 30 amino acids long. Prolyl endopeptidases have been reported to be involved in the maturation and degradation of peptide hormones and neuropeptides.

### Lintopride

Lintopride (also referred to as Lintopril) is a 5HT-4 antagonist with moderate SHT-3 antagonist properties. Lintopride has been shown in humans to increase the lower oesophageal sphincter (LOS) motility basal tone without affecting LOS physiological relaxation after swallowing and leads to an increase of peristaltic waves in the oesophagus.

It can be administered orally or intravenously and has been used at dosages of 0.1, 0.3, 0.5 mg/kg in humans.

Whole body and muscle specific knockdown of the Drosophila gene CG6919 lead to a reduction in triglyceride levels in the fly. The human orthologue of this gene is HTR4. This gene is a member of the family of serotonin receptors, which are G protein coupled receptors that stimulate cAMP production in response to serotonin (5-hydroxytryptamine). The gene product is a glycosylated transmembrane protein that functions in both the peripheral and central nervous system to modulate the release of various neurotransmitters.

### Fenoprofen

Fenoprofen, non-steroidal anti-inflammatory drug, is a propionic acid derivative with analgesic, anti-inflammatory and antipyretic properties. Fenoprofen calcium is used for symptomatic relief for rheumatoid arthritis, osteoarthritis, and mild to moderate pain.

Whole body, neuronal and liver specific knockdown of CG8451 lead to a reduction of fly triglyceride levels. This gene is an orthologue of the human gene SLC5A8. Inflammatory drugs such fenoprofen function as blockers of this transporter.

Fenoprofen is taken orally at doses ranging from 200mg up to a maximum recommended dose of 3.2g per day.

### Sulfaphenazole

Sulfaphenazole is a sulfonamide antibacterial and a specific inhibitor of CYP2C9. It blocks the pro-inflammatory and atherogenic effects of linoleic acid (increase in oxidative stress and activation of AP-1) mediated by CYP2C9.

Sulfaphenazole has been administered to patients by intravenous and intra-arterial perfusion (0.03 µg/100 ml tissue/min) (Giannarelli et al., 2009).

Whole body and liver specific knockdown of the fly gene CG3466 resulted in a reduction of triglyceride levels. CG3466 is a member of the cytochrome P450 enzyme family. Sulfaphenazole functions as an inhibitor of cytochrome function.

### Fluticasone

Fluticasone propionate (Ubizol) is a synthetic corticosteroid derived from fluticasone used to treat asthma and allergic rhinitis (hay fever). It is also used to treat eosinophilic esophagitis. Fluticasone propionate is delivered as an aerosol formulation and is also available as a cream for the treatment of eczema and psoriasis.

A recent study has indicated that Fluticasone propionate functions as a Smoothened (Smo) agonist that activate Hedgehog signalling (Wang et al., 2010).

The maximum recommended dose for fluticasone propionate aqueous nasal spray is 200 micrograms daily. Intranasal administration of 2 mg (10 times the recommended dose) of fluticasone propionate twice daily for 7 days to healthy human volunteers was well tolerated. Single oral doses up to 16 mg have been studied in human volunteers with no acute toxic effects reported. Repeat oral doses up to 80 mg daily for 10 days in volunteers and repeat oral doses up to 10 mg daily for 14 days in patients were well tolerated.

### Rolipram

Rolipram is a phosphodiesterase IV inhibitor with antidepressant properties. It is an anti-inflammatory drug being studied as a possible alternative to current antidepressants. Recent studies show that rolipram may have antipsychotic effects. Other beneficial effects of rolipram include improved long term memory, increased wakefulness, and increased neuroprotection. Rolipram shows promise in ameliorating Alzheimer's disease, Parkinson's disease and also in the regeneration of severed spinal cord axonal bodies.

Rolipram can be administered orally or intravenously usually at doses ranging from 0.001-10 mg per day.

### Febuxostat

Febuxostat (INN) is an inhibitor of xanthine oxidase. Xanthine oxidase functions to successively oxidize both hypoxanthine and xanthine to uric acid. Inhibition of xanthine oxidase by febuxostat reduces production of uric acid. Febuxostat is indicated for use in the treatment of hyperuricemia and gout. The recommended dose is of 40 mg or 80 mg orally once daily.

### Verapamil, Norverapamil

Verapamil and its salt Verapamil Hydrochloride is an L-type calcium channel blocker of the Phenylalkylamine class. It has been used in the treatment of hypertension, angina pectoris, cardiac arrhythmia, and migraine. Verapamil has also been used as a vasodilator during cryopreservation of blood vessels. It is a class 4 antiarrhythmic. The maximum recommended human daily dose is 480 mg/day by oral administration.

Further prefered compounds are associated with the gene CG9438 and the human orthologue CYP3A4 from the list of erlotinib, gefitinib and lapatinib:

### Erlotinib

Erlotinib, preferably used in its hydrochloride salt form (trade name Tarceva), is a drug used to treat non-small cell lung cancer, pancreatic cancer and several other types of cancer. It is a tyrosine kinase inhibitor, which acts on the epidermal growth factor receptor (EGFR).

### Gefitinib

Gefitinib (trade name Iressa) is an EGFR inhibitor drug used in the treatment of advanced non-small cell lung cancer (NSCLC).

### Lapatinib

Lapatinib, preferably used in the form of lapatinib ditosylate (USAN) (Tykerb/Tyverb, GSK), is an orally active drug for treatment of breast cancer and other solid tumours. It is a dual tyrosine kinase inhibitor which disrupts the HER2 growth receptor pathway.

Further prefered compounds are associated with the gene CG8222 and its human orthologue KDR selected from the list of axitinib, pazopanib, and semaxanib (also known as Semaxinib or SU 5416):

### Axitinib

Axitinib is a small molecule tyrosine kinase inhibitor. It has been shown to significantly inhibit growth of breast cancer in xenograft models and has been successful in trials with renal cell carcinoma (RCC) and several other tumour types.

### Pazopanib

Pazopanib is a multi-targeted receptor tyrosine kinase inhibitor. It has been approved for treatment of renal cell carcinoma. Pazopanib may also be active in ovarian cancer and soft tissue sarcoma and in the treatment of non-small cell lung carcinoma.

### Semaxanib (also known as Semaxinib or SU 5416)

Semaxanib is a tyrosine kinase inhibitor that has been withdrawn from clinical trials after failing to show efficacy in the treatment of patients with advanced stage colorectal cancer. Further prefered compounds are associated with the gene CG6919 and its human orthologue HTR4 selected from the list of cisapride, mosapride, piboserod, prucalopride, tegaserod, tropisetron, renzapride, and zacopride:

### Cisapride

Cisapride is a gastroprokinetic agent, a drug which increases motility in the upper gastrointestinal tract. Cisapride increases muscle tone in the esophageal sphincter in patients with gastroesophageal reflux disease. It has alos been used to treat bowel constipation.

### Mosapride

Mosapride is a gastroprokinetic agent which accelerates gastric emptying and is used for the treatment of acid reflux, irritable bowel syndrome and functional dyspepsia.

### Piboserod

Piboserod (also known as SB 207266) is used for the treatment of atrial fibrillation and irritable bowel syndrome. It is also being investigated as a treatment for heart failure

### Prucalopride

Prucalopride treats the impaired motility associated with chronic constipation, thus normalising bowel movements.

### Renzapride

Renzapride is a gastroprokinetic agent and antiemetic, which was being investigated for the treatment of constipation-predominant irritable bowel syndrome (IBS-C). It is also potentially effective for irritable bowel syndrome with alternating stool pattern (IBS-A). However it failed to show efficacy over placebo in Phase III clinical trials and development was discontinued.

### Tegaserod

Tegaserod functions as a motility stimulant that stimulates gastrointestinal motility and the peristaltic reflex. It was approved for the treatment of irritable bowel syndrome and constipation.

### Tropisetron

Tropisetron is an antiemetic used to treat nausea and vomiting following chemotherapy. It has also been used experimentally as an analgesic in the local treatment of tendinopathies and myofascial pain syndromes.

### Zacopride

Zacopride been shown to have many activities including anxiolytic and nootropic effects. It has also been shown to have antiemetic and pro-respiratory effects, both reducing sleep apnoea and reversing opioid-induced respiratory depression in animal studies.

The above examples show that the inventive obesity tests revealed pharmaceutical compounds that are well known to be therapeutically applicable for the treatment of human conditions and diseases. The compounds may now also be used for the treatment of obesity and associated secondary diseases such as diabetes or the metabolic syndrome. Of course the full list of compounds according to table 1 provides new therapeutic concepts.

Further compounds to be used in any form of treatment, e.g. in combination with the above compounds or for use alone, according to the present invention are selected from any one of (5-(2-methoxy-5-chloro-5-phenyl)furan-2-ylcarbonyl)guanidine, (E)-4-(2-(2-(N-acetyl-N-(4-methoxybenzenesulfonyl)amino)stilbazole)) 1-oxide, (melle-4)cyclosporin, 1-(1-cyclohexylethylamino)-4-phenylphthalazine, 1-(2-methoxyphenyl)-4-(4-(2-phthalimido)butyl)piperazine, 15-deoxyprostaglandin J2, 17-(allylamino)-17-demethoxygeldanamycin, 17-(dimethylaminoethylamino)-17-demethoxygeldanamycin, 1-aminooxy-3-aminopropane, 1-beta-D-arabinofuranosyl-Cytosine, 1-carbamoyl-4-phenylpyrrolidone-2, 1-Carboxyglutamic Acid, 1-deoxygalactonojirimycin, 1-hydroxymethylmidazolam, 2-[2-[2-[2-[2-amino-3-(4-hydroxyphenyl)-1-oxo-propyl]amino-1-oxoethyl]amino-1-oxo-ethyl]amino-1-oxo-3-phenyl-propyl]amino-4-methyl-pentanoic acid, 25-desacetylrifabutin, 2-AAF, 2-AAF, 2-AG, 2-AG, 2-AP, 2-cyclopentyl-5-(5-isoquinolylsulfonyl)-6-nitro-1H-benzo(D)imidazole, 2-DG, 2-hydroxy-1-naphthylaldehyde isonicotinoyl hydrazone, 2-methoxyacetic acid [2-[2-[3-(1H-benzoimidazol-2-yl)propyl-methyl-amino]ethyl]-6-fluoro-1-isopropyl-tetralin-2-yl] ester, 2-methylarachidonyl-2'-fluoroethylamide, 2-phenyl-4-oxohydroquinoline, 2-propylquinoline, 3-(5-((4-(methylsulfonyl)-1-piperazinyl)methyl)-1H-indole-2-yl)quinolin-2(1H)-one, 3-AB, 3'-deamino-3'-hydroxydoxorubicin, 3-HF, 3-Methoxyoestradiol, 3-MF, 4-(4-(1-Amino-1-methylethyl)phenyl)-2-(4-(2-morpholin-4-yl-ethyl)phenylamino)pyrimidine-5-carbonitrile, 4'-epidoxorubicin, 4-methyl-N-(3-(4-methylimidazol-1-yl)-5-(trifluoromethyl)phenyl)-3-((4-pyridin-3-ylpyrimidin-2-yl)amino)benzamide, 4'-N-benzoylstaurosporine, 4PBA, 4-PCB, 5-(4'-(N-piperidinyl)phenylazo)indazole, 5-bromo-4-chloro-3-indolyl beta-galactoside, 5-carboxamidotryptamine, 5-demethylovalicin, 5'-O-(((2-decanoylamino-3-phenylpropyloxycarbonyl)amino)sulfonyl)uridine, 6'-N-methylsisomicin, 7 HC, 7,8-BF, 7C3MT, 7-hydroxystaurosporine, 7-ketocholesterol, 8-Hydroxy-2-(di-n-propylamino)tetralin, 8-hydroxyguanosine, 9-(2-hydroxy-3-nonyl)adenine, 9-beta-D-erythrofuranosyladenine, 9-CRA, 9-hydroxy-risperidone, A-300-I, a-ADP, AAL 881, AATP, AB 2, abacavir, Abufene, Acenocoumarol, Acetidin, Acetorphan, acetoxymethyl-ester, Aclarubicin, ACNU, Acolen, ACON, actein, acteoside, Actihaemyl, Actosin, adalimumab, Adanon, ADMA, ADMA, Adofeed, Adrenor, Adrin, AEBSF, AEE 788, AG 1879, ajoene, Aklavin, alachlor, Alat, ALDA, Aldara, Aldocorten, alemtuzumab, Alendronate, Alfarol, Alfentanil, ALIMTA, aliskiren, Alli, Allnal, allylamino-demethoxy-geldanamycin, alpha-neoendorphin, alternagin-C, Alvesco, alvimopan, Am 80, Amatin, AMCHA, AMD 070, amentoflavone, Amiloride, Amine BB, Amiodarone, Amiodarone, amlexanox, Amlodipine, Ammo, Ampicillin, amprenavir, amrubicin, AMSA, amsonic acid, Anaboleen, Anandamide, Anco, and-carboxyfluorescein-diacetate-succinimidyl-ester, Androstenediol, anilinyl, Anisomycin, ANSA, antibiotic G 418, antibiotic H107, antineoplaston A10, Antizol, APAP, APDC, Aphidicolin, Aphidicolin, Aphloiol, apicidin, Apigenin, aplidine, aprepitant, APRL, Apsor, aptiganel, Aralen, arctiin, Arecoline, Areether, argatroban, aripiprazole, Armor, Artein, ASTA, astatine, Astemizole, atazanavir, Atorel, atorvastatin, Atosil, Atovaquone, ATRA, Auluton, aureobasidin A, Aurothioglucose, AuTM, Axert, axitinib, Axsain, azacyclonol, Azadc, azamulin, azanediyl group, azaspirane, azelastine, azelnidipine, azidoprazosin, Aziran, Azithromycin, Azor, Azur A, Azure B, BA (VAN), Baclofen, Bagren, baicalein, Barnidipine, BAY 11-7085, Beflavin, Benidipine, benzoyl-staurosporine, beraprost, Berbamine, berberine, bergamottin, bergaptol, BESTATIN, beta-citronellol, beta-eudesmol, beta-funaltrexamine, beta-gamma-iminotriphosphate, bexarotene, Bezafibrate, BI D1870, biapigenin, BIBF 1000, BIBW 22, bifeprunox, Bisoprolol, Bisphenol A-Glycidyl Methacrylate, bizelesin, Bleomycin, BM 41.440, BMS 310705, BMS-262084, BMS453, BMS-470539, BMY 7378, Borrelia-burgdorferi, bortezomib, bosentan, bosutinib, Bo-Xan, Brefeldin A, bremazocine, bryostatin, Budesonide, bufalin, Bumetanide, Bupivacaine, Buprenorphine, Buspirone, Buthionine, Buthionine Sulfoximine, cabergoline, CACP, Calcijex, Calcimycin, calphostin C, Calyculin, Camptothecin, candesartan, candoxatril, candoxatrilat, Canef, CAPE, capsanthin, capsazepine, Carbamazepine, carbamic acid, Cardiolipins, Cardioplegic Solutions, carebastine, carfentanil, Carisoprodol, CARNOSOL, carvacrol, carvedilol, Casodex, caspofungin, casticin, catechins, CD 437, Cefotaxime, Ceftazidime, celecoxib, Celiprolol, CEP 701, cephalomannine, cerivastatin, Cerulenin, Cetirizine, Cetirizine, cetuximab, CGS 26303, CGS 35066, CGS 35601, Chloramphenicol, chloroprocaine, Chlorzoxazone, chymostatin, cicaprost, ciglitazone, Ciguatoxins, Cillora, cilostazol, Cimetidine, CINK4, Cipol N, Ciprofloxacin, Ciprol, Cisapride, Citalopram, Citox, CJ-15161, Cladribine, clavosine B, clavulone II, clobazam, Clodronic Acid, Clofazimine, Clofibric Acid, Clomipramine, Clonazepam, Clonidine, clonidine-displacing substance, clopidogrel, clotiazepam, Clozapine, CMDBS 25, Co 2-1970, Colchicine, Cordanum, cornuside, costunolide, Cotinine, Cotrim, coumarin, coumarin, coumarin, coumermycins, CP 31398, CRA 026440, CREBtide, Crestor, Crodacid, crypto-tanshinone, cryptoxanthin, Cyclandelate, cyclohexanecarboxylic acid (2-(4-(2-bromo-5-methoxybenzyl)piperazin-1-yl)ethyl)-(2-trifluoromethoxyphenyl)amide, cyclopamine, cyclopiazonic acid, cycloprodigiosin, cyclosporin G, cyclotheonamide A, Cyproterone Acetate, Cystamine, cytarabine, D 21266, DA 8159, DABS, Dacarbazine, DADA, DADSO, daidzein, Dalteparin, D-AM, danaproid, Danazol, Dapsone, Daral, Darifenacin, dasatinib, DAU 6285, Daunorubicin, dauricine, Dayfen, Dddd-PGD2, decursin, Deferoxamine, DEHP, dehydroaripiprazole, Dehydroepiandrosterone Sulfate, dehydroxymethylepoxyquinomicin, Delavirdine, delphinidin, delta-1-pyrroline-5-carboxylate, delta8-THC, Denagard, denbinobin, denosumab, deoxyhypusine, deoxyverrucosidin, Depas, dephosphonocalyculin A, Deproceptin, deramciclane, dermorphin, desethylchloroquine, desloratadine, desmethylazelastine, Desmethyldeprenyl, desmethyl-tamoxifen, DEVD-CHO, dexecadotril, dexloxiglumide, Dextropropoxyphene, Diaben, Diacomit, diadenosine tetraphosphate, Didanosine, dillapiol, Diltiazem, Dinoprostone, Diosgenin, diosmetin, dioxirane, Dioxolan, Dipyrone, Disopyramide, Ditiocarb, Dizocilpine Maleate, DNSC1, Doca, dofequidar, Dolomin, Domperidone, Doxazosin, doxifluridine, Doxycycline, DPC 681, DPCPX, DPPH, Droxia, Drysol, duloxetine, Durapatite, Dursbanoxon, DX 9065a, Dxms, dynapen, Dynatra, dynorphin, dysidenin, dysprosium, dystrobrevin, E 10, EACA, ebastine, ebrotidine, Econ, econazole, ecteinascidin 743, ectoine, Edex, Edrophonium, efavirenz, efrapeptin, EGCg, EGRck, EHT 1864, eletriptan, Embelin, embellistatin, EMD 53998, EMD 61753, emetine, E-MIX 80, Emodin, Empecid, emtricitabine, enadoline, Enalapril, Enalaprilat, Enediynes, Enelone, Enoximone, EPIB, Epicar, epicatechin gallate, epimedin C, Epoprostenol, Epostane, Epothilones, epoxybergamottin, epsilon-viniferin, eptifibatide, ergotamine, eriocalyxin B, erlotinib, erucin, Eryc, Eskazine, Estramustine, ET18-Ome, Etfc cpd, Ethacrynic Acid, Ethambutol, Etidronic Acid, Etodolac, Etoposide, etoricoxib, Etorphine, etravirine, Eufor, eumelanin, eupatilin, everolimus, Evex, Evodin, exenatide, Extina, ezetimibe, F 11440, Fanchinine, F-Ara-A, febuxostat, felbamate, Felodipine, fenitrothion, fenofibric acid, Fenoprofen, Fenretinide, fexofenadine, fingolimod, fipronil, fisetin, FLCZ, Flecainide, Flesinoxan, flibanserin, Floxacillin, Fludeoxyglucose F 18, Flunarizine, fluorexon, Fluorouracil, Fluparoxan, Flupenthixol, fluvoxamine, fondaparinux, Fonofos, Format, Forskolin, fosamprenavir, Foscarnet, Frakefamide, fucoidan, fulvestrant, Fura-2, furafylline, Furylfuramide, FYDE, Gambogic acid, gedunin, gefitinib, Geldanamycin, Gemfibrozil, genipin, Gentamicins, gepirone, Gestodene, GF 120918, GGTI 298, GI 129471, Ginkgo-biloba-extract, glabridin, GLCa, Glumin, Glycyron, glyox, Gnidimacrin, gossypetin, gossypol, GR 113808, grifolin, Grofo, Guggulsterone, gusperimus, Harzol, Hbim, HESPERETIN, Hexadimethrine, HMBA, hypsiziprenol A9, hypusine, hyrtioerectine A, ibandronic acid, IBMX, I-BOP, Ibotenic Acid, Icosapent, ICRF 193, idebenone, IDN 5390, Ifosfamide, Ifosfamide, IGF-1, Iloprost, imatinib, imidafenacin, imperatorin, Impulsin, Imrecoxib, Imutex, indazole, Indinavir, indiplon, indirubin-3'-monoxime, infliximab, inogatran, Ipral, Iprivask, ipsapirone, irbesartan, Iressa, irinotecan, irisolidone, irofulven, Irox, Ismo, Isobac, isochaihulactone, Isodonol, isoflavone, Isorhamnetin, isosteviol, Isradipine, Istidina, ITF 2357, Itraconazole, Ivermectin, ixabepilone, J 113397, J 113397, jasplakinolide, juglone, juzentaihoto, K 252, kaempferol, KAFA, kahweol, Kainic Acid, kami-untan-to, Kaolin, KB 2796, K-DR, Keloid, Kemi, ketazocine, Keto-pgf1alpha, KKHA-761, KN 62, KN 93, KNK 437, KP372-1, K-PAM, KPMK, KR 31831, KRM 1648, K-SR, kurarinone, KYNA, L 685458, L797591, LAGA, Lamivudine, lamotrigine, lanreotide, lapachenole, lapatinib, LAQ824, laquinimod, latrunculin A, LBH589, leflunomide, lenalidomide, Lendorm, Lentinan, Leukotriene B4, Leukotriene C4, Leukotriene D4, leukotrienes, Levonorgestrel, liarozole, lintopride, liquiritin, LMWH, LNAC, lonafarnib, lonidamine, Loperamide, lopinavir, Lopril, Loratadine, Lorazepam, Lorex, Losartan, Lovan, loxiglumide, L-T3, lupeol, luteolin, lutetium, Lutex, LY 117018, LY 293111, LY 293284, LY 303511, LY231514, mahanine, Maleimides, Malix, manzamine A, maraviroc, MBCl, MCYST-LR, Mebumal, Medemycin, Medroxyprogesterone 17-Acetate, Melatol, meletin, melitten, meloxicam, Memantine, Menatetrenone, MENT, Mephenytoin, Meprobamate, MeSAdo, mesalamine, Mesaton, mesoglycan, Mesol, metazachlor, Meth, methanopterin, Methorphan, Methoxsalen, methoxymorphinan, Methylprednisolone, Methylthioino-sine, Metkephamid, Metopiron, Metribolone, Miazine, miconazole, Mictonorm, Midazolam, Mifepristone, miglustat, milbemycin, Mimosine, mirtazapine, Mit-C, mithramycin A, Mitoxantrone, MK-0524, MLN 944, Molsidomine, Monensin, monocillin I, monodansylcadaver-ine, mono-N-demethyladinazolam, Monorden, MORIN, morphine-3-glucuronide, mosapride, motapizone, Motuporin, moxifloxacin, MRK 003, MTPA, Muran, Muscarine, N-(4-aminophenethyl)spiroperidol, N-(4-aminophenyl)maleimide, N-(4-cyano-benzo(b)thiophene-2-carbonyl)guanidine, N-(8-amino-1-carboxyoctyl)-alanyl-proline, N(alpha)-(4-amino-4-deoxypteroyl)-N(delta)-hemiphthaloyl-L-ornithine, N-(m-heptyl)-5-chloro-1-naphthalenesulfonamide, N-3-isoquinolinyl-2-((4-pyridinylmethyl)amino)benzamide, NABU, Naftalen, Naloxone, Naltrexone, naltrindole, NAN-190 hydrobromide, nanchangmycin, Naphazoline, NARIGENIN, nateglinide, N-benzyloxycarbonylprolylprolinal, N-dehydroxyzileuton, N-desmethylclobazam, nedaplatin, Nefazodone, Nelfinavir, nemonapride, neodymium, Neomycin, N-ethylmaleimide, netoglitazone, neuromedin C, Neut, Nevirapine, Niacinamide, nicaraven, Nicardipine, Niceritrol, Nigericin, niguldipine, Nimodipine, NK 104, NMDA, N-methylsulfonyl-6-(2-propargyloxyphenyl)hexanamide, NN 703, Nobiletin, NOC 18, Nociceptin, noralfentanil, Norbinaltorphimine, norcantharidin, Nordihydroguaiaretic Acid, Norethindrone, noreximide, norfluoxetine, norlaudanosoline, Nortilidine, norverapamil, novobiocin, NPI 031L, NRDC, NSC 23766, NSC 295558, NSC 366140, NSC 663284, N-tert-butyl-3-[4-(2-methoxyphenyl)piperazin-1-yl]-2-phenylpropanamide, NU2058, NU6102, nutlin 3, NVP-AEW541, obovatol, OC 144-093, ochratoxin A, Octreotide, Octreotide, O-demethyltramadol, O-desethylreboxetine, oenothein B, Ogen, OH-pro, Oktan, Olamine, olanzapine, oleandrin, oleylamide, olmelin, olomoucine, Olymp, omalizumab, omapatrilat, omega-N-Methylarginine, Omeprazole, omeprazole sulfone, Ondansetron, ONO 1301, Optef, Org 31540, Orphenadrine, OSI 930, OSU 03012, Ouabain, ovalicin, Ovex, oxaliplatin, oxatomide, oxcarbazepine, Oxidopamine, oxymatrine, Oxytrol, Paclitaxel, palytoxin, pamidronate, p-Aminohippuric Acid, panaxadiol, pantoprazole, PAPP, Parthenolide, Patulin, pazopanib, PCSO, PD 169316, PD 173074, PD 98059, PDBU, Pectenotoxin II, pegvisomant, Pemetrexed, pentosidine, Pentoxifylline, Peplomycin, Pepstatin A, Perazine, Perillol, Perindopril, perylene bisimide, phen, phen, phenanthrene, phenothiazines, Phenprocoumon, Phenytoin, pheophorbide a, phloretin, Picibanil, picric acid, picropodophyllin, pidotimod, pifithrin, pindobind, pioglitazone, Piroxicam, plumbagin, Pluronic p 85, PMPA, PMSF, posaconazole, PP-IX, Pragmoline, Pravastatin, Prazosin, PRDL, Prednisone, preussin, Primidone, Proadifen, Procasil, Procetofen, Prodigiosin, Prodix, Promegestone, Propofol, prostaglandin A1, prostaglandin D2, prostaglandin E1, prostaglandin F2alpha, prostaglandin H2, prostaglandin J2, prostaglandins, prostaglandins G, prostratin, prucalopride, prunustatin A, Pseu-dohypericin, pseudolaric acid B, psilocybin, Psoralens, PTAP, PTX-B, puerarin, Pugnac, p-XSC, Pyrethrins, pyrvinium, quercitrin, quetiapine, Quicifal, quinupristin-dalfopristin, R 101933, rabeprazole, radester, Raloxifene, ramiprilat, rebamipide, reboxetine, remifentanil, renzapride, repaglinide, Requip, Revex, Rhodinal, Riacon, Ribavirin, Rifabutin, Riluzole, rimorphin, risedronic acid, risperidone, Ritodrine, Ritonavir, rituximab, RMI 12330A, Ro 13-8996, Ro 64-0802, Robitet, Rolipram, romidepsin, ropivacaine, Roquefortine, roscovitine, rosiglitazone, rosmarinic acid, rosuvastatin, Rozevin, RPR 121056, Rulid, rutecarpine, Rutin, S 17092-1, S 9788, S azabisabolene, Safingol, SAHA, saintopin, Salicin, salinomycin, salinosporamide A, salubrinal, salvin, salvinorin A, samarium, sampatrilat, sangivamycin, Saquinavir, Sarasar, sarizotan, SB 203580, SB 207266, SB 216763, SB 225002, SB 415286, SB-649915, SB-706375, SCH 66712, schizandrin B, SCIO-469, scoparone, Sediel, selamectin, Selegiline, Serad, sesamin, sevoflurane, Sildenafil, silybin, Sizofiran, sofalcone, sorafenib, SP 100030, sparfloxacin, spiradoline, spiraprilat, spirogermanium, SR 48692, SR 59230A, SRI 63-154, SRIH, ST 1481, STA 5326, stachy-botrydial, Stanozolol, Stavudine, SU 1498, SU 5416, SU 5614, SU 6656, SU 6668, SU 9516, Sucralfate, Sufentanil, Sulem, Sulfamerazine, Sulfamethazine, sulfamide, Sulfaphenazole, Sulfoximine, Sulindac, sultopride, Sumatriptan, sunitinib, sural, T 0901317, TAC 101, Tacrolimus, Tamogel, tamsulosin, tandospirone, Tangeretin, tanshinone, tariquidar, Taseron, Taurolin, TAXOTERE, tazarotene, TBDZ, TBHQ, TCDD, Tegafur, tegaserod, telithromycin, telmisartan, temozolomide, temsirolimus, Teniposide, Terfenadine, Teriparatide, Tetraprenol, TG101209, thalicarpine, Thapsigargin, Theaflavin, Thiazolidinediones, thiocoraline, thioridazine, Thiorphan, thromboxane B2, thulium, thymalfasin, Thymopentin, thymoquinone, Ticlopidine, Tilidine, tipifarnib, tipranavir, tirilazad, TKI-31, Tmndga, TMPN, Toddalin, Todralazine, tofisopam, Tolterodine, topiramate, Topotecan, Toremifene, Toxaphene, Tramadol, Tramat, trandolapril, Trapidil, trapoxin A, trastuzumab, Trazodone, Tremode, triazolam, triazolobenzodiazepines, tributylstannane, trichostatin A, trichostatins, Trifluoperazine, trioctyl phosphine oxide, Triolein, triptolide, TRK 820, troglitazone, Troleandomycin, tropisetron, Trospium chloride, Tunicamycin, tylophorine, Tylox, tyrphostin AG-490, tyvelose, U 0126, U 69593, Ubizol, UH 301, Usaf B-12, USAN, Valproic Acid, valsartan, valspodar, vandetanib, vapreotide, venlafaxine, Verapamil, verlukast, verrucosidin, versipelostatin, VGA1155, Vigil, vincaleukoblastine, vincristine, Vindesine, vinorelbine, Visken, Viviq, voriconazole, vorozole, vulnibactin, Wakil, Warfarin, Wartmannin, WAY 100635, Wogonin, WR 1065, WS 79089B, xanthohumol, Xaxa, ximelagatran, Xylit, Y 27632, YM-201627, YM-231146, zacopride, zafirlukast, ZD 4190, zeaxanthin, Zeldox, zileuton, Zimco, zincov, ZK 112993, ZM323881, zopiclone, ZSTK474, Zymosan, or a combination thereof. These compounds interact with newly identified key regulators of fat deposit functions, triglyceride circulation and obesity. Mechanistically the inventive treatments involves the modulation of the genes and gene function or interaction with the gene products, in particular proteins, of the genes listed in table 1.

According to the investigation described herein it was found that these compounds modify at least one gene selected from the drosophila genes CG30184, CG10369, CG32401, CG2374, CG8693, CG14909, CG13299, CG7847, CG30462, CG30462, CG15169, CG1650, CG6577, CG30491, CG4373, CG10407, CG2198, CG6356, CG5744, CG9506, CG31169, CG1728, CG9220, CG15625, CG5550, CG13088, CG13188, CG14968, CG1503, CG1666, CG14869, CG2702, CG2984, CG4394, CG9922, CG14529, CG17781, CG17781, CG9153, CG15178, CG5641, CG3879, CG15579, CG1422, CG6299, CG8107, CG7103, CG10617, CG30360, CG32971, CG32336, CG31036, CG12602, CG9676, CG1433, CG1100, CG31697, CG7095, CG2165, CG10230, CG10916, CG3274, CG18767, CG5072, CG3396, CG15582, CG16826, CG6788, CG9487, CG1888, CG4637, CG15162, CG5719, CG2254, CG4695, CG14936, CG17867, CG15646, CG5402, CG15095, CG8250, CG18030, CG14303, CG14164, CG14677, CG12105, CG17440, CG32459, CG11404, CG8954, CG13138, CG9056, CG12997, CG12997, CG5436, CG14330, CG10809, CG1622, CG3893, CG1112, CG31690, CG12664, CG13679, CG17556, CG10062, CG31744, CG9760, CG1555, CG14375, CG32170, CG4271, CG32234, CG7287, CG14341, CG30486, CG31692, CG31421, CG5467, CG30065, CG9086, CG1688, CG17026, CG4415, CG10343, CG15388, CG13984, CG3313, CG13116, CG4662, CG6919, CG17841, CG30411, CG9053, CG1180, CG14166, CG13125, CG13344, CG1490, CG2867, CG5591, CG14362, CG1531, CG15390, CG6689, CG14234, CG14265, CG5674, CG3917, CG8257, CG9028, CG1722, CG18402, CG7082, CG11797, CG3663, CG16704, CG31172, CG31219, CG1363, CG6721, CG5688, CG8527, CG13137, CG6612, CG6947, CG7737, CG1705, CG14704, CG10300, CG3597, CG3425, CG2540, CG6856, CG12259, CG4583, CG3843, CG9634, CG3809, CG9295, CG9485, CG11555, CG11601, CG14095, CG10166, CG2852, CG14164, CG14164, CG2898, CG3162, CG6603, CG8721, CG17742, CG14127, CG8665, CG9438, CG32113, CG32353, CG4957, CG33558, CG11570, CG32669, CG11575, CG30271, CG7830, CG31061, CG2076, CG17596, CG6824, CG17921, CG12875, CG13020, CG13972, CG13673, CG10772, CG8079, CG13127, CG9144, CG8979, CG7097, CG11768, CG10632, CG14903, CG1874, CG33466, CG3367, CG4851, CG17985, CG31229, CG3260, CG13023, CG11125, CG17184, CG31812, CG13360, CG30075, CG30183, CG7485, CG5495, CG5495, CG7065, CG13202, CG7779, CG9322, CG7091, CG16758, CG5071, CG4920, CG1516, CG9554, CG10101, CG3004, CG7796, CG10152, CG18741, CG8444, CG11425, CG10128, CG10542, CG11878, CG14434, CG12345, CG2091, CG31459, CG13319, CG7177, CG7776, CG15005, CG31605, CG7213, CG17283, CG18268, CG3017, CG7567, CG32091, CG9695, CG8222, CG1515, CG8256, CG1975, CG32467, CG3817, CG4038, CG6193, CG1572, CG8117, CG3526, CG7099, CG18525, CG9198, CG30470, CG17273, CG31439, CG1387, CG9952, CG6580, CG10840, CG13221, CG8202, CG8786, CG7199, CG11663, CG12683, CG31161, CG8009, CG17202, CG1683, CG17335, CG33204, CG14694, CG11229, CG16836, CG12209, CG18414, CG13475, CG11621, CG13332, CG11756, CG11133, CG18586, CG4944, CG3213, CG4152, CG6147, CG8515, CG5827, CG12691, CG8308, CG13807, CG2260, CG30004, CG4247, CG4247, CG5739, CG4202, CG4264, CG5245, CG13707, CG3523, CG10686, CG9565, CG4111, CG14673, CG31132, CG5355, CG32149, CG8443, CG17461, CG8190, CG13744, CG9258, CG6043, CG1759, CG8534, CG14792, CG8451, CG8654, CG12806, CG14938, CG9399, CG10542, CG13168, CG31845, CG6277, CG17819, CG2818, CG1688, CG13868, CG17736, CG7546, CG31693, CG12897, CG2146, CG3440, CG3696, CG12426, CG18319, CG18279, CG18279, CG3054, CG2145, CG3825, CG9781, CG13423, CG12030, CG14911, CG3911, CG6122, CG7206, CG8566, CG30476, CG9470, CG6127, CG5381, CG12505, CG1279, CG32140, CG12184, CG31364, CG1963, CG5484, CG4634, CG9748, CG32442, CG1921, CG18740, CG1242, CG9946, CG11121, CG3497, CG6817, CG30080, CG1171, CG11430, CG10691, CG13281, CG11352, CG3839, CG14368, CG14024, CG9936, CG11505, CG11906, CG1263, CG14011, CG11339, CG12015, CG30389, CG17331, CG15432, CG15507, CG14842, CG3906, CG17754, CG5289, CG5378, CG5625, CG6156, CG13243, CG8239, CG1821, CG7762, CG3108, CG8053, CG3605, CG4207, CG8431, CG9098, CG5270, CG5595, CG6064, CG6967, CG7134, CG7549, CG6892, CG10687, CG10712, CG11981, CG12770, CG15599, CG18563, CG7770, CG6322, CG3806, CG3980, CG6054, CG7292, CG3992, CG2998, CG8337, CG13194, CG5147, CG16903, CG11202, CG10084, CG12323, CG31484, CG6949, CG7352, CG10728, CG11376, CG32210, CG7109, CG8615, CG9160, CG8298, CG15115, CG1965, CG12595, CG15321, CG6009, CG11267, CG4453, CG3971, CG17255, CG32791, CG14016, CG14016, CG1740, CG32667, as well as their human orthologues. According to the present invention function of at least one of these genes is modified by the inventive compounds, in particular the small molecules given in table 1. In preferred embodiments the compound modulates at least two, three, four, five or six or more of these genes (or orthologues). Further compounds suitable to modulate gene function include the administration of therapeutic proteins or nucleic acids, such as transgenes or inhibitory nucleic acids (RNAi molecules, siRNA, antisense RNA or DNA). Such interfering nucleic acids bind messages of the genes leading to degradation and reduced gene expression. Preferred therapeutic proteins include the gene products of these genes (as agonists) or antibodies which specifically bind these proteins (as antagonists, but also as agonists if protein activity is increases - such as by binding and blocking an inhibitor binding site). The inventive compounds can act as either agonist by increasing the gene function (via mRNA regulation or interaction with the protein) of a protein in the enzymatic pathway of any one of the above listed genes or an antagonist in said pathways. The antagonizing or activating (agonist) activity of the compounds acts preferably on the identified obesity genes (including their gene product) themselves or on a binding partner thereof. In preferred embodiments antagonists of the obesity genes are used.

Among the genes that can be targeted are also potential regulators of feeding control. For instance, odorant receptor genes 10a, 56a, 65a, 67a, 83cd, CG10407 and gustatory receptors 98b and 36b can be targeted, in particular, suppressed or antagonized. Also, the dopamine receptor *DopR2*, two octopamine receptors (*TyrR* and *oa2*) and the Nmda-receptor associated protein *Nmda1* In addition, altered fat deposition was observed in response modification of genes involved in glucose/lipid mobilization including fructose-1,6-bisphosphatase (fbp), the two members of the glycerol phosphate shuttle (CG31169 and *Gpo-1*), mitochondrial acyl-carrier protein 1 (*mtacp1*), ADP/ATP translocase 2 (*Ant2*), pyruvate carboxykinae (CG1516), and fatty-acid synthetase (fasn). Further examples of genes to be modified according to the present invention includes the *Drosophila* orthologues of glucagon (*akh*), the insulin receptor (*dInR*), as well as the downstream kinases PI3-kinase (*dPI3K*), ribosomal-S6-kinase (*dRSK*), the CREB-coactivator *dTORC,* and the critical TOR-signaling constituent *dTSC-1, Drosophila* homologues of the critical early adipogenic regulators NCOR1/2, Jag1/2, and TAK1, or the metabolic regulators CRTC1/2 and pyruvate carboxylase (PC). To all of the drosophila genes the present invention has identified human orthologs (table 1) that can be targeted by the inventive use of the therapeutic compounds.

According to the present invention all genes listed in table 1, those shown in example 1.2, in particular those illustrated in examples 1.3, 1.4 or 1.5, can be modified by therapeutic administration of suitable compounds. Preferred genes to be modified according to the present invention are those discussed above, as well as the LSD (Lipid Storage Droplet) and LPD (LiPid Depleted) genes, the *Drosophila* insulin like peptides (*Ilp's*), the glucagon homologue *akh* and its receptor akhr, as well as ad*ipose (adp), bubblegum (bbg),* and the *Drosophila* SREBP homologue, *HLH106, CG8451,* of course, as well as their human orthologues.

**Table 1: List of therapeutic compounds: Small Molecule: name of compound (see list of synonyms below), Human ortholog: human gene name of therapeutic target, Human Entrez ID: gene ID, Drosophila: ortholog Drosophila gene ID, Type: Type of interaction Molecule (O: Obesity - obesity pathway interaction, B: validated binding partner)**

| Small Molecules | Human Orthologs | Human Entrez ID | Drosophila | Type |
|---|---|---|---|---|
| (5- (2-methoxy-5-chloro-5-phenyl)furan-2-ylcarbonyl)guanidine | CYP3A4 | 1576 | CG9438 | O |
| (E)-4-(2-(2-(N-acetyl-N-(4-methoxybenzenesulfonyl)amino)s tilbazole)) | ABCB1 | 5243 | CG3879 | O |
| 1-oxide (melle-4)cyclosporin | FK6 | 8468 | CG375 | O |
| 1-(1-cyclohexylethylamino)-4-phenylphthalazine | CYP3A4 | 1576 | CG9438 | O |
| 1-(2-methoxyphenyl)-4-(4-(2-phthalimido)b utyl)piperazine | HTR1A | 3350 | CG7485 | O |
| 15-deoxyprostaglandin J2 | JUN,JUND,FLT1,KDR ,TFDP2,JUNB,APC,H GF | 3725,3727,2321,37 91,7029,3726,324, 3082 | CG2275,CG2275,CG8 222,CG8222,CG4654 ,CG2275,CG6193,CG 13744 | O |
| 17-(allylamino)-17-demethoxygeldanamycin | CDK6,HSP90AA1,CDK 4,HSPA4,DNAJB1,CC ND1,KDR,EIF2S1,AB CB1 | 1021,3320,1019,33 08,3337,595,3791, 1965,5243 | CG5072,CG1242,CG5 072,CG6603,CG1057 8,CG9096,CG8222,C G9946,CG3879 | O |
| 17-(dimethylaminoethylamino)-17-demethoxygeldanamycin | HSPH1,HSPA4,DNAJB 1,FGF2,HSP90AA1 | 10808,3308,3337,2 247,3320 | CG6603,CG6603,CG1 0578,CG4608,CG124 2 | O |
| 1-aminooxy-3-aminopropane | ODC1 | 4953 | CG8721 | O |
| 1-beta-D-arabinofuranosyl-Cytosine | JUN | 3725 | CG2275 | B |
| 1-carbamoyl-4-phenylpyrrolidone-2 | PSMD2 | 5708 | CG7762 | O |
| 1-Carboxyglutamic Acid | GLA | 2717 | CG5731 | B |
| 1-deoxygalactonojirimycin | GLA | 2717 | CG5731 | B |
| 1-hydroxymethyl droxymethylmidazolam | CYP3A4 | 1576 | CG9438 | O |
| 2-[2-[2-[2-[2-amino-3-(4-hydroxyphenyl)-1-oxopropyl]amino-1-oxoethyl]amino-1-oxoethyl]amino-1-oxo-3-phenylpropyl]amino-4-methylpentanoic acid | MME | 4311 | CG9565 | O |
| 25-desacetylrifabutin | CYP3A4 | 1576 | CG9438 | O |
| 2-AAF | RAC1 | 5879 | CG2248 | B |
| 2-AAF | ABCB1 | 5243 | CG3879 | O |
| 2-AG | RAC1 | 5879 | CG2248 | B |
| 2-AG | FAAH | 2166 | CG6007 | B |
| 2-AP | PLG | 5340 | CG13744 | O |
| 2-cyclopentyl-5-(5-isoquinolylsulfonyl)-6-nitro-1H-benzo(D)imidazole | HSPA5,ABCB1,FGF2 | 3309,5243,2247 | CG5436,CG3879,CG4 608 | B |
| 2-DG | CCND1,CCNB1,CDK4 | 595,891,1019 | CG9096,CG3510,CG5072 | O |
| 2-hydroxy-1-naphthylaldehyde isonicotinoyl hydrazone | CCND1 | 595 | CG9096 | B |
| 2-methoxyacetic acid [2-[2-[3-(1H-benzoimidazol-2-yl)propylmethylamino]ethyl]-6-fluoro-1-isopropyltetralin-2-yl] ester | CYP3A4 | 1576 | CG9438 | O |
| 2-methylarachidonyl-2'-fluoroethylamide | FGF2 | 2247 | CG4608 | B |
| 2-phenyl-4-oxohydroquinoline | CDC2 | 983 | CG5363 | B |
| 2-propylquinoline | ABCB1 | 5243 | CG3879 | O |
| 3-(5-((4-(methylsulfonyl)-1-piperazinyl)methyl)-1H-indole-2-yl)quinolin-2(1H)-one | KDR | 3791 | CG8222 | O |
| 3-AB | JUND,JUNB,JUN | 3727,3726,3725 | CG2275,CG2275,CG2 275 | B |
| 3'-deamino-3'-hydroxydoxorubicin | ABCB1 | 5243 | CG3879 | O |
| 3-HF | ABCB1 | 5243 | CG3879 | O |
| 3-Methoxyoestradiol | CYP3A4 | 1576 | CG9438 | O |
| 3-MF | OPRM1 | 4988 | CG7285 | B |
| 4-(4-(1-Amino-1-methylethyl)pheny 1)-2-(4-(2-morpholin-4-ylethyl)phenyla mino)pyrimidi ne-5-carbonitrile | KDR | 3791 | CG8222 | O |
| 4'-epidoxorubicin | CSE1L,ABCB1 | 1434,5243 | CG13281,CG3879 | O |
| 4-methyl-N-(3-(4-methylimidazol-1-yl)-5-(trifluoromethyl)phenyl)-3-((4-pyridin-3-ylpyrimidin-2-yl)amino)benz amide | CYP3A4 | 1576 | CG9438 | O |
| 4'-N-benzoylstaurosporine | CDK2 | 1017 | CG5363 | B |
| 4PBA | HSPA8,HSP90B1,HSP A5 | 3312,7184,3309 | CG31449,CG1242,CG 5436 | O |
| 4-PCB | CYP24A1 | 1591 | CG6042 | B |
| 5- (4'- (N-piperidinyl)phenylazo )indazole | CDK2,CCNA2 | 1017,890 | CG5363,CG3510 | B |
| 5-bromo-4-chloro-3-indolyl beta-galactoside | CYP3A4 | 1576 | CG9438 | O |
| 5-carboxamidotryptamine | HTR1A | 3350 | CG7485 | O |
| 5-demethylovalicin | METAP2 | 10988 | CG4008 | B |
| 5'-0-(((2-decanoylamino-3-phenylpropyloxycarbonyl)amino)sulf onyl)uridine | FYN | 2534 | CG7524 | B |
| 6'-N-methylsisomicin | FBN2 | 2201 | CG3936 | B |
| 7 HC | CCND1 | 595 | CG9096 | B |
| 7,8-BF | CYP3A4,CYP3A7 | 1576,1551 | CG9438,CG9438 | O |
| 7C3MT | GORASP1,CYP3A4 | 64689,1576 | CG7809,CG9438 | O |
| 7-hydroxystaurosporine | CDK4,CDK2,CDK6,CDC2,ABCB1,PDPK1,CC NA2 | 1019,1017,1021,983,5243,5170,890 | CG5072,CG5363,CG5072,CG5363,CG3879 ,CG1210,CG3510 | B |
| 7-ketocholesterol | GORASP1,DYNLL1 | 64689,8655 | CG7809,CG5450 | B |
| 8-Hydroxy-2-(di-n-propylamino)tetralin | HTR1A | 3350 | CG7485 | O |
| 8-hydroxyguanosine | RPS3,CDK4 | 6188,1019 | CG6779,CG5072 | O |
| 9-(2-hydroxy-3-nonyl)adenine | CCND1 | 595 | CG9096 | B |
| 9-beta-D-erythrofuranosyladenine | MTAP | 4507 | CG4802 | B |
| 9-CRA | FBN2,JUND,JUNB,HS PA4,JUN,CCND1,CYP 27B1,CYP24A1 | 2201,3727,3726,33 08,3725,595,1594, 1591 | CG3936,CG2275,CG2 275,CG6603,CG2275 ,CG9096,CG6042,CG 6042 | B |
| 9-hydroxy-risperidone | CYP4F3 | 4051 | CG3466 | B |
| A-300-I | JUNB,CDK4,JUND,NC OR1,DOCK8,OPRM1,E GR1,ABCB1,CCND1,J UN,GATA4 | 3726,1019,3727,96 11,81704,4988,195 8,5243,595,3725,2 626 | CG2275,CG5072,CG2 275,CG7951,CG1137 6,CG7285,CG7847,C G3879,CG9096,CG22 75,CG3992 | O |
| a-ADP | PLAT | 5327 | CG13744 | O |
| AAL 881 | KDR | 3791 | CG8222 | O |
| AATP | CYP3A4,CYP4F3,RAC 3 | 1576,4051,5881 | CG9438,CG3466,CG2 248 | B |
| AB 2 | GLA,JUN | 2717,3725 | CG5731,CG2275 | B |
| abacavir | KLHL1,HSPA4,ABCB1 | 57626,3308,5243 | CG17754,CG6603,CG 3879 | O |
| Abufene | ECE1 | 1889 | CG9565 | O |
| Acenocoumarol | NF2 | 4771 | CG14228 | B |
| Acetidin | ABCB1,SERPINB1 | 5243,1992 | CG3879,CG9456 | B |
| Acetorphan | MME | 4311 | CG9565 | O |
| acetoxymethyl-ester | EGR1 | 1958 | CG7847 | O |
| Aclarubicin | MYB | 4602 | CG9045 | B |
| ACNU | ABCB1 | 5243 | CG3879 | O |
| Acolen | CYP3A4 | 1576 | CG9438 | O |
| ACON | CYP3A4,SF4,CCND1, ABCB1,ABCB4 | 1576,57794,595,52 43,5244 | CG9438,CG31550,CG 9096,CG3879,CG387 9 | |
| actein | CCND1,CDK4 | 595,1019 | CG9096,CG5072 | O |
| acteoside | CCND2,CCND1,CCND3 | 894,595,896 | CG9096,CG9096,CG9 096 | B |
| Actihaemyl | PCSK5 | 5125 | CG10772 | O |
| Actosin | PDGFB | 5155 | CG7103 | O |
| adalimumab | PSMD12 | 5718 | CG1100 | O |
| Adanon | ABCB1,OPRM1,CYP3A 4 | 5243,4988,1576 | CG3879,CG7285,CG9 438 | O |
| ADMA | KDR | 3791 | CG8222 | O |
| ADMA | HSP90AA1 | 3320 | CG1242 | O |
| Adofeed | SLC5A8,PLG,SLC5A1 2 | 160728,5340,15996 3 | CG32669,CG13744,C G32669 | O |
| Adrenor | RAC1,TBXAS1,JUND, PDGFB,HSPB6,JUNB, HGF,FYN,MYH7,JUN | 5879,6916,3727,51 55,126393,3726,30 82,2534,4625,3725 | CG2248,CG3466,CG2 275,CG7103,CG4183 ,CG2275,CG13744,C G7524,CG2146,CG22 75 | B |
| Adrin | CCNE2,RAC1,FOXD3, HSP90AA1,CCND1,EC E1,ABCB1,PLAT,OPR M1 | 9134,5879,27022,3 320,595,1889,5243 ,5327,4988 | CG3938,CG2248,CG3 668,CG1242,CG9096 ,CG9565,CG3879,CG 13744,CG7285 | B |
| AEBSF | PLG,HSPA5,NCOR1 | 5340,3309,9611 | CG13744,CG5436,CG 7951 | B |
| AEE 788 | KDR | 3791 | CG8222 | O |
| AG 1879 | KDR | 3791 | CG8222 | O |
| ajoene | CCNB1 | 891 | CG3510 | B |
| Aklavin | QRSL1 | 55278 | CG6007 | B |
| alachlor | CYP3A4,CYP3A7 | 1576,1551 | CG9438,CG9438 | O |
| Alat | CYP3A4,ABCB1 | 1576,5243 | CG9438,CG3879 | O |
| ALDA | PSMD2 | 5708 | CG7762 | O |
| Aldara | TSC1 | 7248 | CG6147 | O |
| Aldocorten | SERPINC1,FOXD3,IGF1R ,ANGPTL1,WNK1,WNK 4,CPA1,EGR1,CYP4F 3 | 462,27022,3480,90 68,65125,65266,13 57,1958,4051 | CG9456,CG3668,CG1 842,CG5550,CG7177 ,CG7177,CG14820,C G7847,CG3466 | B |
| alemtuzumab | CCND2 | 894 | CG9096 | B |
| Alendronate | CYP27A1,BEST1 | 1593,7439 | CG6042,CG6264 | B |
| Alfarol | BEST1 | 7439 | CG6264 | B |
| Alfentanil | OPRM1,CYP3A4 | 4988,1576 | CG7285,CG9438 | O |
| ALIMTA | TBXAS1 | 6916 | CG3466 | B |
| aliskiren | MAPT,ATP6AP2,CYP3 | 4137,10159,1576 | CG31057,CG8444,CG | O |
| | A4 | | 9438 | |
| Alli | CPB2 | 1361 | CG14820 | B |
| Allnal | HSPA4 | 3308 | CG6603 | O |
| allylamino-demethoxy-geldanamycin | HSP90AA1 | 3320 | CG1242 | O |
| alpha-neoendorphin | OPRK1 | 4986 | CG7285 | B |
| alternagin-C | KDR | 3791 | CG8222 | O |
| Alvesco | CYP3A4 | 1576 | CG9438 | O |
| alvimopan | OPRM1 | 4988 | CG7285 | B |
| Am 80 | JUNB,JUN,JUND | 3726,3725,3727 | CG2275,CG2275,CG2 275 | B |
| Amatin | SMARCA4,SMARCA2 | 6597,6595 | CG5942,CG5942 | B |
| AMCHA | PLG | 5340 | CG13744 | O |
| AMD 070 | CYP3A4 | 1576 | CG9438 | O |
| amentoflavone | GORASP1 | 64689 | CG7809 | B |
| Amiloride | CCNB1,PDPK1,ODC1, PLG | 891,5170,4953,534 0 | CG3510,CG1210,CG8 721,CG13744 | O |
| Amine BB | SERPINB1 | 1992 | CG9456 | B |
| Amiodarone | SLC5A8 | 160728 | CG32669 | O |
| Amiodarone | SLC5A8,CYP3A4 | 160728,1576 | CG32669,CG9438 | O |
| amlexanox | FGF1 | 2246 | CG4608 | B |
| Amlodipine | PLAT,CYP3A4,JAK2, CCND1,ABCB1 | 5327,1576,3717,59 5,5243 | CG13744,CG9438,CG 1594,CG9096,CG387 9 | O |
| Ammo | MBL2 | 4153 | CG7763 | B |
| Ampicillin | PLG | 5340 | CG13744 | O |
| amprenavir | ABCB1,CYP3A4 | 5243,1576 | CG3879,CG9438 | O |
| amrubicin | ABCB1 | 5243 | CG3879 | O |
| AMSA | TOP2B | 7155 | CG10223 | B |
| amsonic acid | CDC2 | 983 | CG5363 | B |
| Anaboleen | CYP24A1,SNAI2,CYP 27B1,ABCB1,CCND1, FGF7,CCNA2,IGF1R, JUN | 1591,6591,1594,52 43,595,2252,890,3 480,3725 | CG6042,CG3758,CG6 042,CG3879,CG9096 ,CG4608,CG3510,CG 1842,CG2275 | B |
| Anandamide | CDK2,HTR1A,FAAH | 1591,6591,1594,52 43,595,2252,890,3 480,3725,1017,335 0,2166 | CG6042,CG3758,CG6 042,CG3879,CG9096 ,CG4608,CG3510,CG 1842,CG2275,CG536 3,CG7485,CG6007 | B |
| Anco | ABCB1,CYP27A1,GOR ASP1,HSPA8,MAPT | 5243,1593,64689,3 312,4137 | CG3879,CG6042,CG7 809,CG31449,CG310 57 | B |
| and-carboxyfluoresceindiacetatesuccinimidylester | CCND1 | 595 | CG9096 | B |
| Androstenediol | KSR1 | 8844 | CG2899 | B |
| anilinyl | FLT1 | 2321 | CG8222 | O |
| Anisomycin | JUN,PSMD12,EGR1 | 3725,5718,1958 | CG2275,CG1100,CG7 847 | O |
| ANSA | HSP90AA1 | 3320 | CG1242 | O |
| antibiotic G 418 | HSPB2,CCNA2,HSPB1 | 3316,890,3315 | CG14207,CG3510,CG 14207 | B |
| antibiotic H107 | PSMD12 | 5718 | CG1100 | O |
| antineoplaston A10 | ATF2,MAPT,JUN,PDC D2,GSK3B,ABCB1,GL A,MAGT1,HGF | 1386,4137,3725,51 34,2932,5243,2717 ,84061,3082 | CG30420,CG31057,C G2275,CG326,CG262 1,CG3879,CG5731,C G7830,CG13744 | O |
| Antizol | P11 | 8909 | CG2145 | O |
| APAP | CYP3A4 | 1576 | CG9438 | O |
| APDC | MAGT1,JUNB,HGF,JU N,DNAJB1,IGF1R,GO RASP1,ABCB1,JUND | 84061,3726,3082,3 725,3337,3480,646 89,5243,3727 | CG7830,CG2275,CG1 3744,CG2275,CG105 78,CG1842,CG7809, CG3879,CG2275 | B |
| Aphidicolin | POLS | 11044 | CG11265 | B |
| Aphidicolin | POLS,JUN | 11044,3725 | CG11265,CG2275 | B |
| Aphloiol | GORASP1 | 64689 | CG7809 | B |
| apicidin | ABCB1,CDK2,CDK4,C CND1,CCND3 | 5243,1017,1019,59 5,896 | CG3879,CG5363,CG5 072,CG9096,CG9096 | B |
| Apigenin | CDC2,ABCB1,JUNB,J UN,GORASP1,CCND1, JUND,FBRS,CDK4,CD K2,CCNA2,HGF,CCNB 1 | 983,5243,3726,372 5,64689,595,3727, 64319,1019,1017,8 90,3082,891 | CG5363,CG3879,CG2 275,CG2275,CG7809 ,CG9096,CG2275,CG 9056,CG5072,CG536 3,CG3510,CG13744, CG3510 | B |
| aplidine | JUNB,JUN,RAC1,CYP 4F3,CYP3A4,JUND | 3726,3725,5879,40 51,1576,3727 | CG2275,CG2275,CG2 248,CG3466,CG9438 ,CG2275 | B |
| aprepitant | CYP3A4 | 1576 | CG9438 | O |
| APRL | SSTR4,PCSK7,PIK3C 2A,SSTR1,HTR1A,FY N,CSE1L,TYK2,JUND ,JUNB,METAP2,JUN | 6754,9159,5286,67 51,3350,2534,1434 ,7297,3727,3726,1 0988,3725 | CG7285,CG10772,CG 11621,CG7285,CG74 85,CG7524,CG13281 ,CG1594,CG2275,CG 2275,CG4008,CG227 5 | B |
| Apsor | CYP24A1 | 1591 | CG6042 | B |
| aptiganel | JUN | 3725 | CG2275 | B |
| Aralen | NUP214,JUND,ABCB1 ,JUNB,JUN,PLAT | 8021,3727,5243,37 26,3725,5327 | CG3820,CG2275,CG3 879,CG2275,CG2275 ,CG13744 | O |
| arctiin | CCND1 | 595 | CG9096 | B |
| Arecoline | CDC2 | 983 | CG5363 | B |
| Areether | CYP3A4 | 1576 | CG9438 | O |
| argatroban | SERPINC1 | 462 | CG9456 | B |
| aripiprazole | HTR1A,CYP3A4 | 3350,1576 | CG7485,CG9438 | O |
| Armor | NPDC1 | 56654 | CG30420 | B |
| Artein | CDK2,LPA,PLAT,CCN D3,ABCB1,CCND1,JU N,CYP4F2,CYP3A4 | 1017,4018,5327,89 6,5243,595,3725,8 529,1576 | CG5363,CG13744,CG 13744,CG9096,CG38 79,CG9096,CG2275, CG3466,CG9438 | O |
| ASTA | CNOT6,SERPINB6,CY P27A1 | 57472,5269,1593 | CG31137,CG9456,CG 6042 | B |
| astatine | HSPA4,MAPT,PLAT,S LC5A5 | 3308,4137,5327,65 28 | CG6603,CG31057,CG 13744,CG32669 | O |
| Astemizole | CYP3A4 | 1576 | CG9438 | O |
| atazanavir | CYP3A4,ABCB1 | 1576,5243 | CG9438,CG3879 | O |
| Atorel | HTR1A,SSTR4,SSTR1 | 3350,6754,6751 | CG7485,CG7285,CG7 285 | B |
| atorvastatin | RAC1,CYP3A4,SERPI NC1,JUN,CSE1L,ABC B1,JUNB,JUND,PLAT | 5879,1576,462,372 5,1434,5243,3726, 3727,5327 | CG2248,CG9438,CG9 456,CG2275,CG1328 1,CG3879,CG2275,C G2275,CG13744 | O |
| Atosil | ABCB1 | 5243 | CG3879 | O |
| Atovaquone | CYP3A4 | 1576 | CG9438 | O |
| ATRA | PLAT,CCNB1,PAX6,M APT,CYP3A7,OPRK1, NCOR1,KHDRBS1,PSM D6,HSPB1,SLC5A5,K DR,JUNB,CCND2,MYB L2,TOP2B,TIMM8A,T HOC4,FGR,CDK4,JUN D,CYP27A1,FGF4,PS MD2,PSMD12,SERPIN C1,RAC3,RAC1,AXIN 1,CDK2,CDC2,CCND3 ,CHAT,CDK6,GATA4, FGF2,JUN,ATF2,APC ,CCND1,ABCB1,CCNA 1,CYP4A11,HGF,FGF 9,FGFS,FBN1,GORAS P1,GLA,HSP90B1,CY P4B1,EGR1,MME,FGF 12,FCN2 | 5327,891,5080,413 7,1551,4986,9611, 10657,9861,3315,6 528,3791,3726,894 ,4605,7155,1678,1 0189,2268,1019,37 27,1593,2249,5708 ,5718,462,5881,58 79,8312,1017,983, 896,1103,1021,262 6,2247,3725,1386, 324,595,5243,8900 ,1579,3082,2254,2 250,2200,64689,27 17,7184,1580,1958 ,4311,2257,2220 | CG13744,CG3510,CG 11186,CG31057,CG9 438,CG7285,CG7951 ,CG4816,CG5378,CG 14207,CG32669,CG8 222,CG2275,CG9096 ,CG9045,CG10223,C G1728,CG1101,CG75 24,CG5072,CG2275, CG6042,CG4608,CG7 762,CG1100,CG9456 ,CG2248,CG2248,CG 7926,CG5363,CG536 3,CG9096,CG12345, CG5072,CG3992,CG4 608,CG2275,CG3042 0,CG6193,CG9096,C G3879,CG3510,CG34 66,CG13744,CG4608 ,CG4608,CG3936,CG 7809,CG5731,CG124 2,CG9438,CG7847,C G9565,CG4608,CG55 5 | O |
| Auluton | MAFB,EGR1 | 9935,1958 | CG10034,CG7847 | O |
| aureobasidin A | ABCB1 | 5243 | CG3879 | O |
| Aurothioglucose | PARD6A,GORASP1 | 50855,64689 | CG5884,CG7809 | B |
| AuTM | RAC1,PARD6A | 5879,50855 | CG2248,CG5884 | B |
| Axert | CYP3A4 | 1576 | CG9438 | O |
| axitinib | KDR | 3791 | CG8222 | O |
| Axsain | FOXD3,GORASP1,PLA T,ABCB1,MAPT,FCN2 ,JUND,JUNB,JUN,MM E | 27022,64689,5327, 5243,4137,2220,37 27,3726,3725,4311 | CG3668,CG7809,CG1 3744,CG3879,CG310 57,CG555,CG2275,C G2275,CG2275,CG95 65 | O |
| azacyclonol | CYP3A4 | 1576 | CG9438 | O |
| Azadc | CDK6,DYNLL1,TSC1, ABCB1,FYN,ADAMTS1,JUP,MTAP,RAC2 | 1021,8655,7248,52 43,2534,9510,3728,4507,5880 | CG5072,CG5450,CG6 147,CG3879,CG7524,CG14869,CG11579, CG4802,CG2248 | B |
| azamulin | CYP3A4 | 1576 | CG9438 | O |
| azanediyl group | ABCB1 | 5243 | CG3879 | O |
| azaspirane | FGF2,JAK2 | 2247,3717 | CG4608,CG1594 | B |
| azelastine | CYP3A4 | 1576 | CG9438 | O |
| azelnidipine | JUNB,JUN,CYP3A4,J UND | 3726,3725,1576,37 27 | CG2275,CG2275,CG9 438,CG2275 | B |
| azidoprazosin | ABCB1 | 5243 | CG3879 | O |
| Aziran | SERPINB1,FLT4,PCBD1 | 1992,2324,5092 | CG9456,CG8222,CG1 963 | O |
| Azithromycin | JUN,JUND,JUNB | 3725,3727,3726 | CG2275,CG2275,CG2 275 | B |
| Azor | CYP3A4,CYP3A43 | 1576,64816 | CG9438,CG9438 | O |
| Azur A | MAPT | 4137 | CG31057 | B |
| Azure B | MAPT | 4137 | CG31057 | B |
| BA (VAN) | CYP3A4,CYP3A7 | 1576,1551 | CG9438,CG9438 | O |
| Baclofen | HTR1A | 3350 | CG7485 | O |
| Bagren | CYP3A4 | 1576 | CG9438 | O |
| baicalein | IGF1R,CCNB1,CDK4, CDC2,PLAT | 3480,891,1019,983 ,5327 | CG1842,CG3510,CG5 072,CG5363,CG1374 4 | O |
| Barnidipine | ABCB1 | 5243 | CG3879 | O |
| BAY 11-7085 | CCND1,GORASP1 | 595,64689 | CG9096,CG7809 | B |
| Beflavin | CYP4F3,SF4 | 4051,57794 | CG3466,CG31550 | B |
| Benidipine | ABCB1,CYP3A4 | 5243,1576 | CG3879,CG9438 | O |
| benzoylstaurosporine | KDR | 3791 | CG8222 | O |
| beraprost | PLAT | 5327 | CG13744 | O |
| Berbamine | HSP90AA1 | 3320 | CG1242 | O |
| berberine | ABCB1,JUNB,BEST1, JUND,JUN,CYP3A4 | 5243,3726,7439,37 27,3725,1576 | CG3879,CG2275,CG6 264,CG2275,CG2275 ,CG9438 | O |
| bergamottin | CYP3A4 | 1576 | CG9438 | O |
| bergaptol | CYP3A4 | 1576 | CG9438 | O |
| BESTATIN | CCND1 | 595 | CG9096 | B |
| betacitronellol | ABCB1 | 5243 | CG3879 | O |
| beta-eudesmol | FGF2 | 2247 | CG4608 | B |
| beta-funaltrexamine | OPRM1 | 4988 | CG7285 | B |
| beta-gamma-iminotriphosphate | RAC1 | 5879 | CG2248 | B |
| bexarotene | PSMD12,CCND3,CCND 1 | 5718,896,595 | CG1100,CG9096,CG9 096 | B |
| Bezafibrate | ABCB4,CYP3A4 | 5244,1576 | CG3879,CG9438 | O |
| BI D1870 | RPS6KA3,RPS6KA6,R PS6KA1,RPS6KA2 | 6197,27330,6195,6 196 | CG17596,CG17596,C G17596,CG17596 | O |
| biapigenin | CYP3A4 | 1576 | CG9438 | O |
| BIBF 1000 | FGF2 | 2247 | CG4608 | B |
| BIBW 22 | ABCB1 | 5243 | CG3879 | O |
| bifeprunox | HTR1A | 3350 | CG7485 | O |
| Bisoprolol | CYP3A4 | 1576 | CG9438 | O |
| Bisphenol A-Glycidyl Methacrylate | CLTB | 1212 | CG6948 | B |
| bizelesin | SERPINB3 | 6317 | CG9456 | B |
| Bleomycin | FGF7,TSC1,ABCB1,H SPA1A | 2252,7248,5243,33 03 | CG4608,CG6147,CG3 879,CG5436 | O |
| BM 41.440 | PCSK7 | 9159 | CG10772 | O |
| BMS 310705 | CYP3A4 | 1576 | CG9438 | O |
| BMS-262084 | ABCB1 | 5243 | CG3879 | O |
| BMS453 | CDK2 | 1017 | CG5363 | B |
| BMS-470539 | TUBB3 | 10381 | CG3401 | B |
| BMY 7378 | HTR1A | 3350 | CG7485 | O |
| Borreliaburgdorferi | PLG | 5340 | CG13744 | O |
| bortezomib | CYP27A1,JUND,JUN, TUBA1B,HSPA1A,JUNB, PSMB8,PSMB5,ABCB1 ,CCND1,EIF2S1,MAG T1,BAG3,HSPA4,HSP A5,HSPB1,HSPB2,HS P90AA1,GORASP1 | 1593,3727,3725,10 376,3303,3726,569 6,5693,5243,595,1 965,84061,9531,33 08,3309,3315,3316 ,3320,64689 | CG6042,CG2275,CG2 275,CG1913,CG5436 ,CG2275,CG12323,C G12323,CG3879,CG9 096,CG9946,CG7830 ,CG32130,CG6603,C G5436,CG14207,CG1 4207,CG1242,CG780 9 | B |
| bosentan | CYP3A4 | 1576 | CG9438 | O |
| bosutinib | CDK2 | 1017 | CG5363 | B |
| Bo-Xan | ABCB1,IK | 5243,3550 | CG3879,CG18005 | B |
| Brefeldin A | JUN,CCNB1,JUND,HS PA5,JUNB,YIF1A | 3725,891,3727,330 9,3726,10897 | CG2275,CG3510,CG2 275,CG5436,CG2275 ,CG5484 | O |
| bremazocine | OPRK1,OPRM1 | 4986,4988 | CG7285,CG7285 | B |
| bryostatin | JUNB,ABCB1,JUN,JU ND | 3726,5243,3725,37 27 | CG2275,CG3879,CG2 275,CG2275 | B |
| Budesonide | CYP3A4,ABCB1 | 1576,5243 | CG9438,CG3879 | O |
| bufalin | CYP24A1,MYB | 1591,4602 | CG6042,CG9045 | B |
| Bumetanide | BEST1 | 7439 | CG6264 | B |
| Bupivacaine | LPA | 4018 | CG13744 | O |
| Buprenorphine | CYP3A4,OPRM1,OPRK 1 | 1576,4988,4986 | CG9438,CG7285,CG7 285 | B |
| Buspirone | HTR1A,CYP3A4 | 3350,1576 | CG7485,CG9438 | O |
| Buthionine | ABCB1 | 5243 | CG3879 | O |
| Buthionine Sulfoximine | SERPINB6 | 5269 | CG9456 | B |
| cabergoline | KDR | 3791 | CG8222 | O |
| CACP | PCBD1,PCSK5,PPP1R 15A,ZNHIT1,JUNB,J UN,KLHL1,HSPA5,PD XP,JUP,GORASP1,RP SA,SERPINB3,EIF4G 1,SNAI2,SERPINB6, EGR1,EIF2S1,FLT1, EZR,JUND,CCND1,HG F,DFFA,HMGB2,CSE1 L,HSPB1,IGF1R,HSP B2,HSPA1A,HSP90AA 1,JAK2 | 5092,5125,23645,1 0467,3726,3725,57 626,3309,57026,37 28,64689,3921,631 7,1981,6591,5269, 1958,1965,2321,74 30,3727,595,3082, 1676,3148,1434,33 15,3480,3316,3303 ,3320,3717 | CG1963,CG10772,CG 3825,CG31917,CG22 75,CG2275,CG17754 ,CG5436,CG4755,CG 11579,CG7809,CG14 792,CG9456,CG1081 1,CG3758,CG9456,C G7847,CG9946,CG82 22,CG10701,CG2275 ,CG9096,CG13744,C G8357,CG17921,CG1 3281,CG14207,CG18 42,CG14207,CG5436 ,CG1242,CG1594 | B |
| Calcijex | CYP24A1,PSMD12,KS R1,CYP27B1 | 1591,5718,8844,15 94 | CG6042,CG1100,CG2 899,CG6042 | B |
| Calcimycin | EGR1,JUN,CPA1,HSP 90B1,HSPA5,HSPA4, DYNLL1 | 1958,3725,1357,71 84,3309,3308,8655 | CG7847,CG2275,CG1 4820,CG1242,CG543 6,CG6603,CG5450 | B |
| calphostin C | KDR,FGF13,FGF2,JU ND,ADAMTS1,JUNB,J UN | 3791,2258,2247,37 27,9510,3726,3725 | CG8222,CG4608,CG4 608,CG2275,CG1486 9,CG2275,CG2275 | B |
| Calyculin | EGR1,GSK3B,ETV5,M APT,PPA1 | 1958,2932,2119,41 37,5464 | CG7847,CG2621,CG6 892,CG31057,CG463 4 | O |
| Camptothecin | GSK3B,MYB,CCNA2,R PSA,HGF,MAPT,ABCB 1 | 2932,4602,890,392 1,3082,4137,5243 | CG2621,CG9045,CG3 510,CG14792,CG137 44,CG31057,CG3879 | O |
| candesartan | BEST1 | 7439 | CG6264 | B |
| candoxatril | MME | 4311 | CG9565 | O |
| candoxatrilat | MME | 4311 | CG9565 | O |
| Canef | CPB2,CYP3A4,LPA,P PA1,HGF | 1361,1576,4018,54 64,3082 | CG14820,CG9438,CG 13744,CG4634,CG13 744 | O |
| CAPE | CCND1,ABCB1 | 595,5243 | CG9096,CG3879 | O |
| capsanthin | ABCB1 | 5243 | CG3879 | O |
| capsazepine | PCSK7 | 9159 | CG10772 | O |
| Carbamazepine | JUND,JUNB,CYP4F3, CYP3A4,ABCB1,JUN | 3727,3726,4051,15 76,5243,3725 | CG2275,CG2275,CG3 466,CG9438,CG3879 ,CG2275 | B |
| carbamic acid | FAAH,ABCB1 | 2166,5243 | CG6007,CG3879 | O |
| Cardiolipins | RPS6KA3,FOXD3 | 6197,27022 | CG17596,CG3668 | B |
| Cardioplegic Solutions | PIK3C2A | 5286 | CG11621 | O |
| carebastine | ABCB1 | 5243 | CG3879 | O |
| carfentanil | OPRM1 | 4988 | CG7285 | B |
| Carisoprodol | DDX3X | 1654 | CG9748 | O |
| CARNOSOL | CCNB1 | 891 | CG3510 | B |
| carvacrol | JUN,JUNB,JUND | 3725,3726,3727 | CG2275,CG2275,CG2 275 | B |
| carvedilol | ABCB1 | 5243 | CG3879 | O |
| Casodex | CYP4F3,TMSB4X,EZR | 4051,7114,7430 | CG3466,CG4944,CG1 0701 | B |
| caspofungin | CYP3A4 | 1576 | CG9438 | O |
| casticin | CDC2,CCNA2 | 983,890 | CG5363,CG3510 | B |
| catechins | HGF,IK,POLS | 3082,3550,11044 | CG13744,CG18005,C G11265 | B |
| CD 437 | JUNB,HSPA5,PPP1R1 5A,GORASP1,JUN,JU ND,TXNIP | 3726,3309,23645,6 4689,3725,3727,10 628 | CG2275,CG5436,CG3 825,CG7809,CG2275 ,CG2275,CG18745 | B |
| Cefotaxime | CYP27A1 | 1593 | CG6042 | B |
| Ceftazidime | CYP27A1 | 1593 | CG6042 | B |
| celecoxib | ABCB1,PDPK1,CDK2, HSPA5,CCND1,GORAS P1,CDK4 | 5243,5170,1017,33 09,595,64689,1019 | CG3879,CG1210,CG5 363,CG5436,CG9096 ,CG7809,CG5072 | O |
| Celiprolol | ABCB1 | 5243 | CG3879 | O |
| CEP 701 | CYP3A4, (JAK2) | 1576, (3717) | CG9438, (CG1594) | O |
| cephalomannine | CYP3A4 | 1576 | CG9438 | O |
| cerivastatin | CYP3A4,CDK2,CSE1L | 1576,1017,1434 | CG9438,CG5363,CG1 3281 | O |
| Cerulenin | RPSA | 3921 | CG14792 | O |
| Cetirizine | ABCB1 | 5243 | CG3879 | O |
| Cetirizine | TSC1 | 7248 | CG6147 | O |
| cetuximab | KDR | 3791 | CG8222 | O |
| CGS 26303 | ECE1 | 1889 | CG9565 | O |
| CGS 35066 | ECE1 | 1889 | CG9565 | O |
| CGS 35601 | MME | 4311 | CG9565 | O |
| Chloramphenicol | CLTB | 1212 | CG6948 | B |
| chloroprocaine | PHC2 | 1912 | CG18414 | O |
| Chlorzoxazone | CYP4F3 | 4051 | CG3466 | B |
| chymostatin | CAPN2,CAPNS1,FGF2 | 824,826,2247 | CG8107,CG8107,CG4 608 | B |
| cicaprost | CCNA2 | 890 | CG3510 | B |
| ciglitazone | GORASP1,CCND1,NUPR1 | 64689,595,26471 | CG7809,CG9096,CG6 770 | B |
| Ciguatoxins | CYP27A1 | 1593 | CG6042 | B |
| Cillora | KLHL1 | 57626 | CG17754 | O |
| cilostazol | CYP3A4,LPA | 1576,4018 | CG9438,CG13744 | O |
| Cimetidine | ABCB1,CYP3A4,CYP3 A7,CYP4F3 | 5243,1576,1551,40 51 | CG3879,CG9438,CG9 438,CG3466 | B |
| CINK4 | CCND1 | 595 | CG9096 | B |
| Cipol N | HSPB1,JUNB,PDGFB, KLHL1,NUP214,PLAT ,PPA1,GORASP1,JUN D,PPIB,SERPINC1,C CND2,CYP3A4,DYNLL 1,FOXD3,PPIF,CYP2 7A1,ABCB1,HSPA5,H SPB2,JUN,HGF,ATP2 A3,PPIA,HEY1 | 3315,3726,5155,57 626,8021,5327,546 4,64689,3727,5479 ,462,894,1576,865 5,27022,10105,159 3,5243,3309,3316, 3725,3082,489,5478,23462 | CG14207,CG2275,CG 7103,CG17754,CG38 20,CG13744,CG4634 ,CG7809,CG2275,CG 2852,CG9456,CG909 6,CG9438,CG5450,C G3668,CG2852,CG6042,CG3879,CG5436, CG14207,CG2275,CG 13744,CG32451,CG2 852,CG11194 | B |
| Ciprofloxacin | CYP27A1,NF2 | 1593,4771 | CG6042,CG14228 | B |
| Ciprol | CIDEA | 1149 | CG1975 | O |
| Cisapride | CYP3A4,HTR4 | 1576,3360 | CG9438,CG6919 | O |
| Citalopram | CYP3A4 | 1576 | CG9438 | O |
| Citox | CYP3A4 | 1576 | CG9438 | O |
| CJ-15161 | OPRK1 | 4986 | CG7285 | B |
| Cladribine | TSC1 | 7248 | CG6147 | O |
| clavosine B | PPA1 | 5464 | CG4634 | O |
| clavulone II | CCND1 | 595 | CG9096 | B |
| clobazam | CYP3A4 | 1576 | CG9438 | O |
| Clodronic Acid | BEST1 | 7439 | CG6264 | B |
| Clofazimine | ABCB1 | 5243 | CG3879 | O |
| Clofibric Acid | ABCB5,ABCB4,CYP3A 4 | 340273,5244,1576 | CG3879,CG3879,CG9 438 | O |
| Clomipramine | CYP3A4 | 1576 | CG9438 | O |
| Clonazepam | CYP3A4,CYP4F3 | 1576,4051 | CG9438,CG3466 | B |
| Clonidine | MAPT,TBXAS1 | 4137,6916 | CG31057,CG3466 | B |
| clonidinedisplacing substance | FOXD3 | 27022 | CG3668 | B |
| clopidogrel | ABCB1,CYP3A4,SERP INC1 | 5243,1576,462 | CG3879,CG9438,CG9 456 | B |
| clotiazepam | CYP3A4 | 1576 | CG9438 | O |
| Clozapine | CCND1,PSMD12,AXIN 1,GSK3B,PPA1,HTR1 A,CYP4F3,CYP3A4 | 595,5718,8312,293 2,5464,3350,4051, 1576 | CG9096,CG1100,CG7 926,CG2621,CG4634 ,CG7485,CG3466,CG 9438 | O |
| CMDBS 25 | FGF2,FGF13 | 2247,2258 | CG4608,CG4608 | B |
| Co 2-1970 | P11 | 8909 | CG2145 | O |
| Colchicine | MAPT,MAF,ABCB1,CY P3A4,PPIF,WNK3 | 4137,4094,5243,15 76,10105,65267 | CG31057,CG10034,C G3879,CG9438,CG28 52,CG7177 | O |
| Cordanum | ABCB1 | 5243 | CG3879 | O |
| cornuside | GORASP1 | 64689 | CG7809 | B |
| costunolide | KDR | 3791 | CG8222 | O |
| Cotinine | ABCB1 | 5243 | CG3879 | O |
| Cotrim | CYP4F3 | 4051 | CG3466 | B |
| coumarin | CSE1L | 1434 | CG13281 | O |
| coumarin | CSE1L,CYP3A4 | 1434,1576 | CG13281,CG9438 | O |
| coumarin | CSE1L,CYP3A4,SERP INC1 | 1434,1576,462 | CG13281,CG9438,CG 9456 | B |
| coumermycins | JAK1 | 3716 | CG1594 | B |
| CP 31398 | TSC1 | 7248 | CG6147 | O |
| CRA 026440 | NCOR2 | 9612 | CG7951 | B |
| CREBtide | RPS6KA3 | 6197 | CG17596 | O |
| Crestor | CYP3A4 | 1576 | CG9438 | O |
| Crodacid | PSMD12 | 5718 | CG1100 | O |
| cryptotanshinone | CYP3A4 | 1576 | CG9438 | O |
| cryptoxanthin | CCND1 | 595 | CG9096 | B |
| Cyclandelate | SERPINC1 | 462 | CG9456 | B |
| cyclohexanecarboxylic acid (2-(4-(2-bromo-5-methoxybenzyl)piperazin -1-yl)ethyl)-(2-trifluoromethoxyphenyl)amide | HTR1A | 3350 | CG7485 | O |
| cyclopamine | SHH,CDK2 | 6469,1017 | CG4637,CG5363 | B |
| cyclopiazonic acid | CYP3A4,ATP2A2 | 1576,488 | CG9438,CG32451 | B |
| cycloprodigiosin | JUNB,JUN,JUND | 3726,3725,3727 | CG2275,CG2275,CG2 275 | B |
| cyclosporin G | CYP4F3,ABCB1 | 4051,5243 | CG3466,CG3879 | O |
| cyclotheonamide A | PLAT | 5327 | CG13744 | O |
| Cyproterone Acetate | SERPINC1 | 462 | CG9456 | B |
| Cystamine | KDR,RAC1,IK | 3791,5879,3550 | CG8222,CG2248,CG1 8005 | B |
| cytarabine | JUN | 3725 | CG2275 | B |
| D 21266 | ABCB1 | 5243 | CG3879 | O |
| DA 8159 | MMEL1 | 79258 | CG9565 | O |
| DABS | CRKL | 1399 | CG1587 | B |
| Dacarbazine | CYP4F3 | 4051 | CG3466 | B |
| DADA | MCRS1 | 10445 | CG1135 | B |
| DADSO | JUND,JUNB,JUN,CYP 3A4 | 3727,3726,3725,15 76 | CG2275,CG2275,CG2 275,CG9438 | O |
| daidzein | JUNB,JUN,JUND,MET AP2,MAP2 | 3726,3725,3727,10 988,4133 | CG2275,CG2275,CG2 275,CG4008,CG3105 7 | B |
| Dalteparin | HGF | 3082 | CG13744 | O |
| D-AM | FOXD3 | 27022 | CG3668 | B |
| danaproid | SERPINC1 | 462 | CG9456 | B |
| Danazol | LPA | 4018 | CG13744 | O |
| Dapsone | CYP4F3 | 4051 | CG3466 | B |
| Daral | CYP3A4,CYP24A1,TS C1,JUND,JUN,CYP27 A1,PDGFA,CYP27B1, JUNB | 1576,1591,7248,37 27,3725,1593,5154 ,1594,3726 | CG9438,CG6042,CG6 147,CG2275,CG2275 ,CG6042,CG7103,CG 6042,CG2275 | B |
| Darifenacin | CYP4F3 | 4051 | CG3466 | B |
| dasatinib | CRKL,src,CYP3A4,N UP214,ABCB1 | 1399,6714,1576,80 21,5243 | CG1587,CG7524,CG9 438,CG3820,CG3879 | O |
| DAU 6285 | HTR4 | 3360 | CG6919 | O |
| Daunorubicin | ABCB1 | 5243 | CG3879 | O |
| dauricine | ABCB1 | 5243 | CG3879 | O |
| Dayfen | SERPINB4,CNOT6,SERPI NB6 | 6318,57472,5269 | CG9456,CG31137,CG 9456 | B |
| Dddd-PGD2 | HSPA4,HSPA1A,HSPA 5 | 3308,3303,3309 | CG6603,CG5436,CG5 436 | O |
| decursin | CCND1 | 595 | CG9096 | B |
| Deferoxamine | JUNB,PLG,CDC2,CCN D1,JUND,CCNA2,HGF ,JUN,CDK2 | 3726,5340,983,595 ,3727,890,3082,37 25,1017 | CG2275,CG13744,CG 5363,CG9096,CG227 5,CG3510,CG13744, CG2275,CG5363 | B |
| DEHP | ABCB1 | 5243 | CG3879 | O |
| dehydroaripiprazole | CYP3A4 | 1576 | CG9438 | O |
| Dehydroepiandrosterone | GATA6 | 2627 | CG3978 | B |
| Sulfate dehydroxymeth droxymethylepoxyquinomicin | ABCB1,GORASP1,CCN D1 | 5243,64689,595 | CG3879,CG7809,CG9 096 | B |
| Delavirdine | KLHL1,CYP3A4 | 57626,1576 | CG17754,CG9438 | O |
| delphinidin | JUN,KDR | 3725,3791 | CG2275,CG8222 | O |
| delta-1-pyrroline-5-carboxylate | PPA1 | 5464 | CG4634 | O |
| delta8-THC | RPS6KA2,JUND,CDC2 ,RPS6KA3,RPS6KA1 | 6196,3727,983,619 7,6195 | CG17596,CG2275,CG 5363,CG17596,CG17 596 | O |
| Denagard | CYP3A4 | 1576 | CG9438 | O |
| denbinobin | CRKL,GORASP1 | 1399,64689 | CG1587,CG7809 | B |
| denosumab | BEST1 | 7439 | CG6264 | B |
| deoxyhypusine | EIF5 | 1983 | CG9177 | B |
| deoxyverrucosidin | HSPA5 | 3309 | CG5436 | O |
| Depas | CYP3A4,CSE1L | 1576,1434 | CG9438,CG13281 | O |
| dephosphonocalyculin A | PPA1 | 5464 | CG4634 | O |
| Deproceptin | OPRM1 | 4988 | CG7285 | B |
| deramciclane | CYP3A4 | 1576 | CG9438 | O |
| dermorphin | OPRM1 | 4988 | CG7285 | B |
| desethylchloroquine | CYP3A4 | 1576 | CG9438 | O |
| desloratadine | ABCB1 | 5243 | CG3879 | O |
| desmethylazel astine | CYP3A4 | 1576 | CG9438 | O |
| Desmethyldeprenyl | CYP3A4 | 1576 | CG9438 | O |
| desmethyl-tamoxifen | CYP3A4 | 1576 | CG9438 | O |
| DEVD-CHO | MST1 | 4485 | CG13744 | O |
| dexecadotril | MME | 4311 | CG9565 | O |
| dexloxiglumide | CYP4F3 | 4051 | CG3466 | B |
| Dextropropoxyphene | CYP3A4 | 1576 | CG9438 | O |
| Diaben | CYP3A4 | 1576 | CG9438 | O |
| Diacomit | CYP3A4 | 1576 | CG9438 | O |
| diadenosine tetraphosphate | CDC2 | 983 | CG5363 | B |
| Didanosine | BEST1 | 7439 | CG6264 | B |
| dillapiol | CYP3A4 | 1576 | CG9438 | O |
| Diltiazem | PREP,CYP3A4,ABCB1 ,CYP4F3 | 5550,1576,5243,40 51 | CG5355,CG9438,CG3 879,CG3466 | B |
| Dinoprostone | PSMD12,RAC1,TSC1, JUND,FGF9,JUN,PHC 2,JAK3,FGF2,VEGFC ,C2orf3,FGF7,HGF, ATF2,MAP4K1,ABCB1 ,CCND1,JUNB,EGR1, JAG1 | 5718,5879,7248,37 27,2254,3725,1912 ,3718,2247,7424,6 936,2252,3082,138 6,11184,5243,595, 3726,1958,182 | CG1100,CG2248,CG6 147,CG2275,CG4608 ,CG2275,CG18414,C G1594,CG4608,CG71 03,CG1965,CG4608, CG13744,CG30420,C G7097,CG3879,CG90 96,CG2275,CG7847, CG6127 | O |
| Diosgenin | GORASP1 | 64689 | CG7809 | B |
| diosmetin | RAC1 | 5879 | CG2248 | B |
| dioxirane | TBXAS1 | 6916 | CG3466 | B |
| Dioxolan | RAC1 | 5879 | CG2248 | B |
| Dipyrone | CYP4F3,CYP3A4 | 4051,1576 | CG3466,CG9438 | O |
| Disopyramide | MAF,PPA1,PREP | 4094,5464,5550 | CG10034,CG4634,CG 5355 | |
| Ditiocarb | HSPA1B,HSPA2 | 3304,3306 | CG31449,CG5436 | O |
| Dizocilpine Maleate | TSC1,HTR1A,CCND1 | 7248,3350,595 | CG6147,CG7485,CG9 096 | B |
| DNSCl | CYP3A4,SERPINC1 | 1576,462 | CG9438,CG9456 | B |
| Doca | EGR1 | 1958 | CG7847 | O |
| dofequidar | ABCB1 | 5243 | CG3879 | O |
| Dolomin | CSE1L | 1434 | CG13281 | O |
| Domperidone | ABCB1 | 5243 | CG3879 | O |
| Doxazosin | HTR1A | 3350 | CG7485 | O |
| doxifluridine | STUB1 | 10273 | CG5203 | B |
| Doxycycline | NOTCH1,MYB,JUN,CY P3A4,FGF7 | 4851,4602,3725,15 76,2252 | CG3936,CG9045,CG2 275,CG9438,CG4608 | B |
| DPC 681 | CYP3A4 | 1576 | CG9438 | O |
| DPCPX | CPA1 | 1357 | CG14820 | B |
| DPPH | JAG1 | 182 | CG6127 | O |
| Droxia | JUN,ABCB1,CYP3A4, FOXD3,CDC2 | 3725,5243,1576,27 022,983 | CG2275,CG3879,CG9 438,CG3668,CG5363 | B |
| Drysol | MAPT | 4137 | CG31057 | B |
| duloxetine | MAPT | 4137 | CG31057 | B |
| Durapatite | JAK1,PPA1 | 3716,5464 | CG1594,CG4634 | O |
| Dursbanoxon | CYP3A4 | 1576 | CG9438 | O |
| DX 9065a | HGF | 3082 | CG13744 | O |
| Dxms | PLAT,RPSA,SLC5A5, JUNB,JUND,FGF2,MB L2,MME,TSC1,CYP4Z 1,HSPB2,HSPB8,FGF 7,VEGFC,CCND1,CHA T,ABCB1,CYP4A11,C YP3A4,MAGT1,TXNIP ,CCND3,CCNB1,CYP2 7A1,PSMD2,AXIN2,H GF,HSP90AA1,JUN,H SPB6,GORASP1 | 5327,3921,6528,37 26,3727,2247,4153 ,4311,7248,199974 ,3316,26353,2252, 7424,595,1103,524 3,1579,1576,84061 ,10628,896,891,15 93,5708,8313,3082 ,3320,3725,126393 , 64689 | CG13744,CG14792,C G32669,CG2275,CG2 275,CG4608,CG7763 ,CG9565,CG6147,CG 3466,CG14207,CG14 207,CG4608,CG7103 ,CG9096,CG12345,C G3879,CG3466,CG94 38,CG7830,CG18745 ,CG9096,CG3510,CG 6042,CG7762,CG792 6,CG13744,CG1242, CG2275,CG4183,CG7 809 | B |
| dynapen | ABCB1 | 5243 | CG3879 | O |
| Dynatra | RA-SA3,SSTR4,MAPT,SE RPINB1,TBXAS1,FOX D3,METAP2,DTN1,CP A1,HTR1A,HSPA4,MA P2,PPA1,SSTR1,OPR M1,NF2 | 22821,6754,4137,1 992,6916,27022,10 988,84062,1357,33 50,3308,4133,5464 ,6751,4988,4771 | CG6721,CG7285,CG3 1057,CG9456,CG346 6,CG3668,CG4008,C G6856,CG14820,CG7 485,CG6603,CG3105 7,CG4634,CG7285,C G7285,CG14228 | B |
| dynorphin | OPRK1 | 4986 | CG7285 | B |
| dysidenin | OPRK1 | 4986 | CG7285 | B |
| dysprosium | SLC5A5 | 6528 | CG32669 | O |
| dystrobrevin | MAPT | 4137 | CG31057 | B |
| E 10 | MAF,CCND1 | 4094,595 | CG10034,CG9096 | B |
| EACA | PLG,SERPINC1 | 5340,462 | CG13744,CG9456 | O |
| ebastine | SERPINC1 | 462 | CG9456 | B |
| ebrotidine | CYP4F3 | 4051 | CG3466 | B |
| Econ | PDPK1,CYP4F2,FYN, IK,PCSK7 | 5170,8529,2534,35 50, 9159 | CG1210,CG3466,CG7 524,CG18005,CG107 72 | B |
| econazole | PCSK7 | 9159 | CG10772 | O |
| ecteinascidin 743 | CYP3A4,ABCB1,CYP4 F3 | 1576,5243,4051 | CG9438,CG3879,CG3 466 | O |
| ectoine | CYP4F3 | 4051 | CG3466 | B |
| Edex | MME,ATP1B1,PDGFB, CNGA2,PLAT | 4311,481,5155,126 0,5327 | CG9565,CG9258,CG7 103,CG7779,CG1374 4 | O |
| Edrophonium | PLAT | 5327 | CG13744 | O |
| efavirenz | ABCB1,MAPT,KLHL1, CYP4F3,CYP3A4 | 5243,4137,57626,4 051,1576 | CG3879,CG31057,CG 17754,CG3466,CG94 38 | B |
| efrapeptin | CYP3A4 | 1576 | CG9438 | O |
| EGCg | FGF1,HSPA5,IGF1R, CDK2,RPSA,JUND,FG F2,ABCB1,HSPA4,JU N,JUNB,HGF | 2246,3309,3480,10 17,3921,3727,2247 ,5243,3308,3725,3 726,3082 | CG4608,CG5436,CG1 842,CG5363,CG1479 2,CG2275,CG4608,C G3879,CG6603,CG22 75,CG2275,CG13744 | B |
| EGRck | HGF | 3082 | CG13744 | O |
| EHT 1864 | PLAT | 5327 | CG13744 | O |
| eletriptan | RAC1 | 5879 | CG2248 | B |
| Embelin | CYP3A4 | 1576 | CG9438 | O |
| embellistatin | GORASP1 | 64689 | CG7809 | B |
| EMD 53998 | FGF2 | 2247 | CG4608 | B |
| EMD 61753 | TNC | 3371 | CG5550 | O |
| emetine | OPRK1 | 4986 | CG7285 | B |
| E-MIX 80 | CCND1 | 595 | CG9096 | B |
| Emodin | CCND1,JUND,RAC1,K DR,GORASP1,JUN,JU NB | 595,3727,5879,379 1,64689,3725,3726 | CG9096,CG2275,CG2 248,CG8222,CG7809 ,CG2275,CG2275 | B |
| Empecid | CYP3A4,CYP3A7,THO C4 | 1576,1551,10189 | CG9438,CG9438,CG1 101 | O |
| emtricitabine | THOC4 | 10189 | CG1101 | B |
| enadoline | KLHL1 | 57626 | CG17754 | O |
| Enalapril | KLHL1,PLAT | 57626,5327 | CG17754,CG13744 | O |
| Enalaprilat | PLAT | 5327 | CG13744 | O |
| Enediynes | PLAT | 5327 | CG13744 | O |
| Enelone | CCNA2 | 890 | CG3510 | B |
| Enoximone | ATF2 | 1386 | CG30420 | B |
| EPIB | MME | 4311 | CG9565 | O |
| Epicar | CYP4A11 | 1579 | CG3466 | B |
| epicatechin gallate | SERPINB3 | 6317 | CG9456 | B |
| epimedin C | CCND1 | 595 | CG9096 | B |
| Epoprostenol | SERPINC1,RAC1,CYP3A4 ,FIGF,FGF1 | 462,5879,1576,227 7,2246 | CG9456,CG2248,CG9 438,CG7103,CG4608 | O |
| Epostane | FGF1 | 2246 | CG4608 | B |
| Epothilones | FOXD3 | 27022 | CG3668 | B |
| epoxybergamottin | ABCB1 | 5243 | CG3879 | O |
| epsilon-viniferin | CYP3A4 | 1576 | CG9438 | O |
| eptifibatide | CYP4F3 | 4051 | CG3466 | B |
| ergotamine | PLAT | 5327 | CG13744 | O |
| eriocalyxin B | HTR1A | 3350 | CG7485 | O |
| erlotinib | CCND1,ABCB1,JAK2, CYP3A4 | 595,5243,3717,157 6 | CG9096,CG3879,CG1 594,CG9438 | B |
| erucin | CYP3A4 | 1576 | CG9438 | O |
| Eryc | JUN,CYP3A4,ABCB1, HGF,JUND,PPA1,JUN B | 3725,1576,5243,30 82,3727,5464,3726 | CG2275,CG9438,CG3 879,CG13744,CG227 5,CG4634,CG2275 | O |
| Eskazine | EGR1,MAPT | 1958,4137 | CG7847,CG31057 | O |
| Estramustine | MAPT | 4137 | CG31057 | B |
| ET18-Ome | MAPT | 4137 | CG31057 | B |
| Etfc cpd | PCSK7 | 9159 | CG10772 | O |
| Ethacrynic Acid | CYP3A4 | 1576 | CG9438 | O |
| Ethambutol | GORASP1 | 64689 | CG7809 | B |
| Etidronic Acid | ABCB1 | 5243 | CG3879 | O |
| Etodolac | SERPINB3,CCNB1,CDK4, FGF2,CCNA2,CDC2,C DK2 | 6317,891,1019,224 7,890,983,1017 | CG9456,CG3510,CG5 072,CG4608,CG3510 ,CG5363,CG5363 | B |
| Etoposide | TOP2A,JUND,ABCB1, JUNB,SF4,JUN | 7153,3727,5243,37 26,57794,3725 | CG10223,CG2275,CG 3879,CG2275,CG315 50,CG2275 | B |
| etoricoxib | JUN | 3725 | CG2275 | B |
| Etorphine | CYP3A4 | 1576 | CG9438 | O |
| etravirine | OPRM1 | 4988 | CG7285 | B |
| Eufor | CYP4F3,CYP3A4,HTR 1A | 4051,1576,3350 | CG3466,CG9438,CG7 485 | O |
| eumelanin | HTR1A | 3350 | CG7485 | O |
| eupatilin | CCND1,CCNB1,CDK2 | 595,891,1017 | CG9096,CG3510,CG5 363 | B |
| everolimus | CDK2 | 1017 | CG5363 | B |
| Evex | CCNB1,NOTCH1,TSC1 ,NCOR1,NCOR2,QRSL 1,JUND,PLAT,PLG,J UNB,HSPA5,JAG1,JU N,MLH1,TBXAS1,KDR ,MAPT,EGR1,YES1,I GF1R,CYP27B1,EGR3 ,SSTR2,EZR,SERPIN C1,SHH,SNAI2,RPSA ,SFRS10,SSTR1,CYP 3A4,BEST1,GSK3B,N RF1,CYP4F3,DDX17, TMSB4X,ABCB1,MSN, FBN1,TXNL1,FGF13, CDK2,CDC2,SMARCA4 ,CCND1,CPA1,CYP27 A1,CCNG2,GATA4,HM GB1,HSPA1A,FGF9,H GF,HSPB2,HSPB8,SL C39A6,CDK4,FGF4,F GF7,CCNA2,FGF2,FO XD3,FURIN,HSPB1 | 891,4851,7248,961 1,9612,55278,3727 ,5327,5340,3726,3 309,182,3725,4292 ,6916,3791,4137,1 958,7525,3480,159 4,1960,6752,7430, 462,6469,6591,392 1,6434,6751,1576, 7439,2932,4899,40 51,10521,7114,524 3,4478,2200,9352, 2258,1017,983,659 7,595,1357,1593,9 01,2626,3146,3303 ,2254,3082,3316,2 6353,25800,1019,2 249,2252,890,2247 ,27022,5045,3315 | CG3510,CG3936,CG6 147,CG7951,CG7951 ,CG6007,CG2275,CG 13744,CG13744,CG2 275,CG5436,CG6127 ,CG2275,CG11482,C G3466,CG8222,CG31 057,CG7847,CG7524 ,CG1842,CG6042,CG 7847,CG7285,CG107 01,CG9456,CG4637, CG3758,CG14792,CG 10128,CG7285,CG94 38,CG6264,CG2621, CG3114,CG3466,CG1 0279,CG4944,CG387 9,CG10701,CG3936, CG5495,CG4608,CG5 363,CG5363,CG5942 ,CG9096,CG14820,C G6042,CG11525,CG3 992,CG17921,CG543 6,CG4608,CG13744, CG14207,CG14207,C G6817,CG5072,CG46 08,CG4608,CG3510, CG4608,CG3668,CG1 0772,CG14207 | O |
| Evodin | HSPB1 | 3315 | CG14207 | B |
| exenatide | CCND1,TXNIP,JAK1, EGR1 | 595,10628,3716,19 58 | CG9096,CG18745,CG 1594,CG7847 | B |
| Extina | CYP4F3,KSR1,ABCB1 ,CCND3,CYP3A4,CYP 4F2 | 4051,8844,5243,89 6,1576,8529 | CG3466,CG2899,CG3 879,CG9096,CG9438 ,CG3466 | O |
| ezetimibe | CYP4F2 | 8529 | CG3466 | B |
| F 11440 | ABCB1 | 5243 | CG3879 | O |
| Fanchinine | GORASP1,ABCB1,CCND1 ,FGF2 | 64689,5243,595,22 47 | CG7809,CG3879,CG9 096,CG4608 | B |
| F-Ara-A | HTR1A | 3350 | CG7485 | O |
| febuxostat | CYP3A5,CYP4F3, (FGF2) | 1577,4051,(2247) | CG3466,CG3466, (CG 4608) | B |
| felbamate | CYP4F3 | 4051 | CG3466 | B |
| Felodipine | ABCB1,CYP3A4 | 5243,1576 | CG3879,CG9438 | O |
| fenitrothion | CYP3A4 | 1576 | CG9438 | O |
| fenofibric acid | CYP3A4,CYP4A11 | 1576,1579 | CG9438,CG3466 | O |
| Fenoprofen | SLC5A8, (CYP4A11) | 160728, (1579) | CG8451, (CG3466) | B |
| Fenretinide | TUBB3,SF4,JAK1,EI F2S1,JUN,PLAT,CCN D1 | 10381,57794,3716, 1965,3725,5327,59 5 | CG3401,CG31550,CG 1594,CG9946,CG227 5,CG13744,CG9096 | O |
| fexofenadine | CCND1 | 595 | CG9096 | B |
| fingolimod | ABCB1 | 5243 | CG3879 | O |
| fipronil | JAK3 | 3718 | CG1594 | B |
| fisetin | CDK6,CDK4,GORASP1 | 1021,1019,64689 | CG5072,CG5702,CG7 809 | |
| FLCZ | CYP3A4,ABCB1 | 1576,5243 | CG9438,CG3879 | O |
| Flecainide | ABCB1 | 5243 | CG3879 | O |
| Flesinoxan | PREP | 5550 | CG5355 | O |
| flibanserin | HTR1A | 3350 | CG7485 | O |
| Floxacillin | CYP3A4,ABCB1 | 1576,5243 | CG9438,CG3879 | O |
| Fludeoxyglucose F 18 | ABCB1 | 5243 | CG3879 | O |
| Flunarizine | JAG1 | 182 | CG6127 | O |
| fluorexon | ABCB1 | 5243 | CG3879 | O |
| Fluorouracil | PCSK7,MLH1,TXNIP, BEST1,WNK1,TBXAS1 ,CCNA2,CPA1,ABCB1 ,EGR1,FGF7,HSPA4, CSE1L,GORASP1 | 9159,4292,10628,7 439,65125,6916,89 0,1357,5243,1958, 2252,3308,1434,64 689 | CG10772,CG11482,C G18745,CG6264,CG7 177,CG3466,CG3510 ,CG14820,CG3879,C G7847,CG4608,CG66 03,CG13281,CG7809 | O |
| Fluparoxan | GORASP1 | 64689 | CG7809 | B |
| Flupenthixol | HTR1A | 3350 | CG7485 | O |
| fluvoxamine | ABCB1,CYP3A4,HTR1 | 5243,1576,3350 | CG3879,CG9438,CG7 | O |
| | A | | 485 | |
| fondaparinux | HTR1A | 3350 | CG7485 | O |
| Fonofos | SERPINC1 | 462 | CG9456 | B |
| Format | CYP3A4 | 1576 | CG9438 | O |
| Forskolin | TUBB3,PLAT,PAX6,F GF7,CYP27B1,SLC5A 5,PRPF4,ATP1B1,OP RL1,MSN,HMGB1,PDG FB,MAPT,SSTR1,JUN ,ATP2B2,OPRM1,ABC B1,CCND1,CHAT,GAT A6,ADAMTS16,EGR1, FGF2,FLT4,CYP3A4, HTR1A,HGF | 10381,5327,5080,2 252,1594,6528,912 8,481,4987,4478,3 146,5155,4137,675 1,3725,491,4988,5 243,595,1103,2627 ,170690,1958,2247 ,2324,1576,3350,3 082 | CG3401,CG13744,CG 11186,CG4608,CG60 42,CG32669,CG6322 ,CG9258,CG7285,CG 10701,CG17921,CG7 103,CG31057,CG728 5,CG2275,CG2198,C G7285,CG3879,CG90 96,CG12345,CG3978 ,CG14869,CG7847,C G4608,CG8222,CG94 38,CG7485,CG13744 | O |
| fosamprenavir | HGF | 3082 | CG13744 | O |
| Foscarnet | CYP3A4 | 1576 | CG9438 | O |
| Frakefamide | PPA1 | 5464 | CG4634 | O |
| fucoidan | KDR,FGF2,HGF,SERP INC1,PLG | 3791,2247,3082,46 2,5340 | CG8222,CG4608,CG1 3744,CG9456,CG137 44 | B |
| fulvestrant | JUN,CCND2,ATF2,CC ND1 | 3725,894,1386,595 | CG2275,CG9096,CG3 0420,CG9096 | B |
| Fura-2 | ABCB1,PCSK7 | 5243,9159 | CG3879,CG10772 | O |
| furafylline | CYP3A4,FURIN | 1576,5045 | CG9438,CG10772 | O |
| Furylfuramide | FURIN | 5045 | CG10772 | O |
| FYDE | PREP,X1,FOXD3,GOR ASP1,JUN,PDXP,MAP T | 5550,7494,27022,6 4689,3725,57026,4 137 | CG5355,CG9415,CG3 668,CG7809,CG2275 ,CG4755,CG31057 | B |
| Gambogic acid | CDC2,KDR | 983,3791 | CG5363,CG8222 | B |
| gedunin | KDR | 3791 | CG8222 | O |
| gefitinib | CYP3A4,(HSP90AA1) | 1576, (3320) | (CG1242),CG9438 | O |
| Geldanamycin | HSPA4,HSPA2,HSPA1 B,HSPA5,STUB1,HSP B1,JUN,RAC1,CCNB1 ,CCND1,CCNT1,FGF2 ,CDC2,CDK2,HGF,HS P90AA1,CDC37,HSPA 1A | 3308,3306,3304,33 09,10273,3315,372 5,5879,891,595,90 4,2247,983,1017,3 082,3320,11140,33 03 | CG6603,CG5436,CG3 1449,CG5436,CG520 3,CG14207,CG2275, CG2248,CG3510,CG9 096,CG6292,CG4608 ,CG5363,CG5363,CG 13744,CG1242,CG12 | B |
| | | | 019,CG5436 | |
| Gemfibrozil | CYP3A4,PLG,JUNB,S LC22A7,JUN,JUND | 1576,5340,3726,10 864,3725,3727 | CG9438,CG13744,CG 2275,CG8654,CG227 5,CG2275 | B |
| genipin | JUND | 3727 | CG2275 | B |
| Gentamicins | JUN | 3725 | CG2275 | B |
| gepirone | CYP3A4,HTR1A | 1576,3350 | CG9438,CG7485 | O |
| Gestodene | HTR1A | 3350 | CG7485 | O |
| GF 120918 | CYP3A4,ABCB1,KLHL 1 | 1576,5243,57626 | CG9438,CG3879,CG1 7754 | O |
| GGTI 298 | KLHL1 | 57626 | CG17754 | O |
| GI 129471 | CDK2 | 1017 | CG5363 | B |
| Ginkgo-biloba-extract | JUND,CYP3A4,CYP4F 3,JUNB,JUN,PLAT,C YP27B1 | 3727,1576,4051,37 26,3725,5327,1594 | CG2275,CG9438,CG3 466,CG2275,CG2275 ,CG13744,CG6042 | O |
| glabridin | CYP27B1 | 1594 | CG6042 | B |
| GLCa | SERPINC1,EXT1 | 462,2131 | CG9456,CG10117 | B |
| Glumin | THOC4,MSN,HSP90AA 1,DNAJB1,HSPA8 | 10189,4478,3320,3 337,3312 | CG1101,CG10701,CG 1242,CG10578,CG31 449 | B |
| Glycyron | JUND,KLHL1,HMGB1, JUNB,JUN,GORASP1 | 3727,57626,3146,3 726,3725,64689 | CG2275,CG17754,CG 17921,CG2275,CG22 75,CG7809 | B |
| glyox | GORASP1 | 64689 | CG7809 | B |
| Gnidimacrin | CYP27B1 | 1594 | CG6042 | B |
| gossypetin | CDK2 | 1017 | CG5363 | B |
| gossypol | AGL,CCND1,GORASP1 | 178,595,64689 | CG9485,CG9096,CG7 809 | B |
| GR 113808 | GORASP1 | 64689 | CG7809 | B |
| grifolin | CCND1,CDK4 | 595,1019 | CG9096,CG5072 | B |
| Grofo | CYP4F3,CYP3A4,ABC B1 | 4051,1576,5243 | CG3466,CG9438,CG3 879 | O |
| Guggulsterone | ABCB1,CCND1 | 5243,595 | CG3879,CG9096 | O |
| gusperimus | HSPA8,HSP90AA1 | 3312,3320 | CG31449,CG1242 | B |
| Harzol | CCNB1,TUBA1B,MAP2 ,CDC2 | 891,10376,4133,98 3 | CG3510,CG1913,CG3 1057,CG5363 | B |
| Hbim | OPRL1,KDR | 4987,3791 | CG7285,CG8222 | B |
| HESPERETIN | KDR | 3791 | CG8222 | O |
| Hexadimethrine | CDK4 | 1019 | CG5072 | O |
| HMBA | SERPINC1 | 462 | CG9456 | B |
| hypsiziprenol A9 | FGF4 | 2249 | CG4608 | B |
| hypusine | CCND1 | 595 | CG9096 | B |
| hyrtioerectine A | EIF5 | 1983 | CG9177 | B |
| ibandronic acid | EYA1 | 2138 | CG9554 | O |
| IBMX | JAK3,MME,HGF,LPA | 3718,4311,3082,40 18 | CG1594,CG9565,CG1 3744,CG13744 | O |
| I-BOP | CCND1,EYA1 | 595,2138 | CG9096,CG9554 | B |
| Ibotenic Acid | LPA | 4018 | CG13744 | O |
| Icosapent | PLAT | 5327 | CG13744 | O |
| ICRF 193 | POLS | 11044 | CG11265 | B |
| idebenone | TOP2B | 7155 | CG10223 | B |
| IDN 5390 | HSPA4 | 3308 | CG6603 | O |
| Ifosfamide | TUBB3 | 10381 | CG3401 | B |
| Ifosfamide | CYP4F3 | 4051 | CG3466 | B |
| IGF-1 | CYP3A4 | 1576 | CG9438 | O |
| Iloprost | RBM6,RAC1 | 10180,5879 | CG4887,CG2248 | B |
| imatinib | CYP3A4,EGR1,JUN,C CND1,PDGFB,CCND2, CCNA2,KIF5B,PCSK7 ,JAK2,JUNB,KDR,HS PA4,ABCB1,FGF2,CD K2,CRKL,HGF | 1576,1958,3725,59 5,5155,894,890,37 99,9159,3717,3726 ,3791,3308,5243,2 247,1017,1399,308 2 | CG9438,CG7847,CG2 275,CG9096,CG7103 ,CG9096,CG3510,CG 7765,CG10772,CG15 94,CG2275,CG8222, CG6603,CG3879,CG4 608,CG5363,CG1587 ,CG13744 | B |
| imidafenacin | ABCB1 | 5243 | CG3879 | O |
| imperatorin | CYP3A4 | 1576 | CG9438 | O |
| Impulsin | CCND1 | 595 | CG9096 | B |
| Imrecoxib | FAAH | 2166 | CG6007 | B |
| Imutex | HIST2H3C,MAPT,HIS T1H3C,BEST1,CYP3A 7,HIST1H3E,CYP4F3 | 126961,4137,8352, 7439,1551,8353,40 51 | CG5825,CG31057,CG 5825,CG6264,CG943 8,CG5825,CG3466 | B |
| indazole | CYP4F3 | 4051 | CG3466 | B |
| Indinavir | CYP4F3,CYP3A4,ABC B1 | 4051,1576,5243 | CG3466,CG9438,CG3 879 | O |
| indiplon | ABCB1 | 5243 | CG3879 | O |
| indirubin-3'-monoxime | CYP3A4 | 1576 | CG9438 | O |
| infliximab | KDR,PSMD12 | 3791,5718 | CG8222,CG1100 | O |
| inogatran | PSMD12 | 5718 | CG1100 | O |
| Ipral | CPB2 | 1361 | CG14820 | B |
| Iprivask | ABCB1 | 5243 | CG3879 | O |
| ipsapirone | PLG | 5340 | CG13744 | O |
| irbesartan | JUNB,JUND,JUN,BES T1 | 3726,3727,3725,74 39 | CG2275,CG2275,CG2 275,CG6264 | B |
| Iressa | RAC1,HGF,CCND1,CY P3A4,IGF1R | 5879,3082,595,157 6,3480 | CG2248,CG13744,CG 9096,CG9438,CG184 2 | O |
| irinotecan | PCSK7,CDC2,CYP3A4 ,KLHL1,ABCB1 | 9159,983,1576,576 26,5243 | CG10772,CG5363,CG 9438,CG17754,CG38 79 | O |
| irisolidone | JUNB,JUND,JUN | 3726,3727,3725 | CG2275,CG2275,CG2 275 | B |
| irofulven | JUN | 3725 | CG2275 | B |
| Irox | EIF5B | 9669 | CG10840 | O |
| Ismo | PHC2 | 1912 | CG18414 | O |
| Isobac | CSE1L | 1434 | CG13281 | O |
| isochaihulactone | CYP3A4 | 1576 | CG9438 | O |
| Isodonol | EGR1 | 1958 | CG7847 | O |
| isoflavone | IGF1R | 3480 | CG1842 | O |
| Isorhamnetin | BEST1 | 7439 | CG6264 | B |
| isosteviol | ABCB1 | 5243 | CG3879 | O |
| Isradipine | POLS | 11044 | CG11265 | B |
| Istidina | CYP3A4 | 1576 | CG9438 | O |
| ITF 2357 | ABCB1 | 5243 | CG3879 | O |
| Itraconazole | ABCB1,CYP3A4,CYP4 F3 | 5243,1576,4051 | CG3879,CG9438,CG3 466 | O |
| Ivermectin | KLHL1,ABCB1 | 57626,5243 | CG17754,CG3879 | O |
| ixabepilone | ABCB1 | 5243 | CG3879 | O |
| J 113397 | CYP3A4 | 1576 | CG9438 | O |
| J 113397 | OPRL1 | 4987 | CG7285 | B |
| jasplakinolide | JUN,JUNB,JUND | 3725,3726,3727 | CG2275,CG2275,CG2 275 | B |
| juglone | JUND | 3727 | CG2275 | B |
| juzentaihoto | MAPT | 4137 | CG31057 | B |
| K 252 | CDC2,PPA1 | 983,5464 | CG5363,CG4634 | B |
| kaempferol | CYP3A4,JAK3,FAAH, JUND,JUN,IK,JUNB | 1576,3718,2166,37 27,3725,3550,3726 | CG9438,CG1594,CG6 007,CG2275,CG2275 ,CG18005,CG2275 | B |
| KAFA | JUNB | 3726 | CG2275 | B |
| kahweol | CYP4F3 | 4051 | CG3466 | B |
| Kainic Acid | MAP2,METAP2,JUN | 4133,10988,3725 | CG31057,CG4008,CG 2275 | B |
| kami-untan-to | JUN | 3725 | CG2275 | B |
| Kaolin | SERPINC1,PLG | 462,5340 | CG9456,CG13744 | B |
| KB 2796 | PLG | 5340 | CG13744 | O |
| K-DR | PPA1 | 5464 | CG4634 | O |
| Keloid | ABCB1 | 5243 | CG3879 | O |
| Kemi | EGR1,PREP,NF2 | 1958,5550,4771 | CG7847,CG5355,CG1 4228 | O |
| ketazocine | NF2 | 4771 | CG14228 | B |
| Ketopgflalpha | OPRK1 | 4986 | CG7285 | B |
| KKHA-761 | SERPINC1 | 462 | CG9456 | B |
| KN 62 | HTR1A | 3350 | CG7485 | O |
| KN 93 | NOTCH1,CDK4 | 4851,1019 | CG3936,CG5072 | B |
| KNK 437 | HSPA1A,HSPB2,HSPB 1,HSPA4 | 3303,3316,3315,33 08 | CG5436,CG14207,CG 14207,CG6603 | B |
| KP372-1 | HSPA4 | 3308 | CG6603 | O |
| K-PAM | CSE1L,HRB,SERPINC 1 | 1434,3267,462 | CG13281,CG3365,CG 9456 | B |
| KPMK | PDPK1 | 5170 | CG1210 | B |
| KR 31831 | GORASP1 | 64689 | CG7809 | B |
| KRM 1648 | KDR | 3791 | CG8222 | O |
| K-SR | PPP2CA,GSK3B,GTF3 C1 | 5515,2932,2975 | CG7109,CG2621,CG7 099 | B |
| kurarinone | CYP3A4 | 1576 | CG9438 | O |
| KYNA | KDR | 3791 | CG8222 | O |
| L 685458 | NOTCH1,HEY1 | 4851,23462 | CG3936,CG11194 | B |
| L797591 | CYP3A4 | 1576 | CG9438 | O |
| LAGA | SSTR1 | 6751 | CG7285 | B |
| Lamivudine | CSE1L,KLHL1 | 1434,57626 | CG13281,CG17754 | O |
| lamotrigine | KLHL1 | 57626 | CG17754 | O |
| lanreotide | HTR1A | 3350 | CG7485 | O |
| lapachenole | PPA1 | 5464 | CG4634 | O |
| lapatinib | CYP3A4 | 1576 | CG9438 | O |
| LAQ824 | TUBA1B,HSP90AA1 | 10376,3320 | CG1913,CG1242 | B |
| laquinimod | HSP90AA1 | 3320 | CG1242 | O |
| latrunculin A | TMSB4X,PCBD1 | 7114,5092 | CG4944,CG1963 | O |
| LBH589 | TUBA1B,HSP90AA1 | 10376,3320 | CG1913,CG1242 | B |
| leflunomide | PDPK1,FYN | 5170,2534 | CG1210,CG7524 | B |
| lenalidomide | JUND,JUNB,JUN | 3727,3726,3725 | CG2275,CG2275,CG2 275 | B |
| Lendorm | JUN | 3725 | CG2275 | B |
| Lentinan | CYP3A4 | 1576 | CG9438 | O |
| Leukotriene B4 | JUNB,PLCB2,JUN,JU ND | 3726,5330,3725,37 27 | CG2275,CG3620,CG2 275,CG2275 | B |
| Leukotriene C4 | JUN,ABCB5,KLHL1,J UND,JUNB | 3725,340273,57626 ,3727,3726 | CG2275,CG3879,CG1 7754,CG2275,CG227 5 | B |
| Leukotriene D4 | JUN,JUNB,JUND | 3725,3726,3727 | CG2275,CG2275,CG2 275 | B |
| leukotrienes | HTR1A,SSTR4,SSTR1 | 3350,6754,6751 | CG7485,CG7285,CG7 285 | B |
| Levonorgestrel | PLAT,PLG,JUN,CSE1 L | 5327,5340,3725,14 34 | CG13744,CG13744,C G2275,CG13281 | B |
| liarozole | CSE1L | 1434 | CG13281 | O |
| lintopride | HTR4, (CYP3A4) | 3360, (1576) | CG6919, (CG9438) | O |
| liquiritin | HTR4 | 3360 | CG6919 | O |
| LMWH | HSPB2,PLAT,MAPT,H SP90AA1,HSPB1,PPI B,SERPINC1,JUN,JU NB,JUND,HGF,CDK2, PLG,GSK3A,ADAMTS5 ,FLT4,FGF2,DDX5,R PSA,CCND1,GORASP1 ,FGF13,FGF1,FGF7, FGF20,CPB2,CYP27A 1 | 3316,5327,4137,33 20,3315,5479,462, 3725,3726,3727,30 82,1017,5340,2931 ,11096,2324,2247, 1655,3921,595,646 89,2258,2246,2252 ,26281,1361,1593 | CG14207,CG13744,C G31057,CG1242,CG1 4207,CG2852,CG945 6,CG2275,CG2275,C G2275,CG13744,CG5 363,CG13744,CG262 1,CG14869,CG8222, CG4608,CG10279,CG 14792,CG9096,CG78 09,CG4608,CG4608, CG4608,CG4608,CG1 4820,CG6042 | B |
| LNAC | HSPAS,CCND2,JUND, HGF,ABCB1,JUN,PDP K1,HSPA4,HSPA1A,J UNB,GORASP1 | 3309,894,3727,308 2,5243,3725,5170, 3308,3303,3726,64 689 | CG5436,CG9096,CG2 275,CG13744,CG387 9,CG2275,CG1210,C G6603,CG5436,CG22 75,CG7809 | B |
| lonafarnib | GORASP1 | 64689 | CG7809 | B |
| lonidamine | ABCB1 | 5243 | CG3879 | O |
| Loperamide | CYP3A4,ABCB1,OPRM 1 | 1576,5243,4988 | CG9438,CG3879,CG7 285 | O |
| lopinavir | ABCB1,CYP3A4 | 5243,1576 | CG3879,CG9438 | O |
| Lopril | PLAT,PLG | 5327,5340 | CG13744,CG13744 | O |
| Loratadine | ABCB1,CYP3A4 | 5243,1576 | CG3879,CG9438 | O |
| Lorazepam | CYP3A4 | 1576 | CG9438 | O |
| Lorex | CPA1 | 1357 | CG14820 | B |
| Losartan | SLC22A12,CYP27B1, MBL2,CYP3A4 | 116085,1594,4153, 1576 | CG8654,CG6042,CG7 763,CG9438 | B |
| Lovan | SMARCA4,PLG,SMARCA2 | 6597,5340,6595 | CG5942,CG13744,CG 5942 | O |
| loxiglumide | SMARCA2 | 6595 | CG5942 | B |
| L-T3 | IK,CYP3A4 | 3550,1576 | CG18005,CG9438 | B |
| lupeol | PPA1 | 5464 | CG4634 | O |
| luteolin | JUND,CYP3A4,IGF1R ,CCND1,HGF,JUN,JU NB | 3727,1576,3480,59 5,3082,3725,3726 | CG2275,CG9438,CG1 842,CG9096,CG1374 4,CG2275,CG2275 | B |
| lutetium Lutex | JUNB SLC19A1,JUN,MAP2, METAP2,HSPB1,FAAH,CD C2,PDGFB,PREP,CDK 2,SERPINC1,RPSA,A BCB1,CCND1,EIF4E1 ,MAPT,CYP3A4,GORA SP1,HGF,HSPB2,EGR 2,FGF7 | 3726 6573,3725,4133,10 988,3315,2166,983 ,5155,5550,1017,4 62,3921,5243,595, 1978,4137,1576,64 689,3082,3316,195 9,2252 | CG2275 CG14694,CG2275,CG 31057,CG4008,CG14 207,CG6007,CG5363 ,CG7103,CG5355,CG 5363,CG9456,CG147 92,CG3879,CG9096, CG8846,CG31057,CG 9438,CG7809,CG137 44,CG14207,CG7847 ,CG4608 | B |
| LY 117018 | FGF7 | 2252 | CG4608 | B |
| LY 293111 | CCNA2,CDK2 | 890,1017 | CG3510,CG5363 | B |
| LY 293284 | CDK2 | 1017 | CG5363 | B |
| LY 303511 | HTR1A | 3350 | CG7485 | O |
| LY231514 | EGR1 | 1958 | CG7847 | O |
| mahanine | TBXAS1 | 6916 | CG3466 | B |
| Maleimides | CCND1 | 595 | CG9096 | B |
| Malix | GSK3A | 2931 | CG2621 | B |
| manzamine A | CYP3A4 | 1576 | CG9438 | O |
| maraviroc | GSK3A | 2931 | CG2621 | B |
| MBC1 | CYP3A4 | 1576 | CG9438 | O |
| MCYST-LR | PPA1,PPP2CA | 5464,5515 | CG4634,CG7109 | O |
| Mebumal | PPP2CA | 5515 | CG7109 | O |
| Medemycin | ATP2B2 | 491 | CG2191 | O |
| Medroxyprogesterone 17-Acetate | CYP3A4,SERPINC1,M SN,JUN | 1576,462,4478,372 5 | CG9438,CG9456,CG1 0701,CG2275 | B |
| Melatol | CCND1,JUN,MAPT,CY P4F3,FOXD3 | 595,3725,4137,405 1,27022 | CG9096,CG2275,CG3 1057,CG3466,CG366 8 | B |
| meletin | JUNB,JUN,HSPB2,CC NB1,GSK3B,TSC1,PL AT,ABCB1,CYP3A4,H SPB1,CDC2,JUND,HS PA4,HSPA1A,CDK2,H SP90AA1 | 3726,3725,3316,89 1,2932,7248,5327, 5243,1576,3315,98 3,3727,3308,3303, 1017,3320 | CG2275,CG2275,CG1 4207,CG3510,CG262 1,CG6147,CG13744, CG3879,CG9438,CG1 4207,CG5363,CG227 5,CG6603,CG5436,C G5363,CG1242 | B |
| melitten | PLAT,RAC1 | 5327,5879 | CG13744,CG2248 | O |
| meloxicam | RAC1 | 5879 | CG2248 | B |
| Memantine | MAPT,CYP3A4 | 4137,1576 | CG31057,CG9438 | B |
| Menatetrenone | CYP3A4 | 1576 | CG9438 | O |
| MENT | SERPINB3,SERPINB4 | 6317,6318 | CG9456,CG9456 | B |
| Mephenytoin | SERPINB4 | 6318 | CG9456 | B |
| Meprobamate | CYP3A4 | 1576 | CG9438 | O |
| MeSAdo | JUN,MTAP,JUNB,JUN D | 3725,4507,3726,37 27 | CG2275,CG4802,CG2 275,CG2275 | B |
| mesalamine | JUND | 3727 | CG2275 | B |
| Mesaton | CPA1,JUN,CCNA2 | 1357,3725,890 | CG14820,CG2275,CG 3510 | B |
| mesoglycan | CCNA2 | 890 | CG3510 | B |
| Mesol | FGF1 | 2246 | CG4608 | B |
| metazachlor | FOXD3 | 27022 | CG3668 | B |
| Meth | JUNB,MAPT,JUND,OP RM1,DTN1,JUN | 3726,4137,3727,49 88,84062,3725 | CG2275,CG31057,CG 2275,CG7285,CG685 6,CG2275 | O |
| methanopterin | JUN | 3725 | CG2275 | B |
| Methorphan | CYP3A4,CYP4F3 | 1576,4051 | CG9438,CG3466 | O |
| Methoxsalen | CYP4F3 | 4051 | CG3466 | B |
| methoxymorphinan | CYP3A4 | 1576 | CG9438 | O |
| Methylprednisolone | MBL2,SERPINC1 | 4153,462 | CG7763,CG9456 | B |
| Methylthioinosine | SERPINC1 | 462 | CG9456 | B |
| Metkephamid | PPA1 | 5464 | CG4634 | O |
| Metopiron | CYP3A4,CYP4F3 | 1576,4051 | CG9438,CG3466 | O |
| Metribolone | CDK4,GSK3B,FGF7 | 1019,2932,2252 | CG5072,CG2621,CG4 608 | B |
| Miazine | FGF7 | 2252 | CG4608 | B |
| miconazole | CYP3A4,CYP4F3 | 1576,4051 | CG9438,CG3466 | O |
| Mictonorm | CYP4F3 | 4051 | CG3466 | B |
| Midazolam | CYP3A4,CYP4F3,ABC B1,MMEL1 | 1576,4051,5243,79 258 | CG9438,CG3466,CG3 879,CG9565 | O |
| Mifepristone | CYP3A4,NCOR1,CCNA 2,CDK2,ABCB1 | 1576,9611,890,101 7,5243 | CG9438,CG7951,CG3 510,CG5363,CG3879 | B |
| miglustat | ABCB1 | 5243 | CG3879 | O |
| milbemycin | KDR | 3791 | CG8222 | O |
| Mimosine | ABCB1 | 5243 | CG3879 | O |
| mirtazapine | EIF5 | 1983 | CG9177 | B |
| Mit-C | JUNB,PPP1R15A,FCN 2,MAF,JUN,SHH,JUN D | 3726,23645,2220,4 094,3725,6469,372 7 | CG2275,CG3825,CG5 55,CG10034,CG2275 ,CG4637,CG2275 | O |
| mithramycin A | JUND | 3727 | CG2275 | B |
| Mitoxantrone | ABCB1,KLHL1,JUN | 5243,57626,3725 | CG3879,CG17754,CG 2275 | |
| MK-0524 | JUN | 3725 | CG2275 | B |
| MLN 944 | TFDP1 | 7027 | CG4654 | B |
| Molsidomine | JUN | 3725 | CG2275 | B |
| Monensin | HRB,HSPA4,CDK2,CC NA2,CDC2,PLAT,CCN D1,CDK6,CDK4 | 3267,3308,1017,89 0,983,5327,595,10 21,1019 | CG3365,CG6603,CG5 363,CG3510,CG5363 ,CG13744,CG9096,C G5072,CG5072 | O |
| monocillin I | CYP3A4 | 1576 | CG9438 | O |
| monodansylcadaverine | HSP90AA1 | 3320 | CG1242 | O |
| mono-N-demethyladinazolam | CDK4 | 1019 | CG5072 | O |
| Monorden | HSP90AB1,HSPA4,FG F2,DNAJB1,HSP90AA | 3326,3308,2247,33 37,3320 | CG1242,CG6603,CG4 608,CG10578,CG124 | B |
| | 1 | | 2 | |
| MORIN | JUND,JUNB,ABCB1,J UN | 3727,3726,5243,37 25 | CG2275,CG2275,CG3 879,CG2275 | O |
| morphine-3-glucuronide | JUN | 3725 | CG2275 | B |
| mosapride | HTR4, (CYP3A4) | 3360, (1576) | CG6919, (CG9438) | O |
| motapizone | HTR4 | 3360 | CG6919 | O |
| Motuporin | MME | 4311 | CG9565 | O |
| moxifloxacin | PPP2CA | 5515 | CG7109 | O |
| MRK 003 | NOTCH4,NOTCH3 | 4855,4854 | CG3936,CG3936 | B |
| MTPA | NOTCH3 | 4854 | CG3936 | B |
| Muran | PLG | 5340 | CG13744 | O |
| Muscarine | FOXD3 | 27022 | CG3668 | B |
| N-(4-aminophenethnethyl)spiroperid ol | CCND1 | 595 | CG9096 | B |
| N-(4-aminophenyl)maleimide | ABCB1 | 5243 | CG3879 | O |
| N-(4-cyano-benzo(b)thiophen e-2-carbonyl)guanidine | CYP3A7,CYP3A4 | 1551,1576 | CG9438,CG9438 | O |
| N-(8-amino-1-carboxyoctyl)-alanylproline | CYP3A4 | 1576 | CG9438 | O |
| N(alpha)-(4-amino-4-deoxypteroyl)-N(delta)-hemiphthaloyl-L-ornithine | HTR1A | 3350 | CG7485 | O |
| N-(m-heptyl)-5-chloro-1-naphthalenesulfonamide | FGF1 | 2246 | CG4608 | B |
| N-3-isoquinolinyl-2-((4-pyridinylmethyl)amino)be nzamide | HSP90AA1 | 3320 | CG1242 | O |
| NABU | SLC19A1 | 6573 | CG14694 | O |
| Naftalen | GORASP1 | 64689 | CG7809 | B |
| Naloxone | CYP3A4 | 1576 | CG9438 | O |
| Naltrexone | OPRM1 | 4988 | CG7285 | B |
| naltrindole | OPRM1 | 4988 | CG7285 | B |
| NAN-190 hydrobromide | OPRK1 | 4986 | CG7285 | B |
| nanchangmycin | HTR1A | 3350 | CG7485 | O |
| Naphazoline | NDUFAB1 | 4706 | CG9160 | O |
| NARIGENIN | CYP3A4,ABCB1 | 1576,5243 | CG9438,CG3879 | O |
| nateglinide | ABCB1 | 5243 | CG3879 | O |
| N-benzyloxycarbonylprolylprolinal | KDR | 3791 | CG8222 | O |
| N-dehydroxyzileuton | PREP | 5550 | CG5355 | O |
| N-desmethylclob azam | CYP4F3 | 4051 | CG3466 | B |
| nedaplatin | CYP3A4 | 1576 | CG9438 | O |
| Nefazodone | CYP4F3,CYP3A4,HTR 1A | 4051,1576,3350 | CG3466,CG9438,CG7 485 | O |
| Nelfinavir | CYP4F3,CSE1L,CYP3 A4,ABCB1 | 4051,1434,1576,52 43 | CG3466,CG13281,CG 9438,CG3879 | O |
| nemonapride | ABCB1 | 5243 | CG3879 | O |
| neodymium | HTR1A | 3350 | CG7485 | O |
| Neomycin | MAPT | 4137 | CG31057 | B |
| N-ethylmaleimide | GTF3C1,HSPA4,EYA1 | 2975,3308,2138 | CG7099,CG6603,CG9 554 | O |
| netoglitazone | SERPINB1 | 1992 | CG9456 | B |
| neuromedin C | TSC1 | 7248 | CG6147 | O |
| Neut | CCND1,ODC1 | 595,4953 | CG9096,CG8721 | B |
| Nevirapine | CSE1L,ABCB1,KLHL1 ,CYP3A4 | 1434,5243,57626,1 576 | CG13281,CG3879,CG 17754,CG9438 | O |
| Niacinamide | TUBA1B,FGF6,MAPT,CY P3A4 | 10376,2251,4137,1 576 | CG1913,CG4608,CG3 1057,CG9438 | B |
| nicaraven | CYP3A4 | 1576 | CG9438 | O |
| Nicardipine | CYP3A4,ABCB1 | 1576,5243 | CG9438,CG3879 | O |
| Niceritrol | ABCB1 | 5243 | CG3879 | O |
| Nigericin | LPA | 4018 | CG13744 | O |
| niguldipine | EIF4G1 | 1981 | CG10811 | B |
| Nimodipine | ABCB1 | 5243 | CG3879 | O |
| NK 104 | RAC1,FGF2,PLAT | 5879,2247,5327 | CG2248,CG4608,CG1 3744 | B |
| NMDA | FOXD3,MAP2,DMRT1, HTR1A,METAP2 | 27022,4133,1761,3 350,10988 | CG3668,CG31057,CG 11094,CG7485,CG40 08 | O |
| N-methylsulfonyl-6-(2-propargyloxyphenyl)hexanamide | PLAT,CYP4F3 | 5327,4051 | CG13744,CG3466 | O |
| NN 703 | METAP2 | 10988 | CG4008 | B |
| Nobiletin | ABCB1,JUN,JUND,CY P3A4,JUNB | 5243,3725,3727,15 76,3726 | CG3879,CG2275,CG2 275,CG9438,CG2275 | O |
| NOC 18 | JUNB | 3726 | CG2275 | B |
| Nociceptin | OPRL1,SSTR4,HTR1A ,SSTR1,OPRM1 | 4987,6754,3350,67 51,4988 | CG7285,CG7285,CG7 485,CG7285,CG7285 | B |
| noralfentanil | OPRM1 | 4988 | CG7285 | B |
| Norbinaltorphimine | CYP3A4 | 1576 | CG9438 | O |
| norcantharidin | OPRK1 | 4986 | CG7285 | B |
| Nordihydroguaiaretic Acid | JUN,IGF1R,JUND,JUNB | 3725,3480,3727,3726 | CG2275,CG1842,CG2 275,CG2275 | B |
| Norethindrone | PLAT,CYP3A4 | 5327,1576 | CG13744,CG9438 | O |
| noreximide | CYP3A4 | 1576 | CG9438 | O |
| norfluoxetine | CYP4F3,CYP3A4 | 4051,1576 | CG3466,CG9438 | B |
| norlaudanosoline | JUNB,JUND,JUN | 3726,3727,3725 | CG2275,CG2275,CG2 275 | B |
| Nortilidine | ABCB1,CYP3A4 | 5243,1576 | CG3879,CG9438 | O |
| norverapamil | CYP3A4 | 1576 | CG9438 | O |
| novobiocin | HSP90AA1,PCSKS,CD C37 | 3320,5125,11140 | CG1242,CG10772,CG 12019 | O |
| NPI 031L | TSC1,RAC1,IGF1R,M SN,MAPT,KCNK6,PDC D2,PCSK7,JUND,FAA H,NCOR1,HSP90AA1, SERPINB3,CCND1,GORAS P1,JUNB,FGF2,FGF7 ,HGF,ABCB1,CCNB1, HSPAS,CDC2,JAK3,J UN,CYP24A1,BEST1 | 7248,5879,3480,44 78,4137,9424,5134 ,9159,3727,2166,9 611,3320,6317,595 ,64689,3726,2247, 2252,3082,5243,89 1,3309,983,3718,3 725,1591,7439 | CG6147,CG2248,CG1 842,CG10701,CG310 57,CG1688,CG326,C G10772,CG2275,CG6 007,CG7951,CG1242 ,CG9456,CG9096,CG 7809,CG2275,CG460 8,CG4608,CG13744, CG3879,CG3510,CG5 436,CG5363,CG1594 ,CG2275,CG6042,CG 6264 | B |
| NRDC | BEST1 | 7439 | CG6264 | B |
| NSC 23766 | RAC1,NOTCH1 | 5879,4851 | CG2248,CG3936 | B |
| NSC 295558 | NOTCH1 | 4851 | CG3936 | B |
| NSC 366140 | CYP3A4,CYP4F3 | 1576,4051 | CG9438,CG3466 | O |
| NSC 663284 | CYP4F3 | 4051 | CG3466 | B |
| N-tert-butyl-3-[4-(2-methoxyphephenyl)piperazin -1-yl]-2-phenylpropanamide | CYP4F3 | 4051 | CG3466 | B |
| NU2058 | CDC2 | 983 | CG5363 | B |
| NU6102 | CDK2 | 1017 | CG5363 | B |
| nutlin 3 | CDK4 | 1019 | CG5072 | O |
| NVP-AEW541 | ABCB1 | 5243 | CG3879 | O |
| obovatol | JUNB,JUND,JUN | 3726,3727,3725 | CG2275,CG2275,CG2 275 | B |
| OC 144-093 | JUN | 3725 | CG2275 | B |
| ochratoxin A | KLHL1,FOXD3 | 57626,27022 | CG17754,CG3668 | O |
| Octreotide | SSTR5,FGF2,SSTR1, IGF1R,FGF3,PPA1 | 6755,2247,6751,34 80,2248,5464 | CG7285,CG4608,CG7 285,CG1842,CG4608 ,CG4634 | B |
| Octreotide | PPA1 | 5464 | CG4634 | O |
| O-demethyltramadol | IGF1R | 3480 | CG1842 | O |
| O-desethylreboxetine | OPRM1 | 4988 | CG7285 | B |
| oenothein B | SSTR2 | 6752 | CG7285 | B |
| Ogen | FGF2,PLG | 2247,5340 | CG4608,CG13744 | B |
| OH-pro | PLG | 5340 | CG13744 | O |
| Oktan | MBL2 | 4153 | CG7763 | B |
| Olamine | OPRM1 | 4988 | CG7285 | B |
| olanzapine | MEA1 | 4201 | CG14341 | O |
| oleandrin | JUN,JUND,JUNB | 3725,3727,3726 | CG2275,CG2275,CG2 275 | B |
| oleylamide | JUNB | 3726 | CG2275 | B |
| olmelin | JUNB,JUN,JUND | 3726,3725,3727 | CG2275,CG2275,CG2 275 | B |
| olomoucine | MAPT,CDC2,CDK2,CD K4 | 4137,983,1017,101 9 | CG31057,CG5363,CG 5363,CG5072 | B |
| Olymp | CDK4 | 1019 | CG5072 | O |
| omalizumab | PDXP | 57026 | CG4755 | B |
| omapatrilat | CYP3A4 | 1576 | CG9438 | O |
| omega-N-Methylarginine | MME | 4311 | CG9565 | O |
| Omeprazole | ABCB1,CYP3A4,CYP4 F3 | 5243,1576,4051 | CG3879,CG9438,CG3 466 | O |
| omeprazole sulfone | CYP4F3 | 4051 | CG3466 | B |
| Ondansetron | CPA1,CYP3A4,ABCB1 | 1357,1576,5243 | CG14820,CG9438,CG 3879 | |
| ONO 1301 | ABCB1 | 5243 | CG3879 | O |
| Optef | P11,PCBD1,VEGFC,P SMD6,HSPB8,TNC,CC ND1,ABCB1,CPA1,PI K3C2A,HGF,SERPINC 1,CYP3A4,HTR1A,NU P214 | 8909,5092,7424,98 61,26353,3371,595 ,5243,1357,5286,3 082,462,1576,3350 ,8021 | CG2145,CG1963,CG7 103,CG5378,CG1420 7,CG5550,CG9096,C G3879,CG14820,CG1 1621,CG13744,CG94 56,CG9438,CG7485, CG3820 | O |
| Org 31540 | NUP214 | 8021 | CG3820 | B |
| Orphenadrine | HGF | 3082 | CG13744 | O |
| OSI 930 | CYP3A4 | 1576 | CG9438 | O |
| OSU 03012 | CCNA2,HSPA4 | 890,3308 | CG3510,CG6603 | B |
| Ouabain | MAPT,ABCB1,JUN,AT P4B,FGF13,FGF2 | 4137,5243,3725,49 6,2258,2247 | CG31057,CG3879,CG 2275,CG9258,CG460 8,CG4608 | B |
| ovalicin | FGF2 | 2247 | CG4608 | B |
| Ovex | CYP3A4,ABCB1 | 1576,5243 | CG9438,CG3879 | O |
| oxaliplatin | NOTCH1,TXNL1,CDC2 | 4851,9352,983 | CG3936,CG5495,CG5 363 | |
| oxatomide | CDC2 | 983 | CG5363 | B |
| oxcarbazepine | CYP3A4 | 1576 | CG9438 | O |
| Oxidopamine | CCND1,JUN,GSK3B | 595,3725,2932 | CG9096,CG2275,CG2 621 | B |
| oxymatrine | GSK3B | 2932 | CG2621 | B |
| Oxytrol | CYP3A4,CYP4F3 | 1576,4051 | CG9438,CG3466 | O |
| Paclitaxel | MAPT,JUND,CCND1,J UN,TMSB4X,TUBA1B, HSPB1,PSMD2,EIF4E 2,TIMM8A,EIF4E1,C CNB1,CDC2,RAB11A, ABCB1,GSK3B,GLA,G ORASP1,HSPB2,CSE1 L,JUNB,CYP3A4 | 4137,3727,595,372 5,7114,10376,3315 ,5708,1979,1678,1 978,891,983,8766, 5243,2932,2717,64 689,3316,1434,372 6,1576 | CG31057,CG2275,CG 9096,CG2275,CG494 4,CG1913,CG14207, CG7762,CG8846,CG1 728,CG8846,CG3510 ,CG5363,CG5771,CG 3879,CG2621,CG573 1,CG7809,CG14207, CG13281,CG2275,CG 9438 | B |
| palytoxin | CYP3A4 | 1576 | CG9438 | O |
| pamidronate | JUN | 3725 | CG2275 | B |
| p-Aminohippuric Acid | CYP4F3 | 4051 | CG3466 | B |
| panaxadiol | BEST1 | 7439 | CG6264 | B |
| pantoprazole | CDK2 | 1017 | CG5363 | B |
| PAPP | CYP3A4 | 1576 | CG9438 | O |
| Parthenolide | GORASP1,ADAMTS4,HSP A1A | 64689,9507,3303 | CG7809,CG14869,CG 5436 | |
| Patulin pazopanib | HSPA1A KDR, (EGR1) | 3303 3791, (1958) | CG5436 CG8222, (CG7847) | O |
| PCSO | KDR | 3791 | CG8222 | O |
| PD 169316 | HSPB1,ATF2,HSPB2 | 3315,1386,3316 | CG14207,CG30420,C G14207 | B |
| PD 173074 | HSPB2 | 3316 | CG14207 | B |
| PD 98059 | PAX6,JUN,GORASP1, JUND,NPDC1,PHOX2A ,JUNB,PPA1,FGF2,C YP24A1,ADAMTS9,EG R1,HGF,PLG,ABCB1, CCND1,FGF13,CDK2, FCN2 | 5080,3725,64689,3 727,56654,401,372 6,5464,2247,1591, 56999,1958,3082,5 340,5243,595,2258 ,1017,2220 | CG11186,CG2275,CG 7809,CG2275,CG304 20,CG11182,CG2275 ,CG4634,CG4608,CG 6042,CG14869,CG78 47,CG13744,CG1374 4,CG3879,CG9096,C G4608,CG5363,CG55 5 | B |
| PDBU | ETV4,ABCB1 | 2118,5243 | CG6892,CG3879 | O |
| Pectenotoxin II | CDC2,EGR1 | 983,1958 | CG5363,CG7847 | B |
| pegvisomant | EGR1 | 1958 | CG7847 | O |
| Pemetrexed | SLC19A1,TBXAS1,MT AP | 6573,6916,4507 | CG14694,CG3466,CG 4802 | |
| pentosidine | MTAP | 4507 | CG4802 | B |
| Pentoxifylline | TSC1,CCNB1,FCN2 | 7248,891,2220 | CG6147,CG3510,CG5 55 | B |
| Peplomycin | GORASP1,PREP | 64689,5550 | CG7809,CG5355 | B |
| Pepstatin A | CYP3A4,PLG | 1576,5340 | CG9438,CG13744 | O |
| Perazine | PLG | 5340 | CG13744 | O |
| Perillol | JUN,JUNB,JUND | 3725,3726,3727 | CG2275,CG2275,CG2 275 | B |
| Perindopril | JUND | 3727 | CG2275 | B |
| perylene bisimide | MBL2 | 4153 | CG7763 | B |
| phen | MAPT | 4137 | CG31057 | B |
| phen | PHC2 | 1912 | CG18414 | O |
| phenanthrene | MMEL1 | 79258 | CG9565 | O |
| phenothiazines | CYP4F3 | 4051 | CG3466 | B |
| Phenprocoumon | CSE1L,CYP3A4 | 1434,1576 | CG13281,CG9438 | O |
| Phenytoin | CYP3A4,CYP4F3,KLH L1 | 1576,4051,57626 | CG9438,CG3466,CG1 7754 | B |
| pheophorbide a | KLHL1 | 57626 | CG17754 | O |
| phloretin | GSK3B,TXNIP | 2932,10628 | CG2621,CG18745 | B |
| Picibanil | TXNIP | 10628 | CG18745 | B |
| picric acid | SMARCA2 | 6595 | CG5942 | B |
| picropodophyllin | PDXP | 57026 | CG4755 | B |
| pidotimod | IGF1R | 3480 | CG1842 | O |
| pifithrin | CCNB1,HSP90AA1 | 891,3320 | CG3510,CG1242 | B |
| pindobind | HSP90AA1 | 3320 | CG1242 | O |
| pioglitazone | SERPINC1,GORASP1,CYP 3A4,JUNB,JUN,JUND | 462,64689,1576,37 26,3725,3727 | CG9456,CG7809,CG9 438,CG2275,CG2275 ,CG2275 | B |
| Piroxicam | JUNB,SERPINB3,JUN ,JUND | 3726,6317,3725,37 27 | CG2275,CG9456,CG2 275,CG2275 | B |
| plumbagin | GORASP1,CDC2 | 64689,983 | CG7809,CG5363 | B |
| Pluronic p 85 | ABCB1,KLHL1 | 5243,57626 | CG3879,CG17754 | O |
| PMPA | KLHL1 | 57626 | CG17754 | O |
| PMSF | KLHL1 | 57626 | CG17754 | O |
| posaconazole | FAAH | 2166 | CG6007 | B |
| PP-IX | CYP3A4 | 1576 | CG9438 | O |
| Pragmoline | ABCB1 | 5243 | CG3879 | O |
| Pravastatin | CYP3A4,RAC1,JUNB, JUND,ABCB1,CYP4F3 ,JUN,PLAT | 1576,5879,3726,37 27,5243,4051,3725 ,5327 | CG9438,CG2248,CG2 275,CG2275,CG3879 ,CG3466,CG2275,CG 13744 | B |
| Prazosin | PLAT | 5327 | CG13744 | O |
| PRDL | FOXD3,CYP3A4,SMAR CB1,JUN,PLG | 27022,1576,6598,3 725,5340 | CG3668,CG9438,CG1 064,CG2275,CG1374 4 | B |
| Prednisone | MME,NF2,SERPINC1, ABCB1,BEST1 | 4311,4771,462,524 3,7439 | CG9565,CG14228,CG 9456,CG3879,CG626 4 | |
| preussin | BEST1 | 7439 | CG6264 | B |
| Primidone | CDK2 | 1017 | CG5363 | B |
| Proadifen | CYP3A4 | 1576 | CG9438 | O |
| Procasil | SLC5A5,HSPA1A | 6528,3303 | CG32669,CG5436 | O |
| Procetofen | CYP3A4,ABCB1 | 1576,5243 | CG9438,CG3879 | O |
| Prodigiosin | ABCB1 | 5243 | CG3879 | O |
| Prodix | GSK3B | 2932 | CG2621 | B |
| Promegestone | CYP3A4 | 1576 | CG9438 | O |
| Propofol | FAAH,CYP3A4 | 2166,1576 | CG6007,CG9438 | B |
| prostaglandin A1 | CYP3A4 | 1576 | CG9438 | O |
| prostaglandin D2 | SSTR1,TFDP1,GSK3B ,SSTR4,HTR1A | 6751,7027,2932,67 54,3350 | CG7285,CG4654,CG2 621,CG7285,CG7485 | B |
| prostaglandin E1 | HTR1A | 3350 | CG7485 | O |
| prostaglandin F2alpha | FGF2,FLT4,SERPINB 6 | 2247,2324,5269 | CG4608,CG8222,CG9 456 | O |
| prostaglandin H2 | CYP4F12,CYP4F8 | 66002,11283 | CG9438,CG3466 | O |
| prostaglandin J2 | HSPA4,KHDRBS1 | 3308,10657 | CG6603,CG4816 | O |
| prostaglandins | HGF,ATP1B1,CYP24A 1 | 3082,481,1591 | CG13744,CG9258,CG 6042 | |
| prostaglandins G | GORASP1,KDR | 64689,3791 | CG7809,CG8222 | B |
| prostratin | CCNT1,CCNT2 | 904,905 | CG6292,CG6292 | O |
| prucalopride | HTR4, (CCNT2) | 3360, (905) | CG6919, (CG6292) | O |
| prunustatin A | HTR4 | 3360 | CG6919 | O |
| Pseudohypericin | HSPA5 | 3309 | CG5436 | O |
| pseudolaric acid B | CYP3A4 | 1576 | CG9438 | O |
| psilocybin | JAG1 | 182 | CG6127 | O |
| Psoralens | ABCB1,CYP3A4 | 5243,1576 | CG3879,CG9438 | O |
| PTAP | CYP3A4 | 1576 | CG9438 | O |
| PTX-B | JUNB,JUND,JUN | 3726,3727,3725 | CG2275,CG2275,CG2 275 | B |
| puerarin | JUN | 3725 | CG2275 | B |
| Pugnac | JUN,JUNB,JUND | 3725,3726,3727 | CG2275,CG2275,CG2 275 | B |
| p-XSC | KLHL1 | 57626 | CG17754 | O |
| Pyrethrins | CYP3A4,CYP4F3 | 1576,4051 | CG9438,CG3466 | O |
| pyrvinium | HSP90B1,HSPA5 | 7184,3309 | CG1242,CG5436 | O |
| quercitrin | HSPA5 | 3309 | CG5436 | O |
| quetiapine | HTR1A,CYP3A4 | 3350,1576 | CG7485,CG9438 | O |
| Quicifal | CYP3A4 | 1576 | CG9438 | O |
| quinupristin-dalfopristin | CYP3A4 | 1576 | CG9438 | O |
| R 101933 | CYP3A4 | 1576 | CG9438 | O |
| rabeprazole | ABCB1 | 5243 | CG3879 | O |
| radester | ABCB1 | 5243 | CG3879 | O |
| Raloxifene | JUND,NCOR1,JUNB,A BCB1,BEST1,JUN,CY P3A4 | 3727,9611,3726,52 43,7439,3725,1576 | CG2275,CG7951,CG2 275,CG3879,CG6264 ,CG2275,CG9438 | B |
| ramiprilat | JUN,JUND,FBN1,JUN B | 3725,3727,2200,37 26 | CG2275,CG2275,CG3 936,CG2275 | B |
| rebamipide | JUNB | 3726 | CG2275 | B |
| reboxetine | JAG1 | 182 | CG6127 | O |
| remifentanil | CYP3A4 | 1576 | CG9438 | O |
| renzapride | HTR4, (OPRM1) | 3360, (4988) | CG6919, (CG7285) | B |
| repaglinide | HTR4 | 3360 | CG6919 | O |
| Requip | CYP3A4 | 1576 | CG9438 | O |
| Revex | JUN | 3725 | CG2275 | B |
| Rhodinal | OPRK1 | 4986 | CG7285 | B |
| Riacon | HSPB1,HSPB2 | 3315,3316 | CG14207,CG14207 | B |
| Ribavirin | HSPB2 | 3316 | CG14207 | B |
| Rifabutin | CYP3A4,HSP90AA1,C YP4F3 | 1576,3320,4051 | CG9438,CG1242,CG3 466 | O |
| Riluzole | CYP4F3 | 4051 | CG3466 | B |
| rimorphin | HSPA4 | 3308 | CG6603 | O |
| risedronic acid | OPRK1 | 4986 | CG7285 | B |
| risperidone | CYP3A4,ABCB1 | 1576,5243 | CG9438,CG3879 | O |
| Ritodrine | ATP2B2,ATP2B2 | 491,491 | CG2167,CG2172 | O |
| Ritonavir | CYP3A7,NF2,CYP27A 1,ABCB1,CYP24A1,C YP3A4 | 1551,4771,1593,52 43,1591,1576 | CG9438,CG14228,CG 6042,CG3879,CG604 2,CG9438 | B |
| rituximab | JUND,JUNB,JUN,PDP K1,ABCB1 | 3727,3726,3725,51 70,5243 | CG2275,CG2275,CG2 275,CG1210,CG3879 | B |
| RMI 12330A | ABCB1 | 5243 | CG3879 | O |
| Ro 13-8996 | CYP27A1 | 1593 | CG6042 | B |
| Ro 64-0802 | CYP3A4 | 1576 | CG9438 | O |
| Robitet | ABCB1,SLC5A6,CCND 1,PLAT,FGF2,HGF,T IMM8A,HSPA4 | 5243,8884,595,532 7,2247,3082,1678, 3308 | CG3879,CG32669,CG 9096,CG13744,CG46 08,CG13744,CG1728 ,CG6603 | O |
| Rolipram | CHAT,MAPT | 1103,4137 | CG12345,CG31057 | O |
| romidepsin | JUND,JUNB,ABCB1,J UN | 3727,3726,5243,37 25 | CG2275,CG2275,CG3 879,CG2275 | O |
| ropivacaine | JUN | 1678 | CG1728 | O |
| Roquefortine | CYP3A4 | 3308 | CG6603 | O |
| roscovitine | HSPB1,HSPB2,GORAS P1,MAPT,CDK6,CCND 2,CCNT1,CCND1,CDC 2,CDK2 | 3315,3316,64689,4 137,1021,894,904, 595,983,1017 | CG14207,CG14207,C G7809,CG31057,CG5 072,CG9096,CG6292 ,CG9096,CG5363,CG 5363 | B |
| rosiglitazone | ATP2A2, JUND, JUNB, TSC1,JUN,GORASP1, IGF1R,CYP3A4,CIDE A | 488,3727,3726,724 8,3725,64689,3480 ,1576,1149 | CG32451,CG2275,CG 2275,CG6147,CG227 5,CG7809,CG1842,C G9438,CG1975 | B |
| rosmarinic acid | JUN,JUNB,JUND | 3725,3726,3727 | CG2275,CG2275,CG2 275 | O |
| rosuvastatin | ABCB1,KLHL1,SLC22 A7 | 5243,57626,10864 | CG3879,CG17754,CG 8654 | |
| Rozevin | JUN,KLHL1,MAPT,CD C2,ABCB1,JUNB,JUN D | 3725,57626,4137,9 83,5243,3726,3727 | CG2275,CG17754,CG 31057,CG5363,CG38 79,CG2275,CG2275 | O |
| RPR 121056 Rulid | JUND SLC5A5,ABCB1,FLT1 | 10864 6528,5243,2321 | CG8654 CG32669,CG3879,CG 8222 | O |
| rutecarpine | FLT1 | 983 | CG5363 | B |
| Rutin | ABCB1,HSPA4,HSP90 AA1 | 5243,3308,3320 | CG3879,CG6603,CG1 242 | B |
| S 17092-1 | PREP, (HSP90AA1) | 5550, (1576) | CG5355, (CG9438) | O |
| S 9788 | PREP | 6528 | CG32669 | O |
| S azabisabolene | ABCB1 | 5243 | CG3879 | O |
| Safingol | PPA1 | 2321 | CG8222 | O |
| SAHA | KPNA2,ADAMTS1,JAK 1,CCND1,JUND,JUN, TXNIP,DAB1,HSP90A A1,JUNB | 3838,9510,3716,59 5,3727,3725,10628 ,1600,3320,3726 | CG4799,CG14869,CG 1594,CG9096,CG227 5,CG2275,CG18745, CG9695,CG1242,CG2 | O |
| | | | 275 | |
| saintopin | JUNB | 3716 | CG1594 | B |
| Salicin | SSTR1,SSTR4,HTR1A | 6751,6754,3350 | CG7285,CG7285,CG7 485 | B |
| salinomycin | HTR1A | 10628 | CG18745 | B |
| salinosporamide A | ABCB1 | 1600 | CG9695 | O |
| salubrinal | EIF2S1,HSPA5 | 1965,3309 | CG9946,CG5436 | B |
| salvin | HSPA5 | 7153 | CG10223 | B |
| salvinorin A | CCNA2 | 6751 | CG7285 | B |
| samarium | OPRK1 | 6754 | CG7285 | B |
| sampatrilat | PSMD6 | 3350 | CG7485 | O |
| sangivamycin | MME | 5243 | CG3879 | O |
| Saquinavir | CYP3A7,ABCB1,CYP3 A4 | 1551,5243,1576 | CG9438,CG3879,CG9 438 | O |
| Sarasar | CYP3A4,KLHL1 | 1576,57626 | CG9438,CG17754 | B |
| sarizotan | KLHL1 | 9861 | CG5378 | O |
| SB 203580 | CCNB1,EGR1,PLAT,P GLYRP2,PLG,HSPB2, JUND,SSTR4,JUN,JU NB,SSTR1,GORASP1, CDC2,HSPB1,HGF,CY P27B1,HTR1A,ATF2, CCND1,CCNA2 | 891,1958,5327,114 770,5340,3316,372 7,6754,3725,3726, 6751,64689,983,33 15,3082,1594,3350 ,1386,595,890 | CG3510,CG7847,CG1 3744,CG14704,CG13 744,CG14207,CG227 5,CG7285,CG2275,C G2275,CG7285,CG78 09,CG5363,CG14207 ,CG13744,CG6042,C G7485,CG30420,CG9 096,CG3510 | B |
| SB 207266 | HTR4, (CCNA2) | 3360, (983) | CG6919, (CG5363) | O |
| SB 216763 | CCND1,GSK3B,GSK3A ,ATP2A2 | 595,2932,2931,488 | CG9096,CG2621,CG2 621,CG32451 | O |
| SB 225002 | HTR1A,SSTR1,SSTR4 | 3350,6751,6754 | CG7485,CG7285,CG7 285 | B |
| SB 415286 | GSK3B,RAC1,GSK3A | 2932,5879,2931 | CG2621,CG2248,CG2 621 | B |
| SB-649915 | GSK3A | 2931 | CG2621 | B |
| SB-706375 | HTR1A | 488 | CG32451 | B |
| SCH 66712 | RPS6KA2 | 3350 | CG7485 | O |
| schizandrin B | CYP3A4 | 6751 | CG7285 | B |
| SCIO-469 | HSPB1,HSPB2 | 3315,3316 | CG14207,CG14207 | B |
| scoparone | HSPB2 | 5879 | CG2248 | B |
| Sediel | GORASP1 | 2931 | CG2621 | B |
| selamectin | HTR1A | 3350 | CG7485 | O |
| Selegiline | ABCB1 | 6196 | CG17596 | O |
| Serad | JUN | 1576 | CG9438 | O |
| sesamin | CYP3A4 | 5243 | CG3879 | O |
| sevoflurane | JUN,PIK3C2A,JUNB, JUND | 3725,5286,3726,37 27 | CG2275,CG11621,CG 2275,CG2275 | |
| Sildenafil | JUND | 5243 | CG3879 | O |
| silybin | CCND3,JUND,JUNB,I GF1R,JUN,GORASP1, CYP3A4,CDK2,ABCB1 ,CCND1 | 896,3727,3726,348 0,3725,64689,1576 ,1017,5243,595 | CG9096,CG2275,CG2 275,CG1842,CG2275 ,CG7809,CG9438,CG 5363,CG3879,CG909 6 | B |
| Sizofiran | CCND1 | 3726 | CG2275 | B |
| sofalcone | WNK1 | 3480 | CG1842 | O |
| sorafenib | KDR,GSK3B,PSMB5,C CND1,CYP3A4,EIF2S 1 | 3791,2932,5693,59 5,1576,1965 | CG8222,CG2621,CG1 2323,CG9096,CG943 8,CG9946 | B |
| SP 100030 | JUN,JUNB,JUND | 3725,3726,3727 | CG2275,CG2275,CG2 275 | O |
| sparfloxacin | JUND | 2932 | CG2621 | B |
| spiradoline | ABCB1 | 5693 | CG12323 | O |
| spiraprilat | OPRK1 | 595 | CG9096 | B |
| spirogermanium | CSE1L | 1576 | CG9438 | O |
| SR 48692 | SERPINB1,EGR1 | 1992,1958 | CG9456,CG7847 | B |
| SR 59230A | EGR1 | 3726 | CG2275 | B |
| SRI 63-154 | RAC1 | 3727 | CG2275 | B |
| SRIH | SSTR3,SSTR4,SSTR1 ,SSTR5,MAGT1,SSTR 2 | 6753,6754,6751,67 55,84061,6752 | CG7285,CG7285,CG7 285,CG7285,CG7830 ,CG7285 | O |
| ST 1481 | SSTR2 | 5879 | CG2248 | B |
| STA 5326 | ABCB1 | 9159 | CG10772 | O |
| stachybotrydial | | 6753 | CG7285 | B |
| Stanozolol | PLG | 6754 | CG7285 | B |
| Stavudine | SERPINC1 | 6751 | CG7285 | B |
| SU 1498 | CSE1L | 6755 | CG7285 | B |
| SU 5416 | KDR | (84061), 3791 | (CG7830), CG8222 | O |
| SU 5614 | KDR | 6752 | CG7285 | B |
| SU 6656 | KDR | 5243 | CG3879 | O |
| SU 6668 | RAC1 | 3921 | CG14792 | O |
| SU 9516 | CDK2,CCND1 | 1017,595 | CG5363,CG9096 | B |
| Sucralfate | CCND1 | 1434 | CG13281 | O |
| Sufentanil | PHC2 | 3791 | CG8222 | O |
| Sulem | OPRM1 | 3791 | CG8222 | O |
| Sulfamerazine | HSPA4 | 3791 | CG8222 | O |
| Sulfamethazine | MYB | 5879 | CG2248 | B |
| sulfamide | CYP3A4 | 3791 | CG8222 | O |
| Sulfaphenazole | JUNB,JUND,CYP4F3, JUN,CYP3A5 | 3726,3727,4051,37 25,1577 | CG2275,CG2275,CG3 466,CG2275,CG3466 | B |
| Sulfoximine | JUN | 3308 | CG6603 | O |
| Sulindac | CCND1,TSC1,ABCB1 | 595,7248,5243 | CG9096,CG6147,CG3 879 | B |
| sultopride | ABCB1 | 3726 | CG2275 | B |
| Sumatriptan | ABCB1 | 3727 | CG2275 | B |
| sunitinib | KDR,CYP3A4 | 3791,1576 | CG8222,CG9438 | B |
| sural | MMEL1,RPSA | 79258,3921 | CG9565,CG14792 | B |
| T 0901317 | RPSA | 7248 | CG6147 | O |
| TAC 101 | JUNB,JUND,JUN | 3726,3727,3725 | CG2275,CG2275,CG2 275 | O |
| Tacrolimus | CYP3A4,ABCB1 | 1576,5243 | CG9438,CG3879 | O |
| Tamogel | CCND2,CYP3A4 | 894,1576 | CG9096,CG9438 | O |
| tamsulosin | CYP3A4 | 1576 | CG9438 | O |
| tandospirone | CYP3A4,HTR1A | 1576,3350 | CG9438,CG7485 | B |
| Tangeretin | HTR1A | 3725 | CG2275 | B |
| tanshinone | ABCB1,CYP3A4 | 5243,1576 | CG3879,CG9438 | O |
| tariquidar | CYP3A4 | 894 | CG9096 | B |
| Taseron | ABCB1 | 1576 | CG9438 | O |
| Taurolin | HTR1A | 146 | CG18741 | O |
| TAXOTERE | KLHL1,GLA,WNK1,CC ND1,CYP4F3,RAC1,A BCB1,CCNB1,TUBA1B ,CDC2,CYP3A4 | 57626,2717,65125, 595,4051,5879,524 3,891,10376,983,1 576 | CG17754,CG5731,CG 7177,CG9096,CG346 6,CG2248,CG3879,C G3510,CG1913,CG53 63,CG9438 | O |
| tazarotene | TSC1,APC | 7248,324 | CG6147,CG6193 | B |
| TBDZ | CYP4F3,HSPA5,HSPA 4 | 4051,3309,3308 | CG3466,CG5436,CG6 603 | B |
| TBHQ | JUND,JUNB,JUN | 3727,3726,3725 | CG2275,CG2275,CG2 275 | O |
| TCDD | JUN,PLAT,CYP3A7,P PIF,ABCB1,CYP27B1 ,CYP4F3 | 3725,5327,1551,10 105,5243,1594,405 1 | CG2275,CG13744,CG 9438,CG2852,CG387 9,CG6042,CG3466 | B |
| Tegafur | WNK1,MAGT1 | 65125,84061 | CG7177,CG7830 | B |
| tegaserod | HTR4, (MAGT1) | 3360, (5327) | CG6919, (CG13744) | O |
| telithromycin | | 1551 | CG9438 | O |
| telmisartan | CYP3A4 | 10105 | CG2852 | O |
| temozolomide | EIF2S1,CDC2 | 1965,983 | CG9946,CG5363 | B |
| temsirolimus | CCND1,CYP3A4 | 595,1576 | CG9096,CG9438 | O |
| Teniposide | TOP2B,JUN | 7155,3725 | CG10223,CG2275 | O |
| Terfenadine | JUN | 1576 | CG9438 | O |
| Teriparatide | PLG,BEST1,HSPA4 | 5340,7439,3308 | CG13744,CG6264,CG 6603 | B |
| Tetraprenol | HSPA4 | 595 | CG9096 | B |
| TG101209 | TBXAS1 | 1576 | CG9438 | O |
| thalicarpine | JAK2 | 7155 | CG10223 | B |
| Thapsigargin | CDK2,HSPB1,HSPA4, HSPB2,RPSA,HSP90B 1,HTR1A,HSPA1A,JU N,HSPA1B,ABCB1,HS PA5,HSPA2,GSK3B,X 1 | 1017,3315,3308,33 16,3921,7184,3350 ,3303,3725,3304,5 243,3309,3306,293 2,7494 | CG5363,CG14207,CG 6603,CG14207,CG14 792,CG1242,CG7485 ,CG5436,CG2275,CG 31449,CG3879,CG54 36,CG5436,CG2621, CG9415 | |
| Theaflavin | JUND,JUNB,JUN | 3727,3726,3725 | CG2275,CG2275,CG2 275 | B |
| Thiazolidinediones | NRF1,CYP3A4,BEST1 ,CCND1 | 4899,1576,7439,59 5 | CG3114,CG9438,CG6 264,CG9096 | B |
| thiocoraline | CCND1 | 7494 | CG9415 | B |
| thioridazine | CYP3A4 | 3727 | CG2275 | B |
| Thiorphan | MME,ECE1 | 4311,1889 | CG9565,CG9565 | B |
| thromboxane B2 | ECE1 | 4899 | CG3114 | B |
| thulium | SERPINC1 | 1576 | CG9438 | O |
| thymalfasin | ABCB1,JUND,JUN,JU NB | 5243,3727,3725,37 26 | CG3879,CG2275,CG2 275,CG2275 | O |
| Thymopentin | JUNB | 4311 | CG9565 | O |
| thymoquinone | MME | 1889 | CG9565 | O |
| Ticlopidine | SERPINC1,CYP4F3 | 462,4051 | CG9456,CG3466 | B |
| Tilidine | CYP3A4,CSE1L,ABCB 1 | 1576,1434,5243 | CG9438,CG13281,CG 3879 | B |
| tipifarnib | GORASP1,ABCB1 | 64689,5243 | CG7809,CG3879 | O |
| tipranavir | ABCB1 | 64689 | CG7809 | B |
| tirilazad | CYP3A4 | 462 | CG9456 | B |
| TKI-31 | CYP3A4 | 4051 | CG3466 | B |
| Tmndga | KDR | 1576 | CG9438 | O |
| TMPN | ABCB1 | 1434 | CG13281 | O |
| Toddalin | JUN,HSPB1,GORASP1 | 3725,3315,64689 | CG2275,CG14207,CG 7809 | O |
| Todralazine | GORASP1 | 1576 | CG9438 | O |
| tofisopam | PREP | 1576 | CG9438 | O |
| Tolterodine | CYP4F3,CSE1L | 4051,1434 | CG3466,CG13281 | O |
| topiramate | CSE1L | 3725 | CG2275 | B |
| Topotecan | HSPA4,CYP3A4,ABCB 1 | 3308,1576,5243 | CG6603,CG9438,CG3 879 | B |
| Toremifene | ABCB1 | 5550 | CG5355 | O |
| Toxaphene | SERPINC1 | 1576 | CG9438 | O |
| Tramadol | TSC1 | 4051 | CG3466 | B |
| Tramat | HSP90B1,MAPT,CCND 1,DMRT1,HSP90AA1 | 7184,4137,595,176 1,3320 | CG1242,CG31057,CG 9096,CG11094,CG12 42 | O |
| trandolapril | HSP90AA1 | 462 | CG9456 | B |
| Trapidil | PLAT | 7248 | CG6147 | O |
| trapoxin A | FGF2 | 5243 | CG3879 | O |
| trastuzumab | CCNA2,CCND1,PDPK1 ,UHMK1,TOP2A,CDK2 ,NOTCH1,RAC1,CDK6 | 890,595,5170,1279 33,7153,1017,4851 ,5879,1021 | CG3510,CG9096,CG1 210,CG3162,CG1022 3,CG5363,CG3936,C G2248,CG5072 | B |
| Trazodone | CDK6 | 595 | CG9096 | B |
| Tremode | ABCB1 | 5170 | CG1210 | B |
| triazolam | CYP3A4 | 127933 | CG3162 | O |
| triazolobenzodiazepines | CYP3A4 | 7153 | CG10223 | B |
| tributylstannane | PLAT | 1017 | CG5363 | B |
| trichostatin A | CYP4F3 | 4851 | CG3936 | B |
| trichostatins | CDK6,CCND1,CDK4 | 1021,595,1019 | CG5072,CG9096,CG5 072 | O |
| Trifluoperazine | ABCB1,EGR1 | 5243,1958 | CG3879,CG7847 | O |
| trioctyl phosphine oxide | MYBL2,TFDP1,APC,M YB | 4605,7027,324,460 2 | CG9045,CG4654,CG6 193,CG9045 | O |
| Triolein | MYB | 595 | CG9096 | B |
| triptolide | JUND,JUNE,JUN,GOR ASP1,HSPA4,GSK3B | 3727,3726,3725,64 689,3308,2932 | CG2275,CG2275,CG2 275,CG7809,CG6603 ,CG2621 | O |
| TRK 820 | GSK3B | 4602 | CG9045 | B |
| troglitazone | CYP3A7,CYP3A4,JUN D,MYB,JUN,EGR1,CD C2,JUNB,CDK4,CDK2 ,CDK6,CCND1,CCNE1 ,CCND3,CCND2 | 1551,1576,3727,46 02,3725,1958,983, 3726,1019,1017,10 21,595,898,896,89 4 | CG9438,CG9438,CG2 275,CG9045,CG2275 ,CG7847,CG5363,CG 2275,CG5072,CG536 3,CG5072,CG9096,C G3938,CG9096,CG90 96 | B |
| Troleandomycin | CYP4F3,CYP3A4,CYP 3A7 | 4051,1576,1551 | CG3466,CG9438,CG9 438 | B |
| tropisetron | HTR4, (CYP3A7) | 3360, (1021) | CG6919, (CG5072) | O |
| Trospium chloride | HTR4 | 595 | CG9096 | B |
| Tunicamycin | CCNA2,HSPA4,HSPA5 ,NRF1,ABCB1,EIF2S 1,HSP90B1,CCND1 | 890,3308,3309,489 9,5243,1965,7184, 595 | CG3510,CG6603,CG5 436,CG3114,CG3879 ,CG9946,CG1242,CG 9096 | O |
| tylophorine | JUND,JUNB,JUN | 3727,3726,3725 | CG2275,CG2275,CG2 275 | O |
| Tylox | OPRM1,ABCB1 | 4988,5243 | CG7285,CG3879 | O |
| tyrphostin AG-490 | IGF1R,JUND,RAC1,R PS6KA1,EGR1,JUN,J UNB,CCND1,RAC1,AD AMTS4,JAK2 | 3480,3727,5879,61 95,1958,3725,3726 ,595,5879,9507,37 17 | CG1842,CG2275,CG2 248,CG17596,CG784 7,CG2275,CG2275,C G9096,CG2248,CG14 869,CG1594 | B |
| tyvelose | JAK2 | 6195 | CG17596 | O |
| U 0126 | FCN2,GORASP1,HGF, CCND1,RPSA,PLAT,J UND,ADAMTS4,CCNA2 ,JUNB,CDK4,EGR1,A BCB1,JUN,EGR2 | 2220,64689,3082,5 95,3921,5327,3727 ,9507,890,3726,10 19,1958,5243,3725 ,1959 | CG555,CG7809,CG13 744,CG9096,CG1479 2,CG13744,CG2275, CG14869,CG3510,CG 2275,CG5072,CG784 7,CG3879,CG2275,C G7847 | B |
| U 69593 | EGR2 | 9507 | CG14869 | O |
| Ubizol | GORASP1,HSPA4 | 64689,3308 | CG7809,CG6603 | B |
| UH 301 | HSPA4 | 1019 | CG5072 | O |
| Usaf B-12 | CCNA2,SERPINC1,PL G,HSPAS,JUND,PLAT ,SLC19A1,EIF2S1,H SPA4,FBN1,JUNB,JU N | 890,462,5340,3309 ,3727,5327,6573,1 965,3308,2200,372 6,3725 | CG3510,CG9456,CG1 3744,CG5436,CG227 5,CG13744,CG14694 ,CG9946,CG6603,CG 3936,CG2275,CG227 5 | O |
| USAN | HSPA8,CCND1,MME,S ER-PINB3,CDK2,TMSB4X ,HSPA4,KLHL1,HGF | 3312,595,4311,631 7,1017,7114,3308, 57626,3082 | CG31449,CG9096,CG 9565,CG9456,CG536 3,CG4944,CG6603,C G17754,CG13744 | B |
| Valproic Acid | GSK3B,JUNB,JUN,TS C1,JUND,HTR1A,MME ,NOTCH1,MMEL1,CCN D1,CYP3A4,NF2,APC ,HGF,HSPA4,HSPA5, ABCB1,DAB1,PREP | 2932,3726,3725,72 48,3727,3350,4311 ,4851,79258,595,1 576,4771,324,3082 ,3308,3309,5243,1 600,5550 | CG2621,CG2275,CG2 275,CG6147,CG2275 ,CG7485,CG9565,CG 3936,CG9565,CG909 6,CG9438,CG14228, CG6193,CG13744,CG 6603,CG5436,CG387 9,CG9695,CG5355 | O |
| valsartan | PREP | 4771 | CG14228 | B |
| valspodar | CYP3A4,ABCB1 | 1576,5243 | CG9438,CG3879 | O |
| vandetanib | KDR,ABCB1 | 3791,5243 | CG8222,CG3879 | O |
| vapreotide | SSTR2,SSTR1 | 6752,6751 | CG7285,CG7285 | O |
| venlafaxine | SSTR1 | 5550 | CG5355 | O |
| Verapamil | PLAT,MAPT,VHLL,AB CB1,CYP3A4,KLHL1 | 5327,4137,391104, 5243,1576,57626 | CG13744,CG31057,C G13221,CG3879,CG9 438,CG17754 | B |
| verlukast | ABCB1,KLHL1 | 5243,57626 | CG3879,CG17754 | O |
| verrucosidin | KLHL1 | 5327 | CG13744 | O |
| versipelostatin | HSPA5 | 4137 | CG31057 | B |
| VGA1155 | KDR,FLT1 | 3791,2321 | CG8222,CG8222 | O |
| Vigil | FLT1 | 1576 | CG9438 | O |
| vincaleukoblastine | CYP3A4 | 57626 | CG17754 | O |
| vincristine | HSPB2,RPSA,EIF2S1 ,TIMM8A,TXNL1,CYP 3A4,CCNB1,ABCB1,K | 3316,3921,1965,16 78,9352,1576,891, 5243,57626,3315,2 | CG14207,CG14792,C G9946,CG1728,CG54 95,CG9438,CG3510, | O |
| | LHL1,HSPB1,FOXD3 | 7022 | CG3879,CG17754,CG 14207,CG3668 | |
| Vindesine | FOXD3 | 1678 | CG1728 | O |
| vinorelbine | GLA,CYP4F3 | 2717,4051 | CG5731,CG3466 | O |
| Visken | CYP4F3 | 891 | CG3510 | B |
| Viviq | IK | 27022 | CG3668 | B |
| voriconazole | CYP3A4,MAPT | 1576,4137 | CG9438,CG31057 | B |
| vorozole | MAPT | 4051 | CG3466 | B |
| vulnibactin | SERPINB1 | 3350 | CG7485 | O |
| Wakil | FURIN | 50846 | CG4637 | O |
| Warfarin | CYP4F2,CYP3A4,CSE 1L | 8529,1576,1434 | CG3466,CG9438,CG1 3281 | B |
| Wartmannin | JUNB,JAK2,RAC1,MS N,GSK3B,JUN,JUND, CCNB1,FGF2,CYP3A4 ,CCND1,FGF7,ETV5, GORASP1,HGF | 3726,3717,5879,44 78,2932,3725,3727 ,891,2247,1576,59 5,2252,2119,64689 ,3082 | CG2275,CG1594,CG2 248,CG10701,CG262 1,CG2275,CG2275,C G3510,CG4608,CG94 38,CG9096,CG4608, CG6892,CG7809,CG1 3744 | B |
| WAY 100635 | HGF | 891 | CG3510 | B |
| Wogonin | JUND,JUN,JUNB | 3727,3725,3726 | CG2275,CG2275,CG2 275 | B |
| WR 1065 | JUNB | 2252 | CG4608 | B |
| WS 79089B | GORASP1 | 2119 | CG6892 | O |
| xanthohumol | CYP3A4,ABCB1 | 1576,5243 | CG9438,CG3879 | O |
| Xaxa | PLAT,MLH1,JUND,SE RPINC1,JUN,GORASP 1,CCND1,JUNB,ABCB 1 | 5327,4292,3727,46 2,3725,64689,595, 3726,5243 | CG13744,CG11482,C G2275,CG9456,CG22 75,CG7809,CG9096, CG2275,CG3879 | O |
| ximelagatran | ABCB1 | 4292 | CG11482 | B |
| Xylit | ABCB1 | 3727 | CG2275 | B |
| Y 27632 | JUND,RAC1,JUNB,JU N | 3727,5879,3726,37 25 | CG2275,CG2248,CG2 275,CG2275 | B |
| YM-201627 | JUN | 3726 | CG2275 | B |
| YM-231146 | FGF2 | 5243 | CG3879 | O |
| zacopride | HTR4, (KDR) | 3360, (5243) | CG6919, (CG3879) | O |
| zafirlukast | HTR4 | 5550 | CG5355 | O |
| ZD 4190 | CYP3A4 | 3727 | CG2275 | B |
| zeaxanthin | KDR | 5879 | CG2248 | B |
| Zeldox | ABCB1 | 3726 | CG2275 | B |
| zileuton | CYP3A4 | 3725 | CG2275 | B |
| Zimco | JUND,JUN,JUNB | 3727,3725,3726 | CG2275,CG2275,CG2 275 | O |
| zincov | PPA1,MME | 5464,4311 | CG4634,CG9565 | O |
| ZK 112993 | MME | 5243 | CG3879 | O |
| ZM323881 | RPSA | 1576 | CG9438 | O |
| zopiclone | KDR | 4051 | CG3466 | B |
| ZSTK474 | CYP3A4 | 3727 | CG2275 | B |
| Zymosan | PSMC1,MBL2 | 5700,4153 | CG5289,CG7763 | B |

In preferred embodiments, the present invention is definded as follows:
Definition 1. The method of reducing weight and/or body fat in a subject comprising the administration of a therapeutic compound selected from the compounds of table 1.
Definition 2. The method of reducing weight and/or body fat in a subject or to treat obesity comprising the administration of an antagonist of one or more of the genes selected from CG30184, CG10369, CG32401, CG2374, CG8693, CG14909, CG13299, CG7847, CG30462, CG30462, CG15169, CG1650, CG6577, CG30491, CG4373, CG10407, CG2198, CG6356, CG5744, CG9506, CG31169, CG1728, CG9220, CG15625, CG5550, CG13088, CG13188, CG14968, CG1503, CG1666, CG14869, CG2702, CG2984, CG4394, CG9922, CG14529, CG17781, CG17781, CG9153, CG15178, CG5641, CG3879, CG15579, CG1422, CG6299, CG8107, CG7103, CG10617, CG30360, CG32971, CG32336, CG31036, CG12602, CG9676, CG1433, CG1100, CG31697, CG7095, CG2165, CG10230, CG10916, CG3274, CG18767, CG5072, CG3396, CG15582, CG16826, CG6788, CG9487, CG1888, CG4637, CG15162, CG5719, CG2254, CG4695, CG14936, CG17867, CG15646, CG5402, CG15095, CG8250, CG18030, CG14303, CG14164, CG14677, CG12105, CG17440, CG32459, CG11404, CG8954, CG13138, CG9056, CG12997, CG12997, CG5436, CG14330, CG10809, CG1622, CG3893, CG1112, CG31690, CG12664, CG13679, CG17556, CG10062, CG31744, CG9760, CG1555, CG14375, CG32170, CG4271, CG32234, CG7287, CG14341, CG30486, CG31692, CG31421, CG5467, CG30065, CG9086, CG1688, CG17026, CG4415, CG10343, CG15388, CG13984, CG3313, CG13116, CG4662, CG6919, CG17841, CG30411, CG9053, CG1180, CG14166, CG13125, CG13344, CG1490, CG2867, CG5591, CG14362, CG1531, CG15390, CG6689, CG14234, CG14265, CG5674, CG3917, CG8257, CG9028, CG1722, CG18402, CG7082, CG11797, CG3663, CG16704, CG31172, CG31219, CG1363, CG6721, CG5688, CG8527, CG13137, CG6612, CG6947, CG7737, CG1705, CG14704, CG10300, CG3597, CG3425, CG2540, CG6856, CG12259, CG4583, CG3843, CG9634, CG3809, CG9295, CG9485, CG11555, CG11601, CG14095, CG10166, CG2852, CG14164, CG14164, CG2898, CG3162, CG6603, CG8721, CG17742, CG14127, CG8665, CG9438, CG32113, CG32353, CG4957, CG33558, CG11570, CG32669, CG11575, CG30271, CG7830, CG31061, CG2076, CG17596, CG6824, CG17921, CG12875, CG13020, CG13972, CG13673, CG10772, CG8079, CG13127, CG9144, CG8979, CG7097, CG11768, CG10632, CG14903, CG1874, CG33466, CG3367, CG4851, CG17985, CG31229, CG3260, CG13023, CG11125, CG17184, CG31812, CG13360, CG30075, CG30183, CG7485, CG5495, CG5495, CG7065, CG13202, CG7779, CG9322, CG7091, CG16758, CG5071, CG4920, CG1516, CG9554, CG10101, CG3004, CG7796, CG10152, CG18741, CG8444, CG11425, CG10128, CG10542, CG11878, CG14434, CG12345, CG2091, CG31459, CG13319, CG7177, CG7776, CG15005, CG31605, CG7213, CG17283, CG18268, CG3017, CG7567, CG32091, CG9695, CG8222, CG1515, CG8256, CG1975, CG32467, CG3817, CG4038, CG6193, CG1572, CG8117, CG3526, CG7099, CG18525, CG9198, CG30470, CG17273, CG31439, CG1387, CG9952, CG6580, CG10840, CG13221, CG8202, CG8786, CG7199, CG11663, CG12683, CG31161, CG8009, CG17202, CG1683, CG17335, CG33204, CG14694, CG11229, CG16836, CG12209, CG18414, CG13475, CG11621, CG13332, CG11756, CG11133, CG18586, CG4944, CG3213, CG4152, CG6147, CG8515, CG5827, CG12691, CG8308, CG13807, CG2260, CG30004, CG4247, CG4247, CG5739, CG4202, CG4264, CG5245, CG13707, CG3523, CG10686, CG9565, CG4111, CG14673, CG31132, CG5355, CG32149, CG8443, CG17461, CG8190, CG13744, CG9258, CG6043, CG1759, CG8534, CG14792, CG8451, CG8654, CG12806, CG14938, CG9399, CG10542, CG13168, CG31845, CG6277, CG17819, CG2818, CG1688, CG13868, CG17736, CG7546, CG31693, CG12897, CG2146, CG3440, CG3696, CG12426, CG18319, CG18279, CG18279, CG3054, CG2145, CG3825, CG9781, CG13423, CG12030, CG14911, CG3911, CG6122, CG7206, CG8566, CG30476, CG9470, CG6127, CG5381, CG12505, CG1279, CG32140, CG12184, CG31364, CG1963, CG5484, CG4634, CG9748, CG32442, CG1921, CG18740, CG1242, CG9946, CG11121, CG3497, CG6817, CG30080, CG1171, CG11430, CG10691, CG13281, CG11352, CG3839, CG14368, CG14024, CG9936, CG11505, CG11906, CG1263, CG14011, CG11339, CG12015, CG30389, CG17331, CG15432, CG15507, CG14842, CG3906, CG17754, CG5289, CG5378, CG5625, CG6156, CG13243, CG8239, CG1821, CG7762, CG3108, CG8053, CG3605, CG4207, CG8431, CG9098, CG5270, CG5595, CG6064, CG6967, CG7134, CG7549, CG6892, CG10687, CG10712, CG11981, CG12770, CG15599, CG18563, CG7770, CG6322, CG3806, CG3980, CG6054, CG7292, CG3992, CG2998, CG8337, CG13194, CG5147, CG16903, CG11202, CG10084, CG12323, CG31484, CG6949, CG7352, CG10728, CG11376, CG32210, CG7109, CG8615, CG9160, CG8298, CG15115, CG1965, CG12595, CG15321, CG6009, CG11267, CG4453, CG3971, CG17255, CG32791, CG14016, CG14016, CG1740, CG32667 or an ortholog thereof.
Definition 3. The method according to definition 1 or 2, characterized in that the compound or antagonist is administered in a effective therapeutic dose.
Definition 4. The method of any one of definitions 1 to 3, characterized in that the compound is administered topical, enteral or parenteral, in particular preferred oral or rectal, intravenous, intraarterial, intramuscular, subcutaneous, intradermal or intraperitoneal, transdermal, transmucosal or inhalational.
Definition 5. The method of any one of definitions 1 to 4, characterized in that the subject is mammal, preferably a human.
Definition 6. The method of any one of definitions 1 to 5, characterized in that the compound or antagonist is provided in a medicament.
Definition 7. The method of any one of definitions 1 to 6, characterized in that the compound or antagonist is provided together with a pharmaceutically acceptable carrier or buffer.
Definition 8. The method of any one of definitions 1 to 6, characterized in that the compound or antagonist is administered in a dosage of between 0.01 mg/kg and 1 g/kg.
Definition 9. Use of a compound as defined in definitions 1 or an antagonist as defined in definition 2, preferably further defined as in any one of definitions 3 to 8, for the manufacture of a medicament for the therapeutic administration to reduce body weight and/or body fat or to treat or prevent obesity in a subject.

Preferably the present invention is defined as in the following embodiments:
1. The method of reducing weight and/or body fat in a subject comprising the administration of the therapeutic compound Vandetanib.
2. Vandetinib for use in a therapeutic method of reducing weight and/or body fat in a subject comprising the administration of the therapeutic compound Vandetanib.
3. The method of reducing weight and/or body fat in a subject comprising the administration of a therapeutic compound selected from Dasatinib, Tanespimycin, S-17092, and Sorafenib.
4. The method of reducing weight and/or body fat in a subject comprising the administration of a therapeutic compound selected from Lintopride, Fenoprofen, Sulfaphenazole, Fluticasone, Rolipram, Febuxostat and Verapamil.
5. The method of reducing weight and/or body fat in a subject comprising the administration of a therapeutic compound selected from a modulator of gene CG9438, or an ortholog thereof, in particular the human orthologue CYP3A4, the compounds being selected from erlotinib, gefitinib and lapatinib.
6. The method of reducing weight and/or body fat in a subject comprising the administration of a therapeutic compound selected from a modulator of gene CG8222, or an ortholog thereof, in particular the human orthologue KDR, the compounds being selected from axitinib, pazopanib and semaxanib.
7. The method of reducing weight and/or body fat in a subject comprising the administration of a therapeutic compound selected from a modulator of gene CG6919, or an ortholog thereof, in particular the human orthologue HTR4, the compounds being selected from cisapride, mosapride, piboserod, prucalopride, renzapride, tegaserod, tropisetron and zacopride.
8. The method or compound of any one of embodiments 1 to 7 comprising administration of the compounds in combination with any one or more therapeutic compound of table 1, preferably one or more compounds as defined in any one of embodiments 1 to 7.
9. The method or compound of any one of embodiments 1 to 8, characterized in that the treatment is for a therapy of obesity in the subject.
10. The method or compound of embodiment 9, characterized in that the patient suffers from severe Obesity with a body-mass-index (BMI) of at least 35, preferably a BMI of at least 40.
11. The method or compound of any one of embodiments 1 to 10, characterized in that the compound is administered in an effective therapeutic dose.
12. The method or compound of any one of embodiments 1 to 11, characterized in that the compound is administered topical, enteral or parenteral, in particular preferred oral or rectal, intravenous, intraarterial, intramuscular, subcutaneous, intradermal or intraperitoneal, transdermal, transmucosal or inhalational.
13. The method or compound of any one of embodiments 1 to 12, characterized in that the subject is mammal, preferably a human.
14. The method or compound of any one of embodiments 1 to 13, characterized in that the compound or antagonist is provided in a medicament.
15. The method or compound of any one of embodiments 1 to 14, characterized in that the compound or antagonist is provided together with a pharmaceutically acceptable carrier or buffer.
16. The method or compound of any one of embodiments 1 to 15, characterized in that the compound is administered in a dosage of between 0.01 mg/kg and 1 g/kg.
17. The method or compound of any one of embodiments 1 to 16, characterized in that the therapeutic compound as defined in any one of embodiments 1 to 7 is administered as the only therapeutic compound active in a treatment of reducing weight and/or body fat or of obesity in a subject.
18. Use of a compound as defined in any one of embodiments 1 to 7, preferably further defined as in any one of embodiments 8 to 17, for the manufacture of a medicament for the therapeutic administration to reduce body weight and/or body fat or to treat or prevent obesity in a subject.

### Further preferred definitions are given in the claims.

The present invention is further illustrated by the following figures and examples.

### Figures:

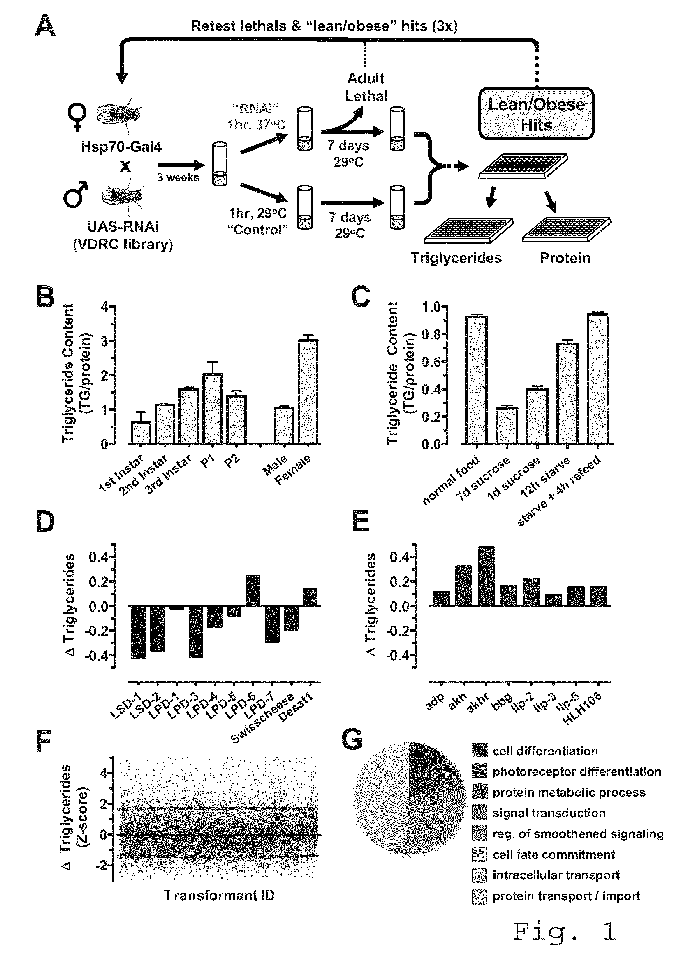

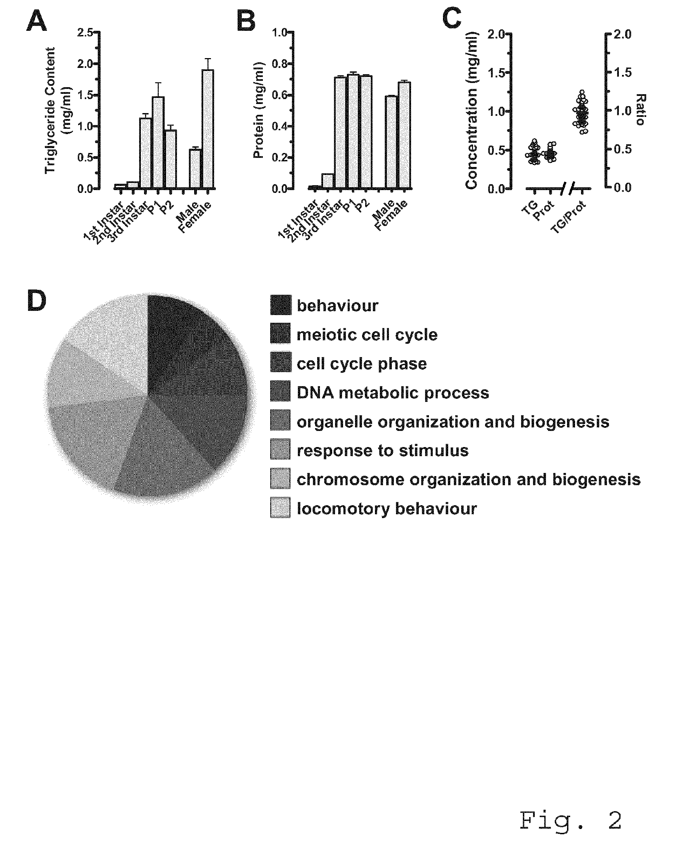

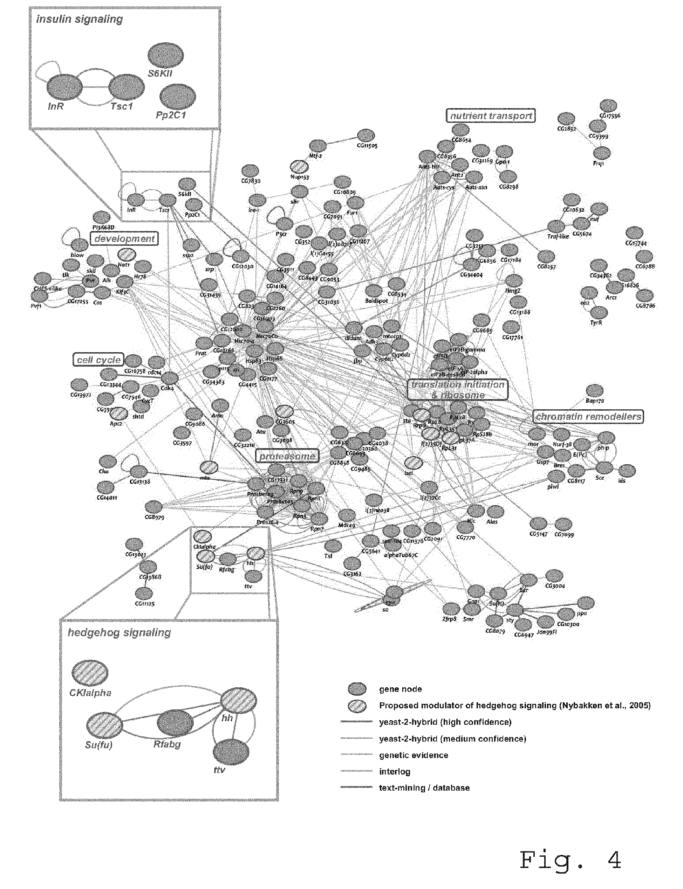

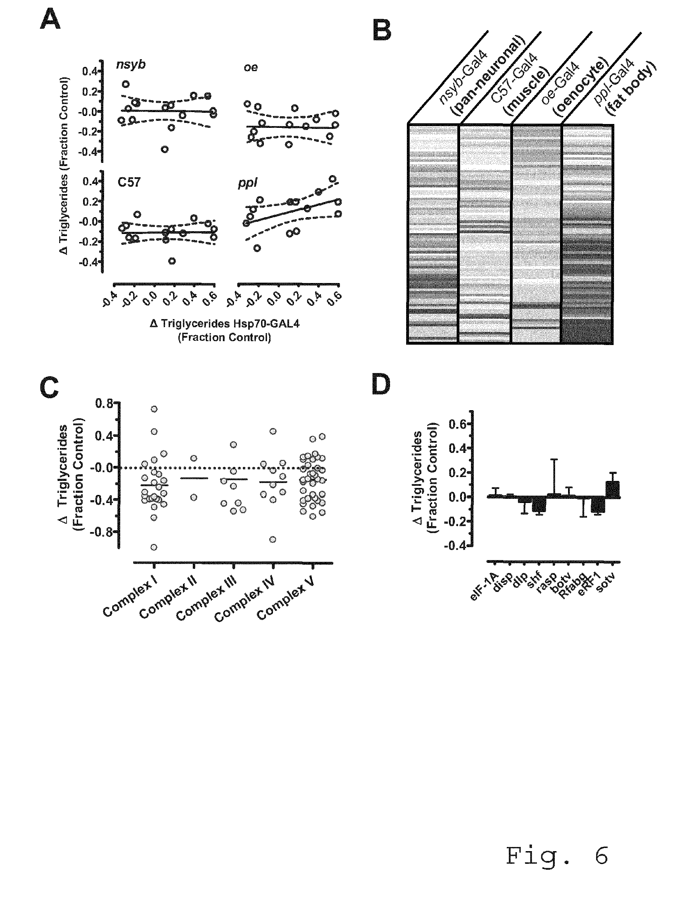

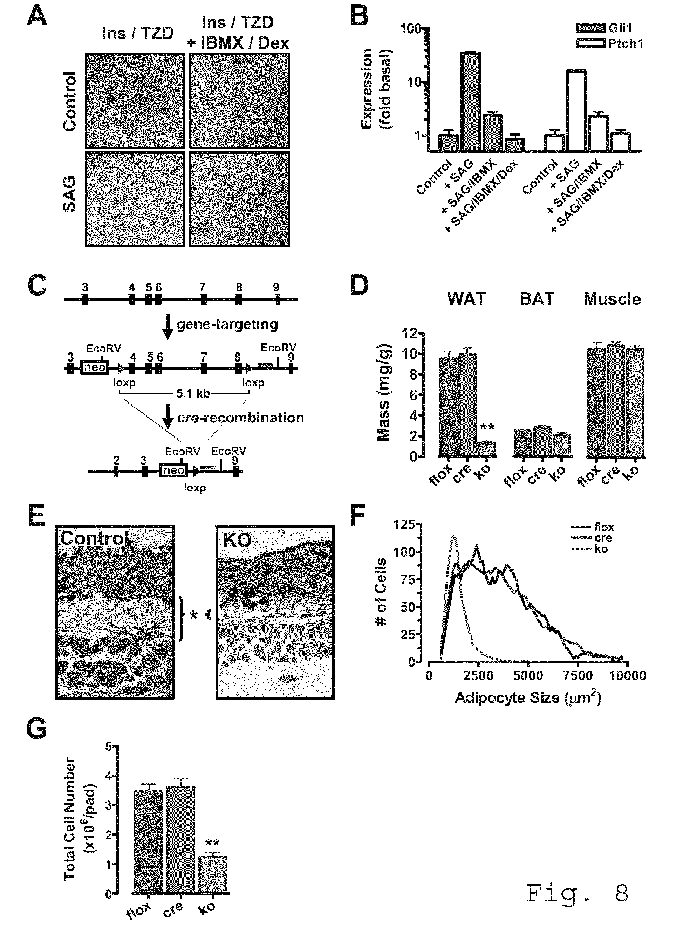

**Figure 1****. Genome-wide RNAi screen for obesity factors in adult Drosophila in vivo.**
   (A) Schematic of the screen design: virgin heat shock inducible (Hsp70-GAL4;Tub-GAL80ts) females were crossed to UAS-RNAi transgenic males. RNAi was induced 2 days post-eclosure and again after 4 days. One week after RNAi-induction triglyceride and protein levels were determined in a 96-well format and compared to internal controls: non-induced progeny of the same cross. (B) The system was capable of detecting developmental and sex-specific fat storage patterns, and (C) a variety of fedding conditions. Data are shown as mean triglyceride content +/- sem. n=8. (D,E) Double blinded retrieval of primary screen results for positive control lines predicted to (D) reduce or (E) increase triglyceride levels. (F) Z-score distribution of the primary screen results. Red lines indicate Z-scores of +1.65 above and -1.65 below baseline levels. (G) Gene ontology analysis with a level 5 cut-off for biological processes for all annotated genes with Z-scores above or below ±1.65 after three rounds of testing. See also Figs. 2 and 4.
**Figure 2****. Basal triglyceride and protein contents in Drosophila. Related to** **Figure 1****.**
   (A) Triglyceride content of w1118 Drosophila strain measured throughout development using a medium-throughput 96-well plate based system with a colorimetric determination endpoint. (B) Protein content of w1118 Drosophila measured with the same experimental set-up. Data in a and b are shown as mean triglyceride content +/- s.e.m. n=5-8. (C) Individual triglyceride and protein content in 80 different sets of 8 male flies each measured 2 to 4 days after eclosure. Measurement was made to validate the medium throughput experimental system designed for the genome-wide screen. (D) Pie chart summarizing the most depleted functional classifications using gene ontology for biological processes for all annotated genes with Z-scores in excess of +/-1.65 through three rounds of testing.

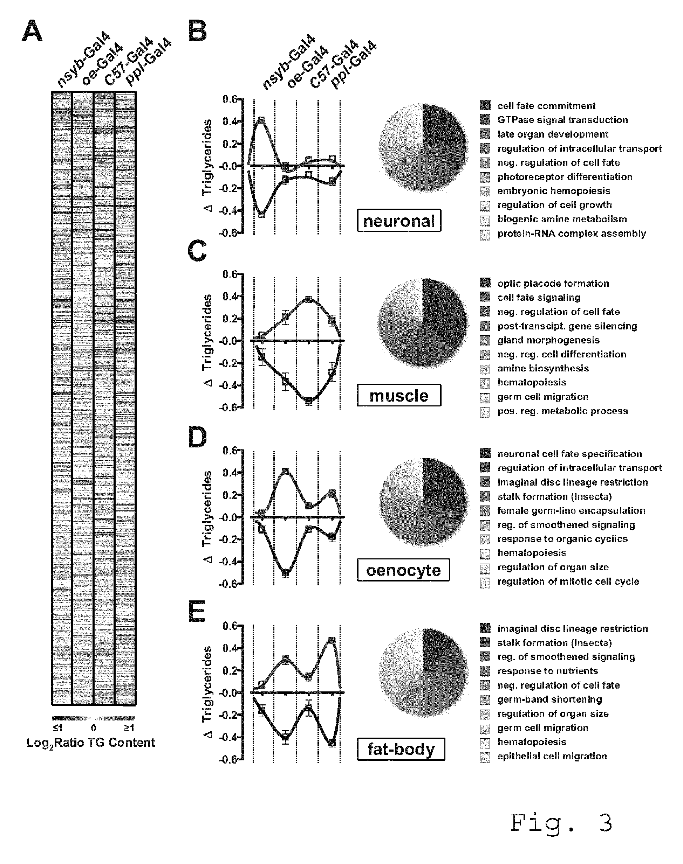

**Figure 3****. Tissue-specific regulation of fat storage.**
   (A) Heat-map of changes in triglyceride for primary screen hits crossed to nsyb-GAL4 (pan-neuronal), oe-GAL4 (oenocyte), C57-GAL4 (muscle), and ppl-GAL4 (fat-body) drivers. Changes are relative to control RNAi-lines and isogenic w1118 flies crossed to the respective GAL4 drivers. (B-E) Left panels show the mean changes in triglycerides after tissue-specific knockdown for the top-scoring fat-enhancing (red lines) and fat-depleting (blue lines) genes in each tissue category. Note the marked neuronal specificity, an overlap in fat-body and oenocyte responses, and a relative lack of specificity for top-scoring muscle responsive genes. Right panels summarize gene-ontology analysis for each category (level 5 cut-off for biological processes). Intensity of the red reflects increased significance of the GO term.
**Figure 4****. Interaction network for candidate obesity genes. Related to** **Figure 1****.**
   The interaction network was assembled using Cytoscape 2.6.2 based on interactions retrieved from STRING, DROIDB, and BI-OGRID. Datasets consisted of yeast-2-hybrid, text-mining, and database annotations (e.g. KEGG). Assembly of the visual layout was performed using manual modification of an automated force-directed layout. Insets highlight the location of both the hedgehog and insulin signaling pathways.

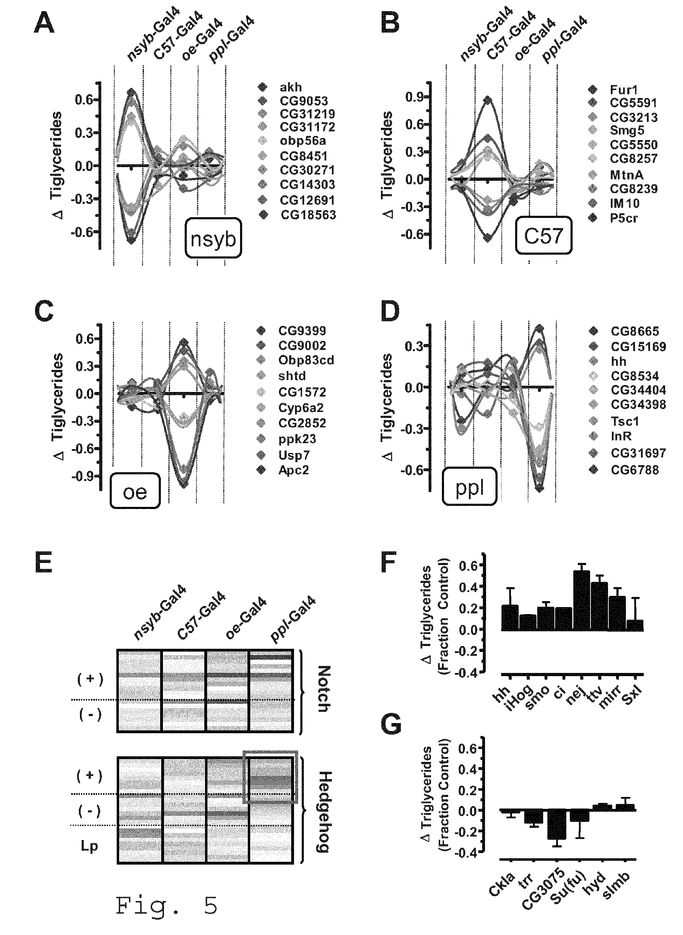

**Figure 5****. Analysis of tissue-specificity reveals hedgehog signaling as a fat-body specific regulator of triglyceride levels.**
   Triglyceride responses of candidate genes. Changes in adiposity in RNAi lines with the most tissue-restricted responses in the (A) pan-neuronal, (B) muscle, (C) oenocyte, and (D) fat-body compartments. (E) Heat-map of adiposity observed in UAS-RNAi transgenic fly lines targeting available annotated hedgehog and notch pathways. Changes are relative to averages of control RNAi-lines and w1118 flies crossed to the respective GAL4 drivers. Genes are grouped according to their role as either positive (+) or negative (-) effectors, or as mediators of ligand processing and release (Lp). (F) Representative triglyceride changes in response to ppl-GAL4 driven knockdown of effectors of hedgehog signaling and (G) repressors of the pathway. Data are presented as mean ± s.e.m., n = 4. * p<0.05. See also Fig. 6.
**Figure 6****. Tissue-specificity of hedgehog and OxPhos pathway triglyceride changes. Related to** **Figure 5****.**
   (A) Correlation analysis of triglyceride levels in RNAi lines targeting hedgehog signaling crossed to the tissue-specific drivers nsyb-GAL4 (pan-neuronal), oe-GAL4 (oenocyte), C57-GAL4 (muscle), and ppl-GAL4 (fat-body). Tissue-specific triglyceride changes (y-axes) are correlated with those observed using the inducible ubiquitous Hsp70-GAL4;Tub-GAL80ts (x-axis). (B) Triglyceride changes in ppl-GAL4 driven UAS-RNAi transgenic lines targeting hedgehog specific ligand processing and release genes. (C) Heat-map of the adiposity of UAS-RNAi transgenic fly lines targeting the members of the gene ontology category oxidative phosphorylation. Changes in adiposity were in response to tissue-specific silencing using the drivers nsyb-GAL4 (pan-neuronal), oe-GAL4 (oenocyte), C57-GAL4 (muscle), and ppl-GAL4 (fat-body). Changes are relative to averages of control RNAi-lines and w1118 flies crossed to the respective GAL4 lines. (D) Triglyceride responses of the same oxidative phosphorylation targeting RNAi-transgenic lines to heat-shock induced ubiquitous knockdown. Data are presented as mean ± s.e.m. n=4.

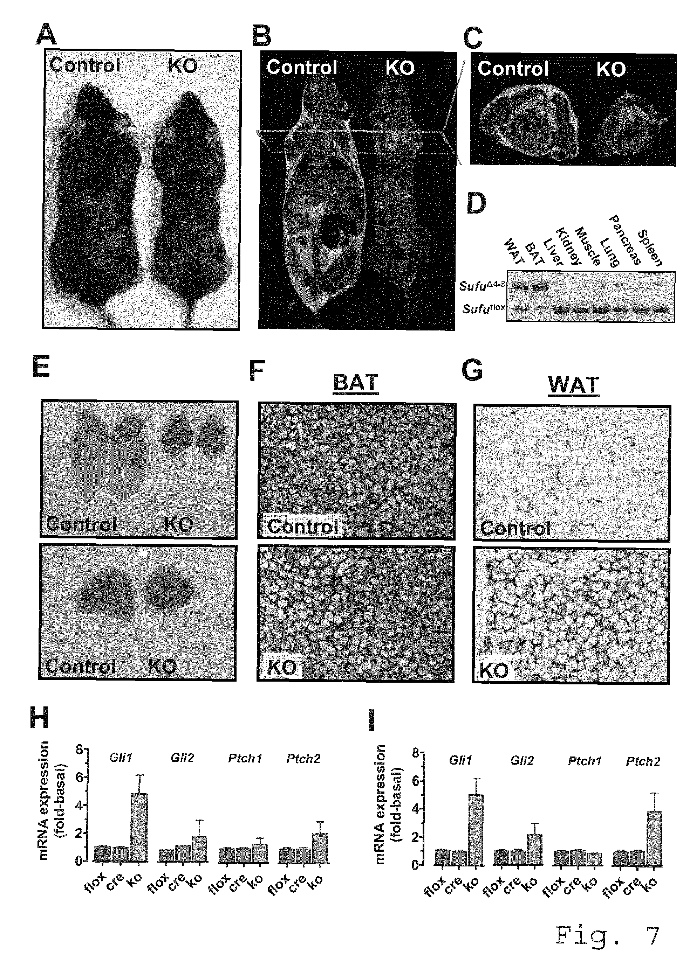

**Figure 7****. aP2-Sufu mice display white adipose tissue specific lipoatrophy.**
   (A) aP2-SufuKO mice are born healthy and at Mendelian ratios. (B) NMR imaging of an aP2-SufuKO mouse and a Sufu-expressing littermate control. (C) Cross-section at the level of the scapulae show unaltered brown adipose depots (yellow dashed lines). (D) PCR revealed robust deletion of the Sufu allele (Sufu□4-8) in both BAT and WAT depots. Minor deletion was detected in skeletal muscle, lung, and the spleen. (E) Tissue dissection white adipose tissue (WAT; upper panel) and brown adipose tissue (BAT; lower panel) revealed fully developed brown adipose depots despite severely compromised white adipose tissue depots in aP2-SufuKO mice. Dashed lines mark white adipose tissue. (F,G) Representative H&E stained sections of (F) brown (BAT) and (G) white (WAT) adipose tissues from aP2-SufuKO and control littermate mice. (H,I) Quantitative RT-PCR of the transcriptional hedgehog targets Gli1, Gli2, Ptch1 and Ptch2 confirmed activation of hedgehog signaling in both (H) WAT and (I) BAT of aP2-SufuKO mice relative to targeted (flox) and aP2-cre (cre) transgenic littermate controls. Data are presented as mean ± s.e.m. n = 5 mice per group. * p<0.05. See also Fig. 8.
**Figure 8****. IBMX and dexamethasone dependence of hedgehog signaling in adipocytes and generation of lipoatrophic Sufu mutant mice. Related to** **Figure 7****.**
   (A) Oil Red O staining of 3T3-L1 cells induced with minimal (Insulin/Troglitazone) or complete (Insulin/Troglitazone/IBMX/Dex) differentiation cocktails in the absence (control) or presence (SAG) of the hedgehog agonist SAG (200nM). One experiment representative of 5 repeats is shown. (B) Quantitative RT-PCR monitoring of hedgehog pathway activation with the target genes Gli1 and Ptch1 confirmed activation by SAG and abrogation of hedgehog induction in the presence IBMX and Dex. (C) To establish an in vivo model to assess hedgehog effects on adipose biology, a targeting strategy was used to generate mice with a conditional Sufu allele. The conditional allele encorporates two Cre-sensitive loxP sites flanking exons 4-8 of the Sufu open reading frame. Numbered boxes indicate exons. (D) White (perigonadal) adipose tissue, interscapular brown adipose tissue and muscle (soleus and gastrocnemius) masses were determined in aP2-SufuKO mice at 8 weeks of age. Data from littermates that carry the floxed allele (flox) or aP2-Cre (cre) are shown as controls. Data are presented as mean ± s.e.m. n = 6 mice per group. ** p<0.01. (E) H&E stained sections of skin highlight a clear reduction in cutaneous adipose tissue. (F) White adipocyte size distributions in perigonadal fat pads taken from 4 week old male aP2-SufuKO mice and littermate floxed (flox) and aP2-Cre (cre) controls. Measurements were made by morphometry on >10,000 (KO) and >35,000 (controls) cells per animal using a combination of scanning of H&E stained interval sectioned adipose tissue (3 per mouse) and subsequent software assisted morphometric analysis (G) Total white adipocyte cell numbers in 4-8 week old male aP2-SufuKO mice and littermate floxed (flox) and aP2-Cre (cre) controls. Data are presented as mean ± s.e.m, n=5.

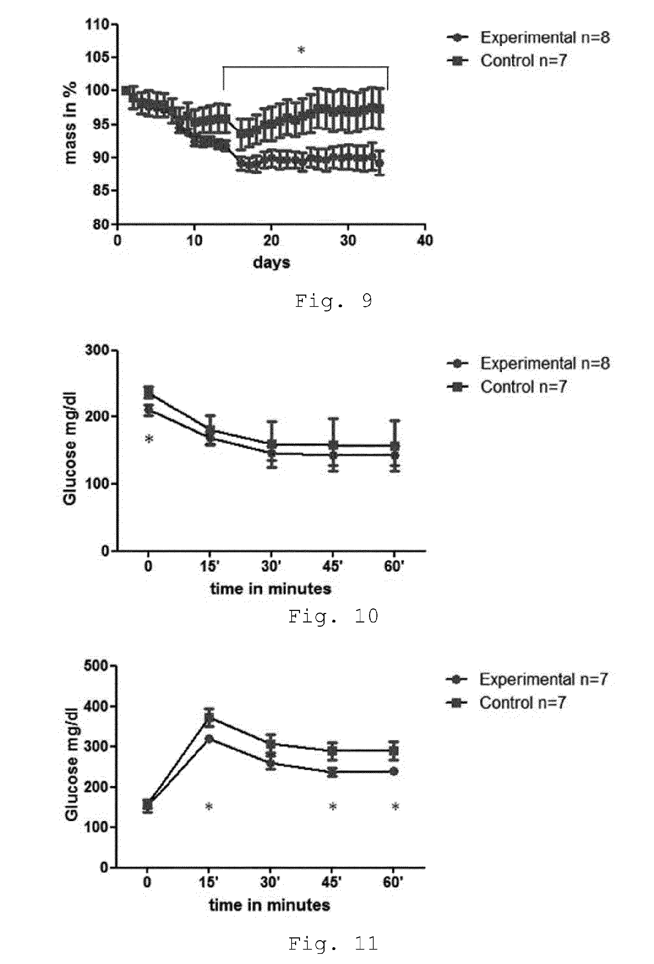

**Fig 9****. Loss of weight in Vandetanib administered mice.** C57BL6/J DIO mice were administered 40/mg/kg/day by oral gavage. The mice were weighed daily and the values were expressed as a percentage of the starting weight (day 1). By day 13 of administration there was significant loss of weight in the vandetanib administered group compared to the vehicle control (p < 0.05; unpaired student t test) which was maintained for the duration of the experiment. Error bars represent standard error of the mean.
**Fig 10****. Lower fasting glucose levels in vandetanib administered mice.** On day 7 of vandetanib administration the mice were subjected to an Insulin Tolerance Test. Briefly the mice were fasted for 2 hours. Blood was collected at time 0 prior to injection of insulin. Blood was collected at 15, 30, 45 and 60 minutes after injection to measure the glucose response. Although the insulin response was similar in both groups, the vandetanib administered mice exhibited lower fasting blood glucose levels compared to the controls (p < 0.05; unpaired student t test). Error bars represent standard error of the mean.
**Fig 11****. Oral glucose tolerance test (oGTT) in vandetanib administered mice compared to vehicle controls.** Mice were fasted overnight and on day 35 a blood sample was taken to measure the fasting glucose levels (time 0). The mice were administered glucose, blood was collected and the glucose levels were measured 15, 45 and 60 minutes after glucose administration. Vandetanib administered mice show an improved glucose response compared to the vehicle administered group 15, 45 and 60 minutes after glucose administration (p < 0.05; unpaired student t test). Error bars represent standard error of the mean.

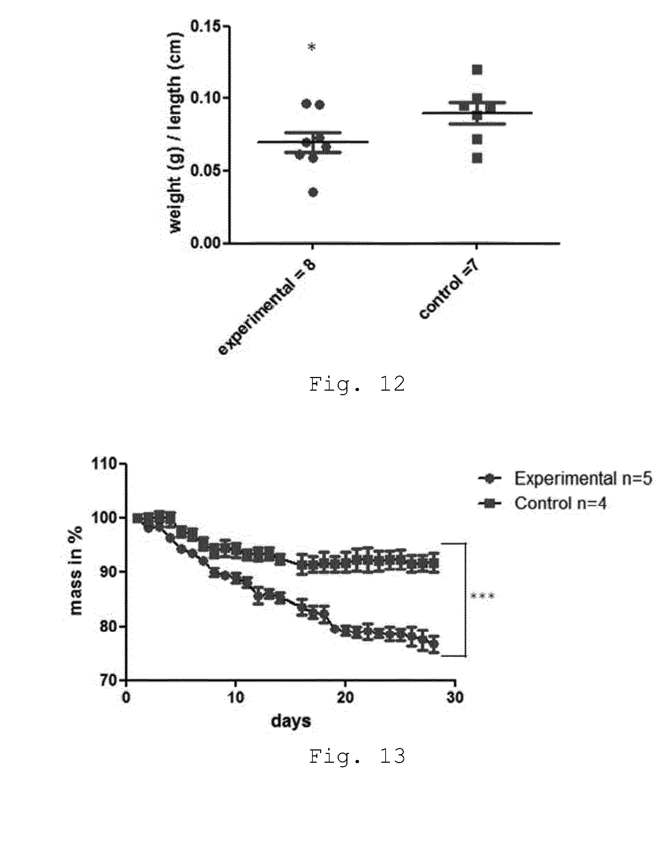

**Fig 12****. Measurement of perigonadal fat pad weights.** The vandetanib and vehicle administered mice were sacrificed on day 38 of administration. The perigonadal fat pads were dissected and the weight (grams) was expressed as a ratio compared to body length (cm). Each point on the graph represents the average weight (g) of the two perigonadal fat pads from each mouse expressed as a ratio to body length (cm). The vandetanib administered mice have a proportionally lower fat pad mass compared to vehicle controls(p < 0.05; unpaired student t test). Error bars represent standard error of the mean.
**Fig 13****. Loss of weight in dasatininb administered mice.** C57BL6/J DIO mice were administered 5/mg/kg/day for 27 days. The mice were weighed daily and the values were expressed as a percentage of the starting weight (day 1). On day 28 there was significant loss of weight in the dasatinib administered group compared to the vehicle control (p < 0.001; unpaired student t test). Error bars represent standard error of the mean.

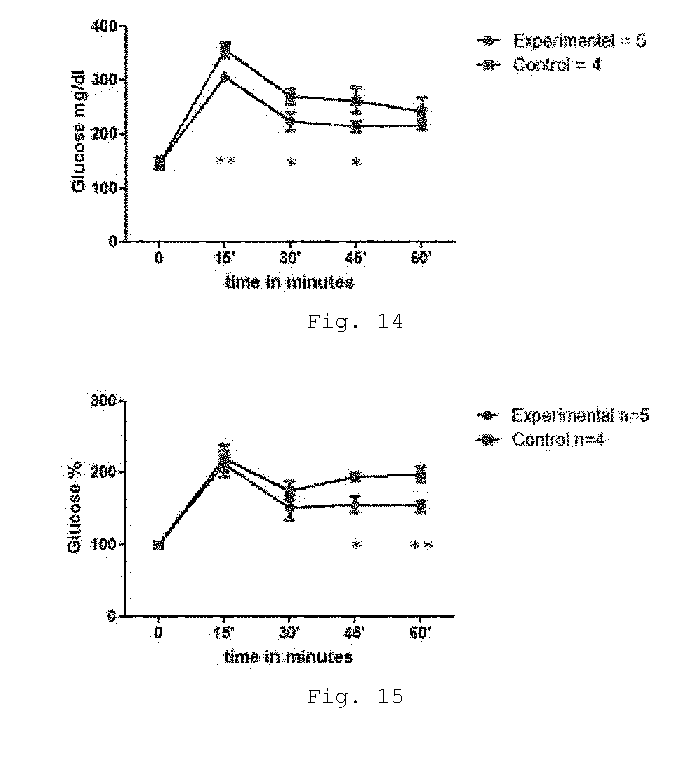

**Fig 14****. Improved insulin response in dasatinib administered mice on day 15 of administration.** An oral glucose tolerance test (oGTT)was performed in mice which were fasted overnight. A blood sample was taken to measure the fasting glucose levels (time 0). The mice were administered glucose, blood was collected and the glucose levels measured 15, 30, 45 and 60 minutes after glucose administration. Dasatinib administered mice show an improved early response to glucose administration at the 15 minute (p < 0.01), 30 and 45 minute (p < 0.05) time point compared to the vehicle administered group. Error bars represent standard error of the mean.
**Fig 15****. Improved insulin response in dasatinib administered mice on day 29.** An oral glucose tolerance test (oGTT)was performed in mice which were fasted overnight. A blood sample was taken to measure the fasting glucose levels (time 0). The mice were administered glucose, blood was collected and the glucose levels measured 15, 45 and 60 minutes after glucose administration. The values obtained for each mouse was expressed as percentage of the starting glucose levels (t = 0). Dasatinib administered mice show improved glucose clearance 45 (p < 0.05) and 60 minutes (p < 0.01) after glucose administration compared to the vehicle administered group (Student t-test; p < 0.05). Error bars represent standard error of the mean.

### Examples:

### Example 1: in vivo high throughput screen for obesity genes in Drosophila

To identify candidate obesity genes, we performed a genome-wide RNAi transgenic-RNAi screen for fat content in adult Dro*sophila* using a heat shock-inducible Hsp70-GAL4 system (Fig. 1A). Triglycerides, the major lipid storage form in animals, were chosen as a direct measure of fly adiposity. Total fly triglyceride levels were measured by colorimetric determination and normalized to protein (Fig. 1A). Using this experimental set-up, we were able to track triglyceride changes throughout development as well as to clearly distinguish sex-specific differences in fat content (Fig 1B; Fig. 2A,B) and those induced by varying nutrient availability (Fig. 1C). After the first round of screening, double-blinded analysis of RNAi lines targeting genes previously reported to regulate fat content revealed lipid alterations consistent with expected lean (Fig. 1D) and obese (Fig. 1E) phenotypes. Included were the LSD (Lipid Storage Droplet) and LPD (LiPid Depleted) genes as well as the *Drosophila* insulin like peptides (*Ilp'*s)*,* the glucagon homologue *akh* and its receptor akhr, as well as *adipose (adp), bubblegum (bbg),* and the *Drosophila* SREBP homologue, *HLH106* (Gronke et al., 2007; Hader et al., 2003; Min and Benzer, 1999).

### Example 1.1: genome-wide obesity screen in adult Drosophila

We tested the fat regulatory potential of 11,594 different UAS-RNAi transgenic lines corresponding to 10,812 transgene constructs and 10,489 distinct ORFs, in the adult fly. Primary screening involved three rounds of testing where candidates with a Z-score greater than 1.65 were selected for retesting (Fig. 1A,F). After three rounds of selection 516 RNAi-transgenic lines remained, 462 of which had only single primary target predictions (S19 score ≥0.8 and ≤6 CAN repeats as described by (Dietzl et al., 2007). Important for the translation of these findings into the mammalian context, 319 of 516 (62%) have human orthologues according to InParanoid, OrthoMCL, and Ensembl databases.

Gene ontology (GO) based pathway analysis for *biological process* revealed enrichment of gene sets involved in cell fate determination, cellular protein metabolic processes, signal transduction, intracellular transport, and regulation of smoothened signaling. Pathways most depleted during the screen, i.e. those not relevant to fat regulation, included genes regulating behavior, cell cycle, organelle organization and biogenesis, locomotory behavior, and chromosome organization. A network interaction assembly based on yeast-2-hybrid, text-mining, and pathway database information on the *Drosophila* hits and their mammalian orthologues revealed an interaction network map (Fig. 4) highlighting genes of development, nutrient transport, cell cycle regulation, the proteasome, protein translation, and chromatin remodeling. Of particular interest, the candidate gene list included a number of potential regulators of feeding control. For instance, six odorant and two gustatory receptor genes were targeted (Odorant receptors10a, 56a, 65a, 67a, 83cd, and CG10407; gustatory receptors 98b and 36b). Also, the dopamine receptor *DopR2,* two octopamine receptors (TyrR and *oa2*) and the Nmda-receptor associated protein *Nmda1* all showed reduced body fat content following RNAi induction. In addition, altered fat deposition was observed in response to RNAi knockdown of known mediators of glucose/lipid mobilization including fructose-1,6-bisphosphatase (fbp), the two members of the glycerol phosphate shuttle (CG31169 and *Gpo-1*), mitochondrial acyl-carrier protein 1 (*mtacp1*)*,* ADP/ATP translocase 2 (Ant2), pyruvate carboxykinae (CG1516), and fatty-acid synthetase (fasn). Also identified were the *Drosophila* orthologues of glucagon (akh), the insulin receptor (*dInR*), as well as the downstream kinases PI3-kinase *(dPI3K),* ribosomal-S6-kinase (*dRSK*), the CREB-coactivator *dTORC,* and the critical TOR-signaling constituent *dTSC-1*,

*Drosophila* homologues of the critical early adipogenic regulators NCOR1/2, Jag1/2, and TAK1 (Ross et al., 2004; Suzawa et al., 2003; Yu et al., 2005), or the metabolic regulators CRTC1/2 and pyruvate carboxylase (PC) (Altarejos et al., 2008; Koo et al., 2005; Zhang et al., 1995). We also hit the *Drosophila* lipoprotein rfabg (retinol fatty-acid binding glycoprotein) previously shown to transport key developmental morphogens such as *hedgehog* (Panakova, 2005). Indeed, "regulation of smoothened [hedgehog] signaling" was the most highly enriched signal transduction pathway in our gene-ontology analysis (Fig. 1G). Thus, our genome-wide approach identified multiple known molecular players previously associated with adipocyte development and function. Most importantly, the screen revealed a large number of candidate genes not previously associated with obesity.

### Example 1.2: Tissue specific mapping of candidate obesity genes

Considering the complexity of metabolism and the recognized diversity of tissue-specific processes that govern lipid-storage (Leopold and Perrimon, 2007; Speakman et al., 2008), we set out to functionally categorize the candidate lipid regulators according to tissue-specificity. RNAi-lines of the 462 primary screen candidate genes were crossed to four independent GAL4 drivers with pan-neuronal (*nsyb*-GAL4), muscle (*C57*-GAL4), oenocyte (*oe*-GAL4), and fat-body (*ppl*-GAL4) specificity, and their respective triglyceride levels determined (Fig. 3A). Interestingly, RNAi lines most strongly regulating fat-content after pan-neuronal (*nsyb*-GAL4) knockdown elicited little or no change in fly triglyceride levels when induced in the muscle, oenocyte or fat body (Fig. 3B). Muscle-specific gene silencing (C57-GAL4), by contrast, enriched for genes that also elicited significant changes in triglycerides when targeted in oenocytes and the fat-body (Fig. 3C). RNAi-lines responding most substantially to oenocyte and fat-body specific knockdown displayed a coordinate and reciprocal pattern of adiposity regulation (Fig. 3D and 3E); these findings are in keeping with the tight regulatory interplay reported for these correlates of the mammalian adipose and liver (Gutierrez et al., 2007). GO analysis of the combined fat-enhancing and fat-diminishing gene sets for each of the four tissues tested are summarized in Fig. 3B-E. In support of the inducible design of the current screen, cell fate, cell differentiation and organ development pathways showed strong enrichment in the analysis (Figure 3B-E, right panels: The neuronal hits (nsyb-GAL4) are: CG5436, CG2091, CG17461, CG11339, CG11202, CG5245, CG14911, CG30075, CG16836, CG5147, CG17184, CG10728, CG17742, CG4851, CG5381, CG18563, CG18268, CG10542, CG12015, CG4152, CG32669, CG32149, CG11756, CG12691, CG12595, CG32210, CG1279, CG17255, CG2260, CG14024, CG2146, CG7776, CG31132, CG3497, CG9936, CG10152, CG14842, CG6043, CG8515, CG9946, CG17819, CG11125, CG2145, CG12030, CG1759, CG1921, CG14303, CG6603, CG3906, CG30271, CG18740, CG12505, CG13202, CG8451, CG15507, CG6817, CG2818, CG12105, CG13423, CG32667, CG18586, CG17736, CG11376, CG11430, CG2898, CG4957, CG3054, CG12345, CG18414, CG14673, CG2254, CG32442, CG6122, CG13360, CG8298, CG6147, CG13319, CG33558, CG5625, CG13679, CG12806, CG3806, CG6064, CG15095, CG10691, CG3971, CG11505, CG3017, CG3108, CG3992, CG17754, CG14164, CG13332, CG3839, CG8443, CG7065, CG10632, CG1516, CG4247, CG4247, CG3004, CG31845, CG31744, CG9028, CG16826, CG13137, CG7082, CG6299, CG32091, CG30184, CG30491, CG11768, CG14968, CG17921, CG4415, CG5072, CG7199, CG4373, CG30411, CG14166, CG1555, CG2198, CG6947, CG6824, CG8079, CG1888, CG9295, CG14903, CG10617, CG10230, CG17781, CG17781, CG6721, CG7103, CG32170, CG3526, CG7737, CG11601, CG32336, CG1975, CG14909, CG11797, CG9922, CG11555, CG30470, CG17841, CG9153, CG31172, CG17335, CG7287, CG3396, CG13116, CG13984, CG33466, CG30065, CG14265, CG32234, CG2076, CG4038, CG14704, CG9053, CG30486, CG31219, CG1171, CG10840, CG6577, CG7099, CG1433, CG13673; The muscle (*C57*-GAL4) *hits are: CG11339, CG18740, CG11376, CG7206, CG4583, CG12015, CG32667, CG14011, CG14024, CG11601, CG9634, CG12505, CG9695, CG14911, CG30486, CG12595, CG17754, CG9936, CG6009, CG1705, CG30080, CG32210, CG3817, CG8202, CG18268, CG31421, CG31692, CG31605, CG9748, CG14434, CG31161, CG12806, CG6947, CG3054, CG12770, CG6127, CG11505, CG1963, CG11555, CG5147, CG13319, CG4634, CG17819, CG32091, CG9160, CG18279, CG18279, CG32467, CG8337, CG8239, CG12259, CG3911, CG8654, CG1279, CG32140, CG7776, CG6892, CG13984, CG2091, CG17026, CG33204, CG11121, CG3497, CG6122, CG15321, CG6949, CG14375, CG10687, CG17283, CG14903, CG10101, CG14677, CG7830, CG9056, CG32459, CG2254, CG1422, CG17867, CG17781, CG17781, CG4373, CG3879, CG11756, CG10916, CG10809, CG15390, CG14265, CG9322, CG1650, CG6299, CG5550, CG2260, CG11570, CG10369, CG16836, CG30075, CG2076, CG3213, CG9220, CG31132, CG13868, CG1180, CG14166, CG14095, CG13127, CG31061, CG14362, CG32401, CG7796, CG1728, CG9506, CG3260, CG15625, CG7779, CG8107, CG30411, CG7103, CG7485, CG9676, CG3809, CG2702, CG15646, CG13972, CG14968, CG12664, CG12997, CG12997, CG4957, CG14869, CG3274, CG5719, CG5591, CG11133, CG7847, CG11768, CG8721, CG9258, CG5674, CG17184, CG13673, CG17461, CG11404, CG1622, CG9554, CG13707, CG13344, CG4264, CG10772, CG13360;* The oenocyte hits (*oe*-GAL4) are: *CG12505, CG33204, CG4207, CG15507, CG1683, CG32353, CG17461, CG11202, CG15432, CG9198, CG9295, CG1490, CG7830, CG31161, CG9028, CG3497, CG14265, CG4944, CG11229, CG11376, CG3825, CG31484, CG32210, CG1705, CG11339, CG11133, CG3806, CG3971, CG13807, CG18414, CG10166, CG12015, CG6127, CG14234, CG12595, CG17596, CG7292, CG2898, CG32091, CG17754, CG5625, CG10632, CG9634, CG2145, CG10084, CG10728, CG3526, CG6967, CG5270, CG15115, CG3425, CG9485, CG13707, CG8009, CG9160, CG33558, CG6892, CG14024, CG6689, CG3906, CG8079, CG1874, CG1516, CG5674, CG12184, CG2852, CG9438, CG6054, CG17921, CG15599, CG8202, CG33466, CG14362, CG2540, CG7352, CG9695, CG1572, CG13319, CG3313, CG13188, CG13137, CG31744, CG30360, CG3893, CG5436, CG3696, CG4851, CG7287, CG10916, CG8527, CG14909, CG3004, CG7213, CG1422, CG9760, CG30486, CG32234, CG13088, CG3440, CG4373, CG7485, CG14936, CG11425, CG10152, CG12345, CG15582, CG15646, CG8693, CG11404, CG15625, CG11125, CG2374, CG14677, CG2702, CG32401, CG9676, CG1622, CG13299, CG8107, CG31421, CG3879, CG5719, CG7737, CG4271, CG13168, CG31692, CG10101, CG17867, CG16758, CG32170, CG7796, CG5641, CG10369, CG17283, CG30184, CG9220, CG3017, CG30462, CG30462, CG31605, CG14968, CG12664, CG6299, CG2984, CG14341, CG8250, CG9399, CG3809, CG9506, CG8444, CG2818, CG31812, CG7103, CG3523, CG30065;* and the fat-body hits (*ppl*-GAL4) are: CG17461, CG15432, CG11339, CG32091, CG9565, CG3839, CG13972, CG30183, CG6721, CG14011, CG13243, CG12323, CG5245, CG11267, CG8515, CG32210, CG8654, CG2260, CG3274, CG9554, CG31845, CG8337, CG8190, CG6788, CG11376, CG3497, CG11229, CG17754, CG31697, CG32467, CG18402, CG2146, CG3523, CG30470, CG30075, CG1975, CG4264, CG32442, CG9322, CG9160, CG10632, CG16836, CG6949, CG6147, CG8079, CG3817, CG5595, CG12806, CG3980, CG16903, CG9487, CG7099, CG13744, CG11133, CG3992, CG9952, CG12875, CG7091, CG3440, CG6156, CG4038, CG32791, CG31484, CG1433, CG10687, CG5625, CG9258, CG6892, CG7776, CG31812, CG30080, CG7199, CG17331, CG6277, CG14016, CG14016, CG1874, CG5071, CG9144, CG12015, CG13332, CG32667, CG1888, CG33466, CG12602, CG31693, CG6580, CG12595, CG9056, CG7779, CG7796, CG5495, CG8298, CG15115, CG3260, CG17985, CG8534, CG4202, CG3108, CG3906, CG6054, CG3971, CG7830, CG5688, CG13868, CG7292, CG18767, CG14434, CG12184, CG11621, CG6356, CG9086, CG15646, CG10542, CG6689, CG3425, CG7206, CG11575, CG5719, CG32669, CG15579, CG1722, CG7287, CG4394, CG9220, CG14164, CG14164, CG14164, CG14704, CG3809, CG15169, CG11570, CG18319, CG31690, CG1531, CG7847, CG31692, CG4583, CG13088, CG8566, CG8107, CG32971, CG31421, CG3054, CG15390, CG14869, CG8444, CG30476, CG3879, CG2145, CG3017, CG10809, CG32459, CG14166, CG14936, CG4957, CG14095, CG8665, CG13984, CG13188, CG17841, CG32170, CG12105, CG14968, CG5381, CG18268, CG13299, CG17781, CG17781, CG4271, CG5641, CG8693, CG32140, CG32353, CG30065, CG14909, CG3893, CG6577, CG10152, CG17867, CG13116, CG3843, CG9153, CG30184, CG2984, CG1180, CG14341, CG11404, CG1622, CG32234, CG9506, CG4373, CG2374, CG12997, CG12997, CG13423, CG1963, CG5674, CG14362, CG13344, CG8250. Thus, we provide functional annotation of ~500 candidate obesity genes in four key metabolic tissues in *Drosophila.*

### Example 1.3: Neuronal hits

In mammals, the leptin/AgRP/POMC axis exemplifies the profound neuronal dependency of feeding behaviour, metabolic rate, insulin resistance and thus, of obesity risk. Flies do not possess known homologues to this axis but their feeding behavior is also neuronally anchored. Approximately one third of the primary screen hit list elicited triglyceride changes >25% when crossed with the neuronal *nsyb*-GAL4 driver. A select number exhibited tight neuronal restriction in their response (Fig. 5A). Included was the *Drosophila* homologue for SLC5A8 (CG8451; Fig 5A) a neuronal fatty-acid and lactate transporter. In rodents, fatty acids are sensed by neuronal processing of lactate generated by adjacent glial cells. Similarly, lines targeting homologues of glucagon (akh, neuronally secreted in *Drosophila*)*,* and the neuronal Zn-transporter SLC39A10 both displayed tight neuronal responses. Also, TSC1 (*dTSC1*), a critical regulator of the amino acid responsive TOR-signaling pathway, showed marked neuronal and fat-body specific responsiveness (Fig. 5D). It is likely that aside from peripheral regulation of nutrient storage, TOR signaling in the CNS might relay amino acid status to feeding behaviour. Additional neuronal responsive targets likely to play a direct role in nutrient sensing included the odorant / gustatory receptors *Obp56a* and *TyR.* Thus, similar to mammals, fat storage in *Drosophila* appears regulated by a complex network of neuronal genes.

### Example 1.4: Muscle hits

Several genes showed tight muscle-specificity (Fig. 5B), including homologues of the proline biosynthetic PYCR1 (*P5cr*)*,* the glycogen debranching enzyme AGL (*CG9485*)*,* and the fbp (fructose-1,6-bisphosphatase), a key regulator of glycolysis. Mevalonate decarboxylase (*CG8239*)*,* which supports cholesterol biosynthesis and is currently being tested therapeutically to reduce cholesterol levels, showed similar muscle-specificity as did the sterol regulating enzyme ARV1 (*CG32442*)*.* Mutants of ARV1 have previously been shown to exhibit altered lipid metabolism. Interestingly, genes involved in TLR-signaling (*IM10*), the ribosome and protein translation (*CG3213*), proteolysis (*Fur1*)*,* transcriptional regulation (*CG5591*)*,* and microRNA mediated silencing (*Smg5*) (Fig. 5B) were also found to regulate triglyceride level specifically in muscle cells.

### Example 1.5: Fat and oenocyte candidate genes

The largest number of primary screen hits showed oenocyte- (oe-GAL4) and fat-body- (*ppl*-GAL4) responses (Fig. 5C,D). Interesting targets included homologues of inflammation-related genes: ARID2 (regulates interferon responsive genes (Yan et al., 2005), *dTraf* (fly Traf-like protein), the pattern recognition receptor PGLYRP2, the interleukin enhancer binding factor ILF2, the extracellular matrix protein tenascin (TNC), the ubiquitin-conjugating enzyme UBE2N (critical for TNF- and Toll-like-receptor signaling), or the de-ubiquitinating enzyme USP7. Additional components of the ubiquitin-ligase machinery were also revealed, namely UBR2, HERC4, and FBWX5 (also controls TSC1 and thus TOR-signaling). Together these data support roles for immune regulatory networks and ubiquitination in fat storage regulation in *Drosophila.*

Oenocyte- and fat body-specific knockdown analyses also identified genes involved in glycerol and lipid metabolism (Fig. 5C,D). For instance, genes related to insulin signaling including the homologues of PP1 (inhibitory subunit 15b), S6KII, EIF2B, PI3K, and the insulin receptor itself (IR). Also, direct mediators of lipid and glucose metabolism were identified, such as homologues of the ADP/ATP symporter ANT, NDUFAB1, GDPD, and GPD2. The latter, part of the glycerol-phosphate shuttle, regulates glycolytic rate and ROS production. Of interest, mice lacking GPD2 exhibit a 40% reduction in white adipose mass (Brown et al., 2002) and share a number of phenotypic features with deficiencies of glycerol kinase (GK), another enzyme found using the oenocyte-specific driver. In addition, we found T3dh (an iron-dependent regulator of fatty acid and ketone body metabolism), *Cyp6a2* (cytochrome P450 proteins catalyze numerous steps of cholesterol, steroids and lipids synthesis), and particularly robust in both the oenocyte- and fat-body analyses, the *Drosophila* homologue of the fatty acid elongase ELOVL6. *Elov16*^{*-*/*-*} mice develop marked obesity and hepatosteatosis and show protection from hyperinsulinemia, hyperglycemia, and hyper-leptinemia (Matsuzaka et al., 2007). Using fat-body specific knockdown we also hit the *Drosophila* homologue of ELOVL7. Perhaps most importantly, we found multiple previously uncharacter-ized genes that regulate fat content in an oenocyte- and/or fat body-dependent manner (Fig. 5C,D). Thus, our screen has revealed a large number of general and tissue specific candidate fly genes and multiple pathways that control triglyceride storage levels.

### Example 2: Mouse in vivo pathway modulation

The biological process *"regulation of smoothened [hedgehog] signaling"* was one of the most prominent signal transduction pathway of all pathways in the primary screen. An additional 8 potential hedgehog signalling members recently identified in a *Drosophila* S2 cell screen for modulators of hedgehog signalling (Nybakken et al., 2005) were also hit in our primary obesity screen. Together these represent a >20-fold enrichment for the hedgehog signaling pathway. Importantly, hedgehog signaling scored third in fat-body-responsive pathways while not scoring at all in muscle or neuronal datasets (Fig. 5E; Fig. 6A). Hedgehog modulation has been suggested previously as a target for obesity therapies (Suh et al. Cell Metabolism, 2006; WO2000/51628). We therefore homed into the hedgehog pathway to provide proof of principle for the fly screen and to translate our *Drosophila* results directly into the mammalian context.

To assess the *in vivo* relevance of the drosophila screen results, hedgehog signaling in mammalian adipogenesis was further investigated. Fat-specific Sufu knockout animals (aP2-*Sufu*KO) were generated (Fig. 8C). Sufu is a potent endogenous inhibitor of hedgehog signaling in mammals. *Sufu*^{flox/flox} mice were crossed to the adipose-tissue deleting aP2-Cre transgenic line (Fig. 8C) and the resulting aP2-*Sufu*KO animals were born healthy and at Mendelian ratios. PCR amplification revealed target deletion in both white (WAT) and brown (BAT) adipose tissue (Fig. 7A,D). aP2-*Sufu*KO mice displayed an immediate and obvious lean phenotype. MRI analysis revealed a significant and global reduction in white adipose tissue mass, including subcutaneous, perigonadal, and mesenteric depots (Fig. 7B). Intriguingly though, in contrast to the gross loss of WAT, cross-sectional examination of the interscapular region revealed fully developed BAT depots of both normal size and lipid content (Fig. 7B,C). Direct measurement of WAT and BAT depot weights corroborated the divergent WAT / BAT phenotype, with an ∼85% reduction in perigonadal fat pad mass in *aP2*-*Sufu*KO mice concomitant with unaltered BAT mass (Fig. 7E, Fig. 8D). Tissue weight and histological analyses confirmed lack of any remarkable phenotype in multiple other tissues including pancreas and liver (no indication of steatosis), and muscle mass was unaffected (Fig. 8D). Cutaneous adipose was also markedly diminished (Fig. 8E). Whereas the morphology of *Sufu*-deficient BAT depots was largely indistinguishable from that of control animals (Fig. 7E,F), examination of multiple WAT pads revealed marked and significant reductions in both adipocyte size (Fig. 7E,G; Fig. 8F) and total numbers (Fig. 8G) in mutant animals. Of note, qPCR showed elevated Gli1, Gli2, and Ptch2 expression in both WAT (Fig. 7H) and BAT (Fig. 7I), verifying the intended pathway activation in both tissues. Thus, deletion of *Sufu* in fat tissue results in a markedly decreased white fat cell number and, remarkably, in normal brown adipose tissue. These results demonstrate that hedgehog activation results in a virtually complete block of WAT development but leaves the differentiation process of brown adipocytes wholly intact.

### Example 3: Testing of active compounds

General preferred layout: The mice were divided into cages of 2-4 mice per cage and allowed to acclimatise to the new housing conditions for at least 2 weeks. The mice were weighed every week to monitor their weight and ensure that the mice are either gaining weight or stabilized prior to initiation of the experiment. Mice were randomly assigned to weight-matched groups for compound administration. Compound dosage and routes of administration were determined based on published literature and pharmacokinetic studies.

To test the positive candidates in mice the following protocol was used: JAX^{®} DIO B6 mice, 18 weeks of age, were divided into groups of 2-4 mice of equivalent body weight upon receipt from Jax labs. The Jax labs protocol for feeding and care of diet-induced obese (DIO) C57BL/6J mice is as follows: Male mice are selected at random at four weeks of age and fed a 6% fat (wt/wt) chow diet (Lab Diet^{®} 5k52). At six weeks of age, mice are placed in wean cages in groups of 10 and are fed high fat diet to induce obesity (Research Diets, Inc. D12492i, 60 kcal% fat). Mice have ad libitum access to food and water. Upon receipt of the animals from Jax labs, animals were kept under similar housing and feeding conditions to those at Jax labs ie. mice continued to receive a high fat diet (Test Diet^{®} 58Y1 60% energy from Fat).

In special methods following protocol was used: Modulator compounds of the new identified genes responsible for triglyceride or fat regulation underwent two rounds of testing. The first round screened all candidate compounds in Drosophila, and positive candidates were subsequently tested in mice induced to be obese through administration of a high fat diet. Two day old W1118 male Drosophila melanogaster were sorted 20 flies per vial and placed for one week on normal fly food in the presence or absence of each test compound. Test compounds were added to the surface of the fly food in liquid form and allowed to absorb into the top-most layer at 3 doses 0.001 umol/kg/day, 1 umol/kg/day, and 1000 umol/kg/day.

After one week of treatment flies were shock-heated to dryness, and dry weight used as an indicator of adiposity. All flies with dry weights <75% of the mean value of control vials were considered positive candidates with obesity lowering activity. Those positive candidates which induced obvious behavioural impairment were removed from the group of positive candidates. Behavioural tests included tapping of the vial (flies should jump into flight) and exposure to light (flies should move towards a light source). All remaining positive candidates were taken forward for analysis in mice.

To test the positive candidates in mice the following protocol was used: JAX^{®} DIO B6 mice, 12 weeks of age, were divided into groups of 8-10 mice of equivalent body weight upon receipt from Jax labs. The Jax labs protocol for feeding and care of diet-induced obese (DIO) C57BL/6J mice is as follows: Male mice are selected at random at four weeks of age and fed a 6% fat (wt/wt) chow diet (Lab Diet^{®} 5k52). At six weeks of age, mice are placed in wean cages in groups of 10 and are fed high fat diet to induce obesity (Research Diets, Inc. D12492i, 60 kcal% fat). Mice have ad libitum access to food and water. Upon receipt of the animals from Jax labs, animals were kept under identical housing and feeding conditions to those at Jax labs ie. mice continued to receive the high fat diet listed above. For all substances known to be orally available in mammals, mice were treated in drinking water at 5x and 250x the recommended human therapeutic dose. For other compounds dosing was based on published pharmacokinetics in mice or, where unavailable, based on the lowest functional dose in flies (ie. the 1x and 50x the lowest functional dose in flies). Compounds known to be orally unavailable in mammals were administered once daily by manual injection i.p. in a cellulose injection vehicle at doses determined as above. Body weight as well as food and water intake were monitored over the two week treatment period, and where mean body weight reductions relative to control animals were significant by t-test (p<0.05) and exceeded 2g (∼ 5% of body weight) body fat composition was assessed by weighing total body weight, as well as peri-gonadal and subcutaneous fat pad weight at sacrifice. Compounds where changes in body weight correlate directly with changes in body fat composition were considered positive therapeutic candidates for treating mammalian obesity.

### Example 3.1. Administration of Vandetanib

Vandetanib also known as ZD6474 is a tyrosine kinase inhibitor that functions as an antagonist of the vascular endothelial growth factor receptor (VEGFR) and the epidermal growth factor receptor (EGFR).

Vandetanib powder was obtained from Selleck Chemicals (S1046). Male C57B16/J DIO mice of 30 weeks of age were weight matched and housed in groups of 2 mice per cage and kept on 12-h light-dark cycle. Food and water were given ad libitum. 8 compound administered mice (experimental) and 7 vehicle administered mice (control) were included in the experiment.

Mice were administered a daily dose of Vandetanib by oral gavage. Vandetanib was delivered in a vehicle consisting of 1% tween in PBS. Control mice were administered vehicle alone, daily, by oral gavage.

The dose of Vandetanib chosen for this study was 40mg/kg/d. This has been determined to be an effective dose for the inhibition of the target VEGFR in various mouse cancer models. In Choi et al 2008, Vandetanib was administered at a dose of 50 mg/kg/d by oral gavage in a vehicle of 1% tween 80 with PBS for 4 weeks in an orthopic nude mouse model of human Adenoid Cycstic Carcinoma. During the treatment period vandetanib was well tolerated by the mice and no adverse side effects or loss in body weight was observed (Choi et al 2008). This dose was effective to significantly reduce tumour volume compared to vehicle administered controls (Choi et al 2008). Treatment at a dose of 50mg/kg for 21 days in a renal cell carcinoma mouse model was well tolerated with no drug related changes in weight or behaviour observed (Drevs et al., 2004). This dose was also effective to reduce primary tumour volume (Drevs et al., 2004). Dosing in mice with Vandetanib in preclinical studies range from 12.5 to 150 mg/kg/day (Wedge et al., 2002; Ciardiello et al., 2003; Taguchi et al., 2004; Damiano et al., 2005). Generally a dose above 25mg/kg/day leads to an inhibition of tumour growth or induction of tumour regression whereas doses at or below this level tend to lead to a slowing of tumour growth (Wedge et al., 2002; Gustafson et al., 2006). Vandetanib has been administered to mice up to a dose of 100mg/kg in mice for 35 days with no obvious effect on clinical condition (Wedge et al., 2002).

The DIO mice were weighed on the same day prior to the first compound administration (day 1). The mice were weighed on a daily basis during the experiment and monitored for overt signs of toxicity or stress. Vandetanib was well tolerated during the course of the study and no adverse side effects were observed. We observed an increased loss of weight in the compound administered mice compared to the vehicle administered controls (Fig 1). By day 13 we observed a statistically significant weight loss in the vandetanib administered mice compared to controls which was maintained for the duration of the experiment (p < 0.05; unpaired student t test).

On day 7 an Insulin tolerance test was performed (Fig 10) Although the insulin response was similar in both experimental and control groups, we did observe a significant reduction in fasting glucose levels in the experimental mice compared to the controls (p < 0.05).

On day 35 an oral glucose tolerance test was performed (Fig 11). We observed a reduction in the level of glucose levels at the 15, 45 and 60 minute time point after glucose administration indicating that the vandetanib administered mice show an improved insulin response compared to controls (p < 0.05).

On day 38 of compound administration the mice were sacrificed. The perigonadal fat pads were isolated and weighed. These values were expressed as a proportion of the body length of the mouse (Fig 12). We observed a reduction of fat pad weight in the vandetanib administered group indicating the loss of weight in these mice is due to loss of fat pad mass (p < 0.05; unpaired student t test).

Taken together these data indicate that Vandetanib leads to an almost 10% reduction of body weight that stabilized over the course of the experiment. The reduction in blood glucose levels and improvement of glucose handling are consistent with the observed reduction in adiposity.

### Example 3.2. Administration of Dasatinib

Dasatinib is a multiple BCR/abl Src family tyrosine kinase inhibitor. It is approved for use in the treatment of chronic myelogenous leukaemia.

Dasatinib was obtained as a powder from Selleck chemicals. C57B16/J DIO mice of 19 weeks of age and weighing more than 33g were weight matched and housed in groups of 4 mice per cage and kept on 12-h light-dark cycle. Food and water were given ad libitum. 5 dasatinib administered mice (experimental) and 4 vehicle administered mice (controls) were used in the experiment. Mice were administered a dose of 5mg/kg/day daily of Dasatinib intraperitoneally in a vehicle of 1:1 propylene glycol/water. Control animals were administered the vehicle alone. 1.25mg/kg/dose BID or 2.5mg/kg/dose QD are considered to be the minimum efficacious dose based on studies in severe combined immunodeficient mice bearing s.c. K562 xenografts (Lee et al., 2004). The preclinical efficacious dose of 2.5mg/kg/day is approximately equivalent to a clinical does of 25mg/day (Luo et al., 2006). A dosing regimen in mice of 5mg/kg, PO) closely mimics the pharmacokinetics of the approved human once-daily dose of 100mg. Based on this we decided to administer a dose of 5mg/kg/day in obese mouse models.

The DIO mice were weighed on the same day prior to the first compound administration (day 1). The mice were weighed on a daily basis during the experiment and monitored for overt signs of toxicity or stress. Dasatinib was well tolerated during the course of the study and no adverse side effects were observed. We observed a significant weight loss in the dasatinib administered mice compared to the vehicle administered controls (Fig 13). On day 5 we observed a significant weight reduction in the dasatinib group and by day 28 these mice had lost an average of 20% of their starting weight. This represented a statistically significant loss compared to vehicle administered mice (p < 0.001; unpaired student t test).

On day 15 an oral Glucose tolerance test was performed. Although both groups exhibit a similar fasting glucose level (time 0) there is an improved insulin response 15, 30 and 45 minutes after glucose administration in the experimental group (Fig 14). This indicates that dasatinib leads to an improvement in insulin response and glucose clearance in mice fed a high fat diet.

On day 29 an oral glucose tolerance test was performed. We compared the glucose measurements to that of the fasting glucose levels at time 0 (taken as 100%) in each group to determine the relative changes in glucose clearance (Fig 15). We observed an improved glucose clearance in the dasatinib administered mice (45 mins p < 0.05; 60 mins p < 0.01). Taken together the improvements in insulin sensitivity and glucose handling are consistent with the observed reduction in weight.

### References:

All of the following publications are incorporated herein by reference.
Altarejos et al. (2008) Nature medicine 14, 1112-1117.
Ashrafi et al. (2003) Nature 421, 268-272.
Baker et al. (2007) Cell metabolism 6, 257-266.
Brown et al. (2002) The Journal of biological chemistry 277, 32899-32904.
Buhman et al. (2004) J Nutr 134, 2979-2984.
Cypess et al. (2009) N Engl J Med 360, 1509-1517.
Dietzl et al. (2007) Nature 448, 151-156.
Fan et al. (1995) Cell 81, 457-465.
Farmer, S.R. (2006) Cell metabolism 4, 263-273.
Farmer, S.R. (2009) Nature 458, 839-840.
Farooqi et al. (2007) Obes Rev 8 Suppl 1, 37-40.
Gesta et al. (2007) Cell 131, 242-256.
Gronke et al. (2005) Cell metabolism 1, 323-330.
Gronke et al. (2007) PLoS Biol 5, e137.
Gutierrez et al. (2007) Nature 445, 275-280.
Hader et al. (2003) EMBO Rep 4, 511-516.
Hebrok et al. (2000). Regulation of pancreas development by hedgehog signaling. Development (Cambridge, England) 127, 4905-4913.
Heine et al. (2009) The Journal of clinical investigation 119, 267-277.
Jiang et al. (2008) Dev Cell 15, 801-812.
Jiang et al. (1995) Cell 80, 563-572.
Koo et al. (2005) Nature 437, 1109-1111.
Lefterova et al. (2009) Trends Endocrinol Metab 20, 107-114. Leopold et al. (2007) Nature 450, 186-188.
Li et al. (2008) Int J Biol Sci 4, 29-36.
Makino et al. (2001) Developmental biology 239, 95-106.
Martin et al. (2006) J Neurochem 98, 279-288.
Matsuzaka et al. (2007) Nature medicine 13, 1193-1202.
Melcher et al. (2007) The Journal of endocrinology 192, 467-472. Min et al. (1999) Science (New York, NY 284, 1985-1988. Nybakken et al. (2005) Nature genetics 37, 1323-1332.
Oldham et al. (2003) Trends Cell Biol 13, 79-85.
Rosen et al. (2006) Nature reviews 7, 885-896.
Ross et al. (2004) Molecular and cellular biology 24, 3505-3513. Schlegel et al. (2007) PLoS Genet 3, e199.
Seale et al. (2009) Genes & development 23, 788-797.
Speakman et al. (2008) Lab Anim 42, 413-432.
Suh et al. (2006) Cell metabolism 3, 25-34.
Suzawa et al. (2003) Nature cell biology 5, 224-230.
Thomas et al. (2000) Diabetes 49, 2039-2047.
Tseng et al. (2008) Nature 454, 1000-1004.
van Marken Lichtenbelt et al. (2009) N Engl J Med 360, 1500-1508.
Virtanen et al. (2009) N Engl J Med 360, 1518-1525.
WHO (2009). Global Strategy on Diet, Physical Activity and Health.
Wu et al. (2002) J Am Chem Soc 124, 14520-14521.
Yan et al. (2005) Genes & development 19, 1662-1667.
Yu et al. (2005) The Journal of biological chemistry 280, 13600-13605.
Zehentner et al. (2000) DNA Cell Biol 19, 275-281.
Zhang et al. (1995) The Biochemical journal 306 ( Pt 1), 205-210.
Zhang et al. (1994) Nature 372, 425-432.
Zingaretti et al. (2009) Faseb J 23, 3113-3120.
Rosik CH. (2005) Obes Surg. 2005;15:677-683.
Sarwer et al. (2004) Obes Surg. 14:1148-1156.
Hensrud et al. (2006) Mayo Clin Proc. Oct;81(10 Suppl):S5-10. Encinosa et al. (2005). Use and costs of bariatric surgery and prescription weight-loss medications. Health Aff (Millwood). 24:1039-1046.
Fontaine et al. (2003). Years of life lost due to obesity. JAMA. 289:187-193.
WHO. Obesity: preventing and managing the global epidemic. Report of a WHO Consultation. WHO Technical Report Series 894. Geneva: World Health Organization, 2000.
Choi et al. (2008) Clin Cancer Res. Aug 15;14(16):5081-9.
Drevs et al. (2004) Angiogenesis. 7(4):347-54.
Wedge et al. (2002) Cancer Res. Aug 15;62(16):4645-55. Ciardiello et al. (2003) Clin Cancer Res. Apr;9(4):1546-56. Taguchi et al. (2004) Cancer Sci. Dec;95(12):984-9.
Damiano et al. (2005) Clin Cancer Res. Aug 1;11(15):5639-44. Gustafson et al. (2006) J Pharmacol Exp Ther. Aug;318(2):872-80. Lee et al. (2004). BMS-354825: a potent dual SRC/ABL kinase inhibitor possessing curative efficacy against imatinib sensitive and resistant human CML models in vivo. In: AACR 95th Annual Meeting; 2004.
Luo et al. (2006) Clin Cancer Res. Dec 1;12(23):7180-6.
Morain et al. (2000) Br J Clin Pharmacol. Oct;50(4):350-9
Wang et al. (2010) Proc Natl Acad Sci U S A. 107(20):9323-8. Giannarelli et al. (2009) Circulation. Mar 31;119(12):1625-33

## Claims

1. S-17092 for use in a method of reducing weight and/or body fat in a subject comprising the administration of the therapeutic compound S-17092.

2. Compound for use according to claim 1, the method comprising administration of the compounds in combination with any one or more therapeutic compound of table 1, preferably one or more compounds selected from Dasatinib, Tanespimycin, Sorafenib, Lintopride, Fenoprofen, Sulfaphenazole, Fluticasone, Rolipram, Febuxostat, Verapamil, erlotinib, gefitinib, lapatinib, axitinib, pazopanib, semaxanib, cisapride, mosapride, piboserod, prucalopride, renzapride, tegaserod, tropisetron and zacopride.

3. Compound for use according to claim 1 or 2, characetreized in that the treatment is for a therapy of obesity in the subject.

4. Compound for use according to claim 3, **characterized in that** the patient suffers from severe Obesity with a body-mass-index (BMI) of at least 35, preferably a BMI of at least 40.

5. Compound for use according to any one of claims 1 to 4, **characterized in that** the compound is administered in an effective therapeutic dose.

6. Compound for use according to any one of claims 1 to 5, **characterized in that** the compound is administered topical, enteral or parenteral, in particular preferred oral or rectal, intravenous, intraarterial, intramuscular, subcutaneous, intradermal or intraperitoneal, transdermal, transmucosal or inhalational.

7. Compound for use according to any one of claims 1 to 6, **characterized in that** the subject is mammal, preferably a human.

8. Compound for use according to any one of claims 1 to 7, **characterized in that** the compound or antagonist is provided in a medicament.

9. Compound for use according to any one of claims 1 to 8, **characterized in that** the compound or antagonist is provided together with a pharmaceutically acceptable carrier or buffer.

10. Compound for use according to any one of claims 1 to 9, **characterized in that** the compound is administered in a dosage of between 0.01 mg/kg and 1 g/kg.

11. Compound for use according to any one of claims 1 to 10, **characterized in that** the therapeutic compound is administered as the only therapeutic compound active in a treatment of reducing weight and/or body fat or of obesity in a subject.

12. Use of the compound S-17092 for the manufacture of a medicament for the therapeutic administration to reduce body weight and/or body fat or to treat or prevent obesity in a subject.
